# EUROPEAN PATENT APPLICATION

(11) **EP 4 309 665 A1**
(43) Date of publication of application: **24.01.2024**
(21) Application number: 22770257.8
(22) Date of filing: 18.02.2022
(51) Int. Cl.: A61K 38/12, C40B 50/06, C40B 70/00

(54) **CYCLIC COMPOUND LIBRARY AND CONSTRUCTION METHOD THEREFOR**

(30) Priority: 18.03.2021 CN 202110291745
(71) Applicant: Yafei Shanghai Biolog Medicine Science & Technology Co., Ltd., Shanghai 201203 (CN)
(72) Inventor: WEI, Wei, Shanghai 201203 (CN); LIU, Chen, Shanghai 201203 (CN); CHEN, Bin, Shanghai 201203 (CN); LIU, Yuan, Shanghai 201203 (CN); BAI, Yuanchao, Shanghai 201203 (CN)
(74) Representative: Bjerkén Hynell KB
(86) International application number: PCT/CN2022/076774
(87) International publication number: WO 2022/193902

(57) **Abstract**

A cyclic compound library and a construction method therefor. The method comprises reacting a solid-phase carrier, a molecule containing a photocleavable group, a linking molecule, a building block, and an A-end ring-closure molecule and a B-end ring-closure molecule at the two ends for synthesizing, removing the solid-phase carrier by means of a method of decomposition under light radiation, and performing a ring-closure reaction by means of using amino acid residue structures of the A-end ring-closure molecule A and the B-end ring-closure molecule B under the action of a cyclic peptide synthetase. The method has the advantages of mild ring-closure conditions and higher universality, which expands the chemical reaction types of the encoded compound library and the diversity space of the compound library.

## Description

### TECHNICAL FIELD

The present invention relates to the field of pharmaceutical chemistry, and in particular to a cyclic compound library and a construction method therefor.

### BACKGROUND ART

Advances in disease characterization and target identification has constantly moved drug discovery into unknown realms. From a pathological perspective, more and more attractive targets emerge, which, however, simultaneously brings a challenge to the discovery of established low molecular weight compounds or biological agents to modulate the function of these targets. A method for coping with this challenge is to establish a compound library. Small molecule compounds derived from a traditional compound library are in principle bioavailable, allowing cell infiltration and specific binding into a defined protein (such as an enzyme).

Compounds derived from a DNA-encoded library (DEL) are similar to small molecules, standing a chance to screen out up to 10⁹ or more possible chemical entities. This greatly increases the chance of looking for new beginnings in chemistry. However, small molecules generally show poor behaviors against protein-protein interactions. Protein interactions tend to have a binding surface with a large extension order, while biological agents are more suitable for such applications and generally have better selectivity and stronger binding force.

Cyclic peptides, as a promising class of therapeutic candidates, have drawn widespread attention. They offer several important advantages over their linear congeneric products. Macrocyclization generally limits the conformation freedom of cyclic peptides, where cyclic molecules can bind more tightly and specifically to target proteins by minimizing an entropy upon binding. In addition, when hydrolyzed by proteases, the cyclic peptides are more stable than linear molecules. In addition, due to their relatively wide structure, the cyclic peptides can more effectively cover large and shallow interfaces involved in protein-protein interactions, while targeting is difficult for traditional drug-like small molecules.

At present, a reference document 1 (Min Hyeon Shin, et al., Bioconjugate Chemstry, 2019, 30, 2931-2938) has reported the establishment of a DNA-encoded cyclic peptidomimetic library, and described a method for establishing the DNA-encoded cyclic peptidomimetic library, in which, however, each step of a synthesis process necessitates a chloroacetic acid for introducing a building block that is simply a primary amine small molecule compound of a single active group -NH₂. As a result, the number and type of atoms between individual building blocks are fixed, which are repeating -CO-CH₂-N- structural units, and the diversity of the compound library is insufficient. A great number of previous drug development experiments have shown that the length and type of carbon atoms between drugs can greatly change the solubility and permeability of drugs. The cyclic peptide library is mainly used to screen protein-protein interaction inhibitors (PPI) with strong binding capacity, and the binding capacity of PPI mainly depends on intramolecular or intermolecular hydrogen bonds formed by N atoms or oxygen atoms. The N atoms formed after the building blocks in molecules from the cyclic compound library of the reference document 1 are tertiary amines, which are not conducive to binding to proteins or show an insufficient binding force. It can thus be seen that the types in this compound library are very limited and cannot meet the needs of modern drug development for a diverse cyclic compound library. In addition, a difficult point in constructing the cyclic compound library lies in forming a ring by reacting both ends of a long chain structure. A ring closure method reported in the reference document 1 is limited to chemical ring closure. When a compound library is to be established, it is necessary to complete ring closure while carrying a DNA sequence, and thus the limitation on a chemical ring closure reaction is relatively high. Moreover, metal ions used in the ring closure reaction are likely to be chelated with DNA molecules, such that the stability of the DNA molecules are adversely affected, and the stability and accuracy of screening results are restricted.

In addition, a reference document 2 (CN102471772A) discloses a peptide library comprising bicyclic peptides. Bicyclic peptide molecules combine the characteristics of antibodies, small molecule drugs and peptides, and have similar affinity and precise targeting specificity as antibodies; at the same time, due to their small molecular weight, the bicyclic peptides can be quickly and deeply infiltrated into tissues to target lesions from interiors of the tissues; and the peptide nature of the bicyclic peptides offers a "regulatable" pharmacokinetic half-life and renal clearance pathway, thereby avoiding the liver and gastrointestinal toxicity commonly found in other drug forms. However, the technical method disclosed in this document is a phage screening technology, which is limited to natural amino acids, and the compound library is greatly limited.

Therefore, it is necessary to establish in this field a ring closure method with milder ring-closure reaction conditions and higher universality, as well as other cyclic molecular structures that are not limited to peptidomimetics, in order to obtain a richer cyclic compound library and increase the diversity of the compound library. In addition, it is necessary to establish in this field a compound library and target protein binding composite technology, in order to directly screen protein-targeted researched and increase the use of the compound library.

### SUMMARY OF THE INVENTION

The first technical problem to be solved by the present invention is to provide a construction method for a cyclic compound library, which has the advantages of milder ring-closure reaction conditions, higher universality, and few side reactions, can be used for the construction of monocyclic and bicyclic compound libraries, and can overcome the limitations of the ring closure methods for the cyclic compound libraries in the prior art.

The second technical problem to be solved by the present invention is to provide a new cyclic compound library having a cyclic molecular structure, in order to increase the diversity of the compound library and expand its application range in the screening of new drugs.

To solve the above technical problems, the present invention provides a first technical solution as follows:
a construction method for a cyclic compound library, comprising the steps of:
1) linking a solid-phase carrier G to a molecule M containing a photocleavable group, directly or indirectly, to obtain G-M;
2) performing the following steps:
   a1. reacting and linking the G—M with an A-end ring-closure molecule A to obtain G-M-A;
   b1. reacting and linking the G-M-A with a linker molecule L1 having at least three functional groups to obtain G-M-A-L1;
   c1. reacting and linking the G-M-A-L1 sequentially with a starting nucleotide molecule HP and an open primer OP to obtain G-M-A-L1-HP-OP, wherein the starting nucleotide molecule HP is linked to the linker molecule L1;
   d1. reacting the resulting product from step c1 with a building block C₁ and a DNA tag tag₁ corresponding to the building block C₁, respectively, to link the building block C₁ to the L1 and to link the DNA tag tag₁ to the OP, thereby obtaining G-M-A-L1(-HP-OP-tag₁)-C₁;
   e1. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining G-M-A-L1(-HP-OP-tag₁-······-tagₙ)-C₁-······-Cₙ, wherein 2 ≤ n ≤ 7, and n is a positive integer;
   f1. reacting the resulting product from step e1 with a B-end ring-closure molecule B and a closed primer CP, to link the B-end ring-closure molecule B to the Cₙ and to link the closed primer CP to the tagₙ, thereby obtaining G-M-A-L1(-DNA)-C₁-······-Cₙ-B, i.e., a compound library S1", respectively, wherein HP-OP-tag₁-······-tagₙ-CP forms a complete DNA-encoded sequence; and
   g1. decomposing the resulting product from step f1 under a light source to cleave the M from the A, thereby obtaining A-L1(-DNA)-C₁-······-Cₙ-B, i.e., a compound library S1'; and
3) subjecting the compound library S1' to a ring-closure reaction under the action of a cyclase to react the A-end ring-closure molecule A with the B-end ring-closure molecule B for ring formation, thereby obtaining cyclic compound library S1. 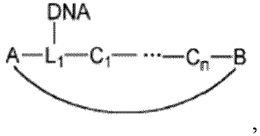 i.e., a

In the first technical solution provided by the present invention, the finally obtained cyclic compound is detached from the solid-phase carrier, and DNA encoding is the compound. The compound library obtained by this solution is suitable for the traditional DEL screening mode, and the selected compounds need to be sequenced at high throughput.

To solve the above technical problems, the present invention further provides a second technical solution as follows:
a construction method for a cyclic compound library, comprising the steps of:
1) linking a solid-phase carrier G to a molecule M containing a photocleavable group, directly or indirectly, to obtain G-M;
2) performing the following steps:
   a2. reacting and linking the G—M with a linker molecule L1 having at least three functional groups to obtain G-M-L1;
   b2. reacting the G-M-L1 sequentially with a starting nucleotide molecule HP and an open primer OP to obtain OP-HP-G-M-L1, wherein the starting nucleotide molecule HP is linked to the solid-phase carrier G;
   c2. reacting the OP-HP-G-M-L1 with a building block C₁ and a DNA tag tag₁ corresponding to the building block C₁, to link the building block C₁ to the L1 and to link the DNA tag tag₁ to the OP, thereby obtaining tag₁-OP-HP-G-M-L1-C₁;
   d2. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining tagₙ-······ -tag₁-OP-HP-G-M-L1-C₁-······-Cₙ, wherein 2 ≤ n ≤ 7, and n is a positive integer;
   e2. reacting the resulting product from the previous step with an A-end ring-closure molecule A to link the A-end ring-closure molecule A to the Cₙ;
   f2. reacting the resulting product from the previous step sequentially with building blocks Cₙ₊₁, ······, Cₙ₊ₘ and DNA tag tagₙ₊₁, ······, tagₙ₊ₘ corresponding thereto according to the extension step, to link the building block Cₙ₊₁ to the linker molecule L1 and to link the DNA tag tagₙ₊₁ to tagₙ, thereby obtaining tagₙ₊ₘ- ··· ··· -tag₁-OP-HP-G-M-L1(-C₁ ··· ··· Cₙ-A)-Cₙ₊₁······-Cₙ₊ₘ by steps e2 and f2, wherein 0 ≤ n ≤ 7, 0 ≤ m ≤ 7, n and m are both integers, and 2 ≤ n+m ≤ 7; and
   g2. reacting the resulting product from the previous step with a B-end ring-closure molecule B and a closed primer CP, to link the B-end ring-closure molecule B to the Cₙ₊ₘ and to link the closed primer CP to the tagₙ₊ₘ, thereby obtaining DNA-G-M-L1(1(-C1······ -Cₙ-A)-Cₙ₊₁······-Cₙ₊ₘ-B, i.e., a compound library S2', wherein HP-OP-tag₁-······ -tagₙ₊ₘ-CP forms a complete DNA-encoded sequence; and
3) subjecting the compound library S2' to a ring-closure reaction under the action of a cyclase to react the A-end ring-closure molecule A with the B-end ring-closure molecule B for ring formation, thereby obtaining 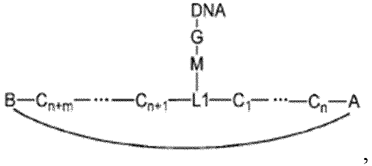 i.e., a cyclic compound library S2.

In the second technical solution, in step 2), the orders of e2 and f2 are interchangeable, to be specific:
in one mode, the resulting product tagₙ-······-tag₁-OP-HP-G-M-L1-C1-······ -Cₙ from step d2 first reacts with the A-end ring-closure molecule A to link the A-end ring-closure molecule A to the Cₙ, thereby obtaining tagₙ- ······ -tag₁-OP-HP-G-M-L1-C₁- ······ -Cₙ-A; and then, tagₙ- < -tag₁-OP-HP-G-M-L1-C₁ -······-Cₙ-A sequentially reacts with the building blocks Cₙ₊₁, ······ , Cₙ₊ₘ and the DNA tags tagₙ₊₁, ······ , tagₙ₊ₘ corresponding thereto according to the extension step, to link the building block Cₙ₊₁ to the linker molecule L1 and to link the DNA tag tagₙ₊₁ to the tagₙ, thereby obtaining tagₙ₊ₘ-······-tag₁-OP-HP-G-M-L1(-C₁-······ -Cₙ-A)-Cₙ₊₁-······-Cₙ₊ₘ; and
in the other mode, the resulting product tagₙ-······-tag₁-OP-HP-G-M-L1-C₁-······-Cₙ from step d2 first sequentially reacts with the building blocks Cₙ₊₁, ······ , Cₙ₊ₘ and the DNA tags tagₙ₊₁, ······ , tagₙ₊ₘ corresponding thereto according to the extension step, to link the building block Cₙ₊₁ to the linker molecule L1 and to link the DNA tag tagₙ₊₁ to the tagₙ, thereby obtaining tagₙ₊ₘ-······-tag₁-OP-HP-G-M-L1(-C₁-······-Cₙ)-Cₙ₊₁-······-Cₙ₊ₘ; and then, tagₙ₊ₘ-...-tag₁-OP-HP-G-M-L1(-C₁-······ -Cₙ)-Cₙ₊₁-······ -Cₙ₊ₘ reacts with the A-end ring-closure molecule A to link the A-end ring-closure molecule A to the Cₙ, thereby obtaining tagₙ₊ₘ- ··· ··· -tag₁-OP-HP-G-M-L1(-C₁- ··· ··· -Cₙ-A)-Cₙ₊₁-······-Cₙ₊ₘ.

In the second technical solution provided by the present invention, the finally obtained compound is on the solid-phase carrier, and DNA encoding is also on the solid-phase carrier, and all the synthesis steps are completed on the solid-phase carrier, such that the quality of the compound library is better guaranteed. The compound library obtained by this solution can be applied to fluorescence activated cell sorting (FACS) screening, and the selected cyclic compounds do not require high-throughput sequencing, and can be directly detached from the solid-phase carrier by means of a decomposition reaction under illumination, thereby achieving short screening period and high efficiency.

To solve the above technical problems, the present invention further provides a third technical solution as follows:
a construction method for a cyclic compound library, comprising the steps of:
1) linking a solid-phase carrier G to a molecule M containing a photocleavable group, directly or indirectly, to obtain G-M;
2) performing the following steps:
   a3. reacting and linking the G—M with a linker molecule L1 having at least four functional groups to obtain G-M-L1;
   b3. reacting the G-M-L1 sequentially with a starting nucleotide molecule HP and an open primer OP, to link the starting nucleotide molecule HP to the linker molecule L1, thereby obtaining G-M-L1-HP-OP;
   c3. reacting the G-M-LI-HP-OP with a building block C₁ and a DNA tag tag₁ corresponding to the building block C₁, to link the building block C₁ to the L1 and to link the DNA tag tag₁ to the OP, thereby obtaining G-M-L1(-HP-OP-tag₁)-C₁;
   d3. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining G-M-L1(-UP-OP-tag₁-······-tagₙ)-C₁-······-Cₙ, wherein 2 ≤ n ≤ 7, and n is a positive integer;
   e3. reacting the resulting product from the previous step with an A-end ring-closure molecule A to link the A-end ring-closure molecule A to the Cₙ;
   f3. reacting the resulting product from the previous step sequentially with building blocks Cₙ₊₁, ······, Cₙ₊ₘ and DNA tag tagₙ₊₁, ······, tagₙ₊ₘ corresponding thereto according to the extension step, to link the building block Cₙ₊₁ to the linker molecule L1 and to link the DNA tag tagₙ₊₁ to the tagₙ, thereby obtaining G-M-L1(-HP-OP-tag₁······-tagₙ₊ₘ)(-C₁······ -Cₙ-A)-Cₙ₊₁······-Cₙ₊ₘ by steps e3 and f3, wherein 0 ≤ n ≤ 7, 0 ≤ m ≤ 7, n and m are integers, and 2 ≤ n+m ≤ 7; and
   g3. reacting the resulting product from the previous step with a B-end ring-closure molecule B and a closed primer CP, to link the B-end ring-closure molecule B to the Cₙ₊ₘ and to link the closed primer CP to the tagₙ₊ₘ, thereby obtaining G-M-L1(-DNA)(-C1······ -Cₙ-A)-Cₙ₊₁······Cₙ₊ₘ-B, i.e., a compound library S3', wherein HP-OP-tag₁-······ -tagₙ₊ₘ-CP forms a complete DNA-encoded sequence; and
3) subjecting the compound library S3' to a ring-closure reaction under the action of a cyclase to react the A-end ring-closure molecule A with the B-end ring-closure molecule B for ring formation, thereby obtaining 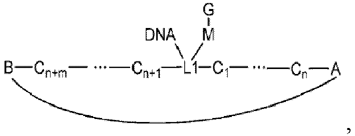 i.e., a cyclic compound library S3.

In the third technical solution, in step 2), the orders of e3 and f3 are interchangeable, to be specific:
in one mode, the resulting product G-M-L1(-UP-OP-tag₁-······-tag₁)-C₁······ -Cₙ from step d3 first reacts with the A-end ring-closure molecule A to link the A-end ring-closure molecule A to the Cₙ, thereby obtaining G-M-L1(-HP-OP-tag₁-······ -tagₙ)-C₁ ······ -Cₙ-A; and then, G-M-L1(-HP-OP-tag₁-······ -tagₙ)-C₁······ -Cₙ-A sequentially reacts with the building blocks Cₙ₊₁, ······, Cₙ₊ₘ and the DNA tags tagₙ₊₁, ··· ··· , tagₙ₊ₘ corresponding thereto according to the extension step, to link the building block Cₙ₊₁ to the linker molecule L1 and to link the DNA tag tagₙ₊₁ to the tagₙ, thereby obtaining G-M-L1(-HP-OP-tag₁······-tagₙ₊ₘ₎(-C1······-Cₙ-A)-Cₙ₊₁······-Cₙ₊ₘ; and
in the other mode, the resulting product G-M-L1(-HP-OP-tag₁-······-tagₙ) -C₁-······-Cₙ from step d3 first sequentially reacts with the building blocks Cₙ₊₁, ······ , Cₙ₊ₘ and the DNA tags tagₙ₊₁, ······ , tagₙ₊ₘ corresponding thereto according to the extension step, to link the building block Cₙ₊₁ to the linker molecule L1 and to link the DNA tag tagₙ₊₁ to the tagₙ, thereby obtaining G-M-L1(-HP-OP-tag₁ ······ -tagₙ₊ₘ)(-C1 ······ -Cₙ)-Cₙ₊₁ ······ —Cn+m; and then, G-M-L1(-HP-OP-tag₁······ -tagₙ₊ₘ)(-C1······-Cₙ)-Cₙ₊₁······-Cₙ₊ₘ reacts with the A-end ring-closure molecule A to link the A-end ring-closure molecule A to the Cₙ, thereby obtaining G-M-L1(-HP-OP-tag₁······ -tagₙ₊ₘ)(-C1······ -Cₙ-A)-Cₙ₊₁··· ···-Cₙ₊ₘ.

In the third technical solution provided by the present invention, the finally obtained compound is on the solid-phase carrier, and DNA encoding is on the solid-phase carrier, and all the synthesis steps are completed on the solid-phase carrier, such that the quality of the compound library is better guaranteed. The compound library obtained by this method can be subjected to secondary screening by cleavage of the molecule M containing the photocleavable groups. The secondary screening comprises: first performing primary screening on the solid-phase carrier, and then detaching the compound from the solid-phase carrier by cleavage of the molecule M containing the photocleavable groups, at which point DNA encoding is on library compounds, and the obtained compound library can be subjected to the secondary screening. For the secondary screening, different screening techniques can be combined and used. For example, any one or two of fluorescence activated cell sorting (FACS) screening, traditional target protein affinity screening, AS-MS screening, etc., can be combined to improve the accuracy of screening results.

In the third technical solution, G-M-L1(-DNA)(-C₁······-Cₙ-A)-Cₙ₊₁······-Cₙ₊ₘ-B obtained from step g3 can be decomposed under a light source to cleave M from L1, thereby obtaining DNA-L1(-C1······-Cₙ-A)-Cₙ₊₁······-Cₙ₊ₘ-B, i.e., a compound library S4'; the compound library S4' is subjected to the ring-closure reaction under the action of the cyclase to react the A-end ring-closure molecule A with the B-end ring-closure molecule B to form a ring, thereby obtaining 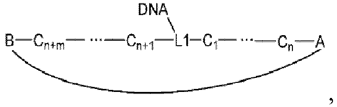 i.e., a cyclic compound library S4. Or, the compound library S3 is decomposed under a light source to cleave M from L1, thereby
obtaining 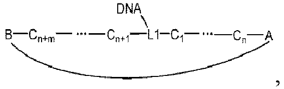 i.e., a compound library S4.

In the three technical solutions provided by the present invention, the solid-phase carrier G is selected from any one or more of the group consisting of a PEG resin, a PEGA resin, a TentaGel resin, and a solid-phase carrier CPG.

In the first and third technical solutions, the solid-phase carrier G contains one active functional group R1. The solid-phase carrier G can be represented by a general formula G0-R1, wherein R1 represents an active functional group of the solid-phase carrier G; and G0 represents a solid-phase load structure of the solid-phase carrier G other than the active functional groups. The solid-phase carrier G is directly or indirectly linked to the molecule M containing the photocleavable group via this active functional group R1. R1 can be selected from the group consisting of: amino, carboxyl, or hydroxyl. In a preferred embodiment, the solid-phase carrier G is selected from solid-phase carriers with an amino active functional group. That is, preferably, the active functional group R1 is selected from amino, for example, primary amino and secondary amino. More preferably, the active functional group R1 is selected from primary amino. The more preferred solid-phase carrier is selected from PEGAs.

In the second technical solution, the solid-phase carrier G contains two active functional groups R1 and R1'. The solid-phase carrier G can be represented by a general formula R1'-G0-R1, with R1 representing an active functional group used to link the linker molecule L1, and R1' representing an active functional group used to link the DNA-encoded sequence. In a preferred embodiment, R1 is amino and R1' is carboxyl.

In the three technical solutions provided by the present invention, the molecule M containing the photocleavable group comprises at least two active functional groups, represented by R2 and R3, respectively. The molecule M containing the photocleavable group can be represented by a general formula R2-M0-R3, with R2 and R3 representing two mutually independent active functional groups. R2 is the active functional group responsible for linking the solid-phase carrier G; and R3 is the active functional group responsible for linking the A-end ring-closure molecule A or the linker molecule L1. R2 and R3 exist independently in a form protected or unprotected by a protective group, respectively, and R2 and R3 do not interfere with each other in linkage reaction. Here, mostly, R3 does not interfere with the linkage reaction process between R2 and the solid-phase carrier G, or R3 is protected by a protective group when R2 is reacted with and linked to the solid-phase carrier G.

In a preferred embodiment, R2 exists in a form unprotected by a protective group and R3 exists in a form protected by a protective group. When R2 is linked to the solid-phase carrier G to obtain G-M, the protective group needs to be removed from R3 for subsequent reaction.

The solid-phase carrier G is linked to the molecule M containing the photocleavable group in two modes. In one mode, R2 and R1 directly undergo a complementary pairing reaction to obtain G—M. The complementary pairing reaction refers to the fact that two active functional groups react exactly to link the chemical structures, where the two active functional groups are located, in the form of a covalent bond. For example, two molecules are linked in the form of a covalent bond to form one molecule. Depending on reaction principles, R1 reacts with R2 to remove all or part of fragments. For instance, one molecule water is removed after R1 and R2 are linked. Or, R1 reacts with R2 without removing any debris. For instance, an addition reaction occurs. When directly undergoing the complementary pairing reaction, R1 and R2 can be selected from the following combinations: amino and carboxyl, amino and hydroxyl, amino and phosphate, amino and alkyl halide or aryl halide, etc.

In the other manner, R2 and R1 undergo a linkage reaction indirectly by means of other linker molecules having double functional groups, to obtain G—M. For example, the linker molecule having double functional groups has two active functional groups, where the first active group can be complementarily paired and reacted with the active functional group R1 of the solid-phase carrier G, and the second active group can be complementarily paired and reacted with the active functional group R2 of the molecule M containing the photocleavable group. The reaction process occurs in two forms. In the first form, the first active group first is reacted with and linked to the active functional group R1 of the solid-phase carrier G, and then the second active group is reacted with and linked to the active functional group R2 of the molecule M containing the photocleavable group. In the second form, the second active group first reacts with the active functional group R2 of the molecule M containing the photocleavable group, and then the first active group is reacted with and linked to the active functional group R1 of the solid-phase carrier G to obtain the indirectly linked G—M. In the indirect linkage mode, whether R2 and R1 have a complementary pairing relationship is no longer required.

In the molecule M containing the photocleavable group, the photocleavable group is preferably: 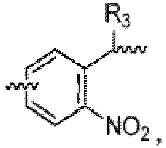 in which R3 is at a C atom that is located on a side chain ortho to nitro and linked to a benzene ring; R2 is linked to 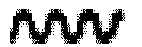 , and R2 is linked to a C atom on the benzene ring by one or more covalent bonds, or R2 is linked to the C atom, which is linked to R3, by one or more covalent bonds; and the benzene ring may comprise 0, 1, or more side chains or substituents that do not interfere with linkage reactions between R2 and R3.

Under suitable illumination conditions, the chemical bond between the C atoms linked by R3 and R3 can be cleaved in the molecule M containing the photocleavable group. The molecule M containing the photocleavable group can be cleaved at 365 nm.

In some preferred embodiments, the molecule M containing the photocleavable group may be selected from the group consisting of structures below:

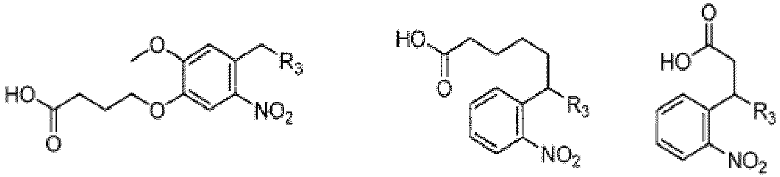

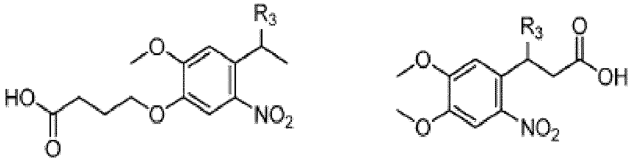

In the above structures, R3 can be selected from the group consisting of -OH, -NH₂, -NHNH₂, -N₃, Cl, and Br, etc.

In the above structure, R2 is represented by carboxyl in each case.

In some specific embodiments, the solid-phase carrier G and the molecule M containing the photocleavable group may be indirectly linked by a small molecule compound having double functional groups.

In some specific embodiments, the solid-phase carrier G has an active functional group amino, and the small molecule compound having double functional group has an active functional group carboxyl, and amino protected by a protective group. The solid-phase carrier G reacts with the small molecule compound having the double functional groups to form an amide bond for linkage; the reaction solvent is an organic solvent, such as dichloromethane, N,N-dimethylformamide; the reaction temperature is 15-30°C, preferably 20-25°C; and the reaction time is 1-12 hours, preferably 2-6 hours. Then, the amino, protected by the protective group, of the small molecule compound having the double functional groups is deprotected, and then the small molecule compound is reacted with and linked to the molecule M containing the photocleavable group. The protective group for this amino is preferably a Fmoc protective group; the reaction solvent for removing the protective group is an organic solvent, such as dichloromethane, N,N-dimethylformamide, and piperidine is added to the reaction solvent; the reaction temperature is 15-30 °C, preferably 20-25 °C; and the reaction time is 1-12 hours, preferably 1-6 hours. Then, after deprotection, the small molecule compound having the double functional groups is reacted with and linked to the molecule M containing the photocleavable group. After deprotection, the small molecule compound having the double functional groups provides an active functional group amino, and the molecule M containing the photocleavable group provides an active functional group carboxyl, which reacts with said active functional group amino to form an amide bond for linkage. The reaction solvent is an organic solvent, such as N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-30°C, preferably 20-25°C; and the reaction time is 1-12 hours, preferably 1-6 hours.

In some specific embodiments, the solid-phase carrier G and the molecule M containing the photocleavage group may be directly linked. The solid-phase carrier G has an active functional group amino, and the molecule M containing the photocleavable group provides an active functional group carboxyl, which reacts with said active functional group amino to form an amide bond for linkage. The reaction solvent is an organic solvent, such as dichloromethane and N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-30°C, preferably 20-25°C; and the reaction time is 1-12 hours, preferably 1-6 hours.

In the three technical solutions provided by the present invention, when the A-end ring-closure molecule A and the B-end ring-closure molecule B are subjected to the ring-closure reaction under the cyclase, some or all of molecular fragments are removed from the A-end ring-closure molecule A; the A-end ring-closure molecule A has two active functional groups R4 and R5; R4 is the active functional group involved in the ring-closure reaction under the cyclase, and is removed during the ring-closure reaction; R5 is the active functional group responsible for reacting and linking with the linker molecule L1 or the building block, and R5 remains in a cyclic structure of a cyclic compound molecule after the ring-closure reaction; and R4 and R5 exist independently in a form protected or unprotected by a protective group, respectively, and R4 and R5 do not interfere with each other in linkage reaction. A molecular structure of the A-end ring-closure molecule A mainly consists of two moieties: a molecular fragment A1 that is removed during the ring closure reaction under the cyclase and a molecular fragment A0 that remains on the ring during the ring-closure reaction. The A-end ring-closure molecule A may be represented by a general formula R4-A1-A0-R5. R4 is located on a molecular fragment A1 that is removed during the ring-closure reaction under the cyclase, and is also removed along with A1 during the ring-closure reaction. R5 is located on a molecular fragment A0 that remains in the ring during the ring-closure reaction, and is retained in the cyclic structure. Since R4 is first reacted and assembled with R3, it is mainly required that R5 does not interfere with the reaction process where R4 is linked with the R3 of the molecule M containing the photocleavable group, or R5 is in a form protected by a protective group when R4 is reacted with and linked to the molecule M containing the photocleavable group.

The molecular fragment A0 structurally comprises a steric hindrance group, which is directly linked to R5. The steric hindrance group is part or all of the structure of the molecular fragment A0. The steric hindrance group is a group capable of increasing the steric hindrance between M and A; and the steric hindrance group may be selected from the group consisting of: groups having carboxyl, fat chains, polyethylene glycol chains, and rigid rings. In a preferred embodiment, the steric hindrance group is an amino acid residue consisting of one or more amino acids. The preferred steric hindrance group is an amino acid residue consisting of 1-10 amino acids. Since the steric hindrance group is retained in the ring, considering the size of the ring and the steric hindrance effect, the steric hindrance group is preferably an amino acid residue consisting of 2-5 amino acids.

In a preferred embodiment, R4 exists in a form unprotected by a protective group and R5 exists in a form protected by a protective group. After R4 is linked to R3 of the molecule M containing the photocleavable group to obtain G-M-A, the protective group is removed from R5 which then undergoes a subsequent reaction. After R4 is linked to a building block, the protective group is removed from R5 which then undergoes a subsequent ring formation reaction.

In the first technical solution, R4 is an active functional group that is complementarily paired with R3 of the molecule M containing the photocleavable group, and the A-end ring-closure molecule A and the molecule M containing the photocleavable group are assembled by reacting R4 with R3, and then isolated and purified to obtain G-M-A. R4 and R3 are complementarily paired reaction groups. In some preferred embodiments, R4 and R3 may be selected from the following combinations: amino and carboxyl, hydroxyl and carboxyl, phosphate and hydroxyl, amino and alkyl halide or aryl halide, etc.

In the second and third technical solutions, R4 is an active functional group that is complementarily paired with an active functional group of the building block, and the A-end ring-closure molecule A and the building block are assembled by reacting R4 with the active functional group of the building block. In some preferred embodiments, R4 and the active functional group of the building block may be selected from the following combinations: amino and carboxyl, hydroxyl and carboxyl, phosphate and hydroxyl, amino and alkyl halide or aryl halide, etc.

In a preferred embodiment, a structure of the moiety A1 removed from the molecular structure of the A-end ring-closure molecule A is an amino acid residue consisting of one or more amino acids. This amino acid residue is linked to A0 in the molecular structure of the A-end ring-closure molecule A by means of a peptide bond. For example, in some preferred embodiments, the amino acid amino sequence of the moiety A1 removed from the molecular structure of the A-end ring-closure molecule A is: FAGDDAE(-Phe-Ala-Gly-Asp-Asp-Ala-Glu, AYDGE(-Ala-Tyr-Asp-Gly-Glu), -OCam-L, FL(-Phe-Leu), AL(-Ala-Leu), GL(-Gly-Leu), HL(-His-Leu) or HV(-His-Val) or SL(Ser-Leu).

In a preferred embodiment, the A-end ring-closure molecule A is a peptide chain consisting of at least 3 amino acids. More preferably, the A-end ring-closure molecule A is a peptide chain consisting of 3-20 amino acids.

In the first technical solution, in some specific embodiments, the molecule M containing the photocleavable group is directly reacted with and linked to the A-end ring-closure molecule A. The molecule M containing the photocleavable group provides an active functional group amino or hydroxyl, and the A-end ring-closure molecule A provides an active functional group carboxyl, which reacts with said active functional group amino or hydroxyl to form an ester or amide bond for linkage. When the amide bond is formd, the reaction solvent is an organic solvent, such as dichloromethane, N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-30°C, preferably 20-25°C; and the reaction time is 1-24 hours, preferably 12-18 hours. The other active functional group of the A-end ring-closure molecule A is amino protected by a protective group; and preferably, the A-end ring-closure molecule A is a tetrapeptide having amino protected by Fmoc. As an active functional group for reacting the A-end ring-closure molecule A with the linker molecule L1, amin needs to undergo a reaction for removing the protective group and then reacts with the linker molecule L1. The reaction solvent for removing the protective group is an organic solvent, such as dichloromethane and N,N-dimethylformamide, and piperidine is added to the reaction solvent; the reaction temperature is 15-30°C, preferably 20-25°C; and the reaction time is 1-24 hours, preferably 12-18 hours.

In the first and third technical solutions, the A-end ring-closure molecule A is directly reacted with and linked to a building block Cₙ. The reaction between the two forms an ester or amide bond for linkage. When the amide bond is formed, the reaction solvent is an organic solvent, such as dichloromethane, N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-30°C, preferably 20-25°C; and the reaction time is 1-24 hours, preferably 12-18 hours. The other active functional group of the A-end ring-closure molecule A is carboxyl protected by a protective group; and preferably, the A-end ring-closure molecule A is a tetrapeptide having carboxyl protected by allyl hydroxyl (AllO-). Between the ring formation reactions, the protective group of carboxyl should be removed from the A-end ring-closure molecule A which then undergoes ring closure under cyclase.

In the three technical solutions provided by the present invention, the linker molecule L1 is a linker molecule having at least three active functional groups, and if necessary, at least four active functional groups.

In the first technical solution, the linker molecule L1 has at least three active functional groups R6, R7 and R8. The linker molecule L1 may be represented by the following general formula: 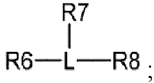 in which R6, R7 and R8 are three active functional groups. R6, R7 and R8 may exist independently in a form protected or unprotected by a protective group, respectively, and R6, R7 and R8 do not interfere with each other in linkage reaction. In the first technical solution, R6 is the active functional group complementarily paired with the active functional group R5 on the A-end ring-closure molecule A, R7 is the active functional group reacted and assembled with the starting nucleotide molecule HP, and R8 is the active functional group reacted and assembled with the building block C₁. The reaction order of the three active functional groups is as follows: first, R6 is reacted and assembled with R5, and thus, R7 and R8 are first required not to interfere with the reaction process for linking R6 to R5; second, R7 reacts with the starting nucleotide molecule HP, and thus, R8 is required not to interfere with the assembly process between R7 and the starting nucleotide molecule HP; and finally, R8 is reacted and assembled with the building block C1. In a preferred embodiment, R6 exists in a form unprotected by a protective group, and R7 and R8 each exist in a form protected by a protective group that has a different deprotection reaction mechanism. For instance, R6 is unprotected carboxyl, R7 is carboxyl protected by a protective group, and R8 is amino protected by a protective group. After R6 is linked to R5 of the A-end ring-closure molecule A to obtain G-M-A-L1, the protective group of R7 is first removed by using a deprotection reaction mechanism, and R7 is reacted and assembled with the starting nucleotide molecule HP, and at the same time, may be continuously assembled with the open primer OP; and then, the protective group of R8 is removed by using a different deprotection reaction mechanism, and R8 is reacted and assembled with the building block C1 for subsequent reactions.

In the second technical solution, the linker L1 has at least three active functional groups R6, R7 and R8. The linker molecule L1 may be represented by the following general formula: 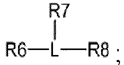 in which R6, R7 and R8 are three active functional groups. R6, R7 and R8 may exist independently in a form protected or unprotected by a protective group, respectively, and R6, R7 and R8 do not interfere with each other in linkage reaction. In the second technical solution, R6 is the active functional group that is complementarily paired with the active functional group R3 of the molecule M containing the photocleavable group, R7 is the active functional group that is reacted and assembled with the building block C₁, and R8 is the active functional group that is reacted and assembled with the building block Cₙ₊₁. The reaction order of the three active functional groups is as follows: first, R6 is reacted and assembled with the molecule M containing the photocleavable group, and thus, R7 and R8 are first required not to interfere with the linkage reaction process of R6; second, R7 reacts with the building block C₁, and thus, R8 is required not to interfere with the assembly reaction process of R7; and finally, R8 is reacted and assembled with the building block Cₙ₊₁. In a preferred embodiment, R6 exists in a form unprotected by a protective group, and R7 and R8 each exist in a form protected by a protective group that has a different deprotection reaction mechanism.

In the third technical solution, the linker molecule L1 has at least four active functional groups R6, R6', R7 and R8. The linker molecule L1 may be represented by the following general formula: 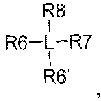 in which R6, R6', R7 and R8 are four active functional groups. R6, R6', R7 and R8 may exist independently in a form protected or unprotected by a protective group, respectively, and R6, R6', R7 and R8 do not interfere with each other in linkage reaction. R6 is the active functional group complementarily paired with the active functional group R3 of the molecule M containing the photocleavable group, R6' is the active functional group reacted and assembled with the starting nucleotide molecule HP, R7 is the active functional group reacted and assembled with the building block C₁, and R8 is the active functional group reacted and assembled with the building block Cₙ₊₁.

In the third technical solution, a preferred embodiment is as follows: the linker molecule L1 is assembled from two linker molecules L1', L1" each having three functional groups, and L1 may be represented by the following general formula: L1'-L1", in which L1' has three active functional groups R6, R6' and R6", L1" has three active functional groups R7, R8 and R8', and R6" and R8' are linked by a complementary pairing reaction.

In the third technical solution, a preferred embodiment is as follows: the linker molecule L1 comprises a decomposable functional group R_{L}, and when R_{L} is decomposed, the linker molecule L1 is split into two molecular fragments, namely, a molecular fragment comprising R6, R6' and a molecular fragment comprising R7, R8.

In the above preferred embodiment of the third technical solution provided by the present invention, the compounds in the compound library obtained after secondary screening may be subjected to secondary segmented cleavage by means of the decomposable functional group R in the linker molecule L1, to obtain the selected compounds directly, whereby the effects of short period and high efficiency are also achieved.

In some specific embodiments, the linker molecule L1 may consist of two linker molecules L1', L1'' each having three functional groups, and a linker molecule L0 linked between L1' and L1". The decomposable functional group R_{L} is located in the structure of the linker molecule L0, or is a functional group linking the linker molecule L0 and the linker molecule L1', or linking the linker molecule L0 and the linker molecule L1". The linker molecule L1 may be represented by the following general formula: L1'-L0-L1". L1' has three active functional groups R6, R6' and R6", L1" has three active functional groups R7, R8 and R8', L0 has two active functional groups R7 and R7', R7 and R6" are linked by a complementary pairing reaction, and R7' and R8' are linked by a complementary pairing reaction. That is to say, the decomposable functional group R_{L} may be a functional group formed after the complementary pairing reaction of R7 and R6", or a functional group formed after the complementary pairing reaction of R7' and R8', or an independent decomposable functional group located in the structure of the linker molecule L0.

In some specific embodiments, in the method, the decomposable functional group R_{L} is an acid cleavable group, or a photocleavable group having a different cleavage wavelength than the molecule M containing the photocleavable group. The acid cleavable group may be an ester bond, an amide bond, etc.

Specifically, the linker molecule L0 is selected from the following structures: 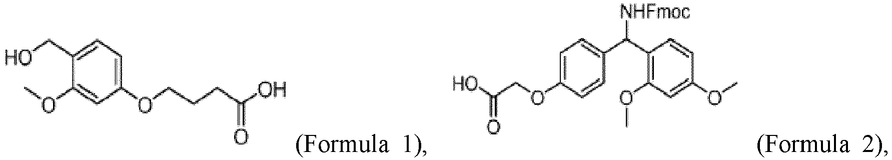 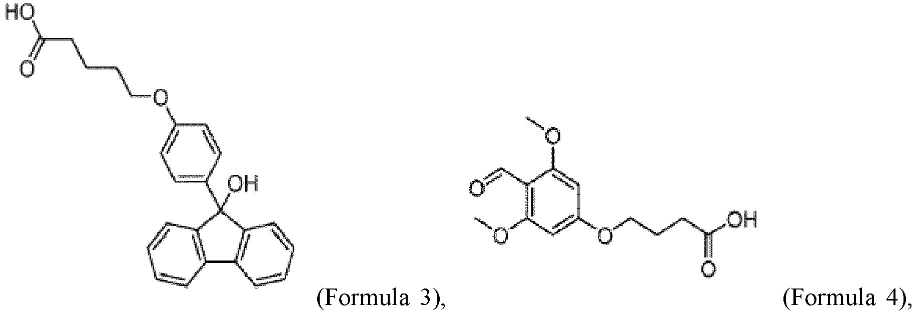 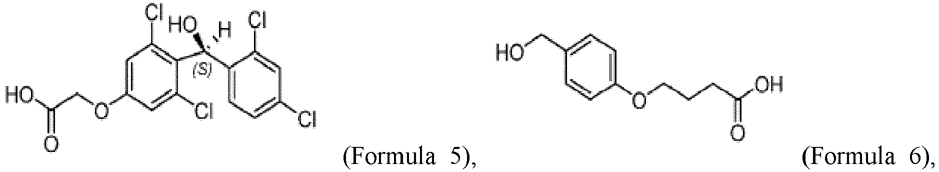 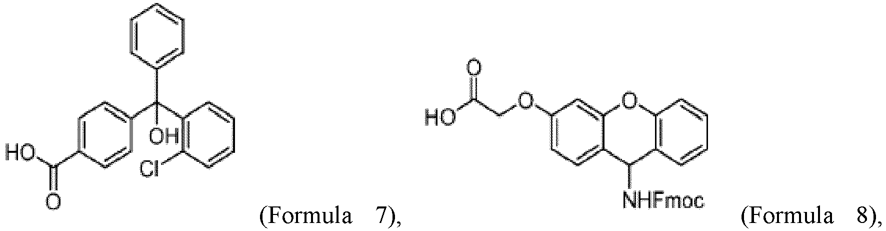 and 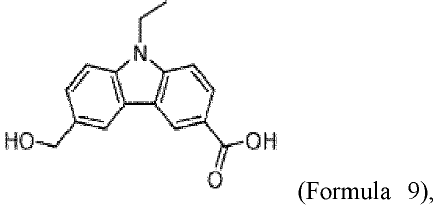 in which in the molecular structure of the decomposable linker molecule L0 shown in Formula 1-Formula 7, hydroxyl and aldehyde group may be used for reaction and assembly with the active functional groups of the linker molecules L1', L1" to form the decomposable functional group R_{L}. R_{L} may be cleaved under acidic conditions or under illumination. For example, the hydroxyl may react with the carboxyl to form an ester bond, which may be cleaved into two molecular fragments under acidic conditions; and the aldehyde may react with the amino group to form an amine bond. In the molecular structure of the decomposable linker molecule L0 shown in Formula 8-Formula 9, the hydroxyl and the amino react with the carboxyl of L1" respectively to form an ester bond and an amide bond, respectively, which are photocleavable under an illumination wavelength of 290 nm.

In some preferred embodiments, any active functional group of the linker molecule L1 and the active functional group complementarily paired with said any active functional group may be selected from the following combinations: amino and carboxyl, hydroxyl and carboxyl, phosphate and hydroxyl, amino and alkyl halide or aryl halide, etc.

In the first technical solution, in some specific embodiments, the A-end ring-closure molecule A and the linker L1 are directly reacted and linked. The active functional group R6 of the linker molecule L1 is carboxyl, and the A-end ring-closure molecule A provides an active functional group amino, which reacts with R6 to form an amide bond for linkage. The reaction solvent is an organic solvent, such as dichloromethane and N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-35°C, preferably 20-30°C; and the reaction time is 1-12 hours, preferably 1-6 hours.

In the second and third technical solutions, in some specific embodiments, the molecule M containing the photocleavable group is directly reacted with and linked to the linker molecule L1. The active functional group R6 of the linker molecule L1 is carboxyl, and the molecule M containing the photocleavable group provides an active functional group hydroxyl, which reacts with R6 to form an ester bond for linkage. The reaction solvent is an organic solvent, such as dichloromethane and N,N-dimethylformamide; one or more of N,N'-diisopropylcarbodiimide (DIC), 1-hydroxybenzotriazole (HOBt), and 4-dimethylaminopyridine (DMAP) are added to the reaction solvent; the reaction temperature is 15-35°C, preferably 20-30°C; and the reaction time is 1-12 hours, preferably 1-6 hours.

In the third technical solution provided by the present invention, the starting nucleotide molecule HP has an active functional group R9, which is complementary paired and reacts with the active functional group of the linker molecule L1, or the active functional group of the solid-phase carrier G.

In the first technical solution, the active functional group R7 of the linker molecule L1 is reacted and assembled with R9 to link the starting nucleotide molecule HP to the linker molecule L1. Further, the starting nucleotide molecule HP may be reacted with and linked to the open primer OP under the action of a DNA ligase to obtain G-M-A-L1-HP-OP, and further, the purpose of extending the DNA-encoded sequence is achieved under the action of the DNA ligase. The starting nucleotide molecule HP refers to a starting linkage end of a DNA chain in the DNA assembly technology. The open primer OP refers to an extendable DNA chain in the DNA assembly technology.

In the second technical solution, the solid-phase carrier G contains two active functional groups R1 and R1', and R1' of the solid-phase carrier G is reacted and assembled with R9 to link the starting nucleotide molecule HP to the solid-phase carrier G. Further, the starting nucleotide molecule HP may be reacted with and linked to the open primer OP under the action of a DNA ligase, and further, the purpose of extending the DNA-encoded sequence is achieved under the action of the DNA ligase.

In the third technical solution, the active functional group R6' of the linker molecule L1 is reacted and assembled with R9 to link the starting nucleotide molecule HP to the linker molecule L1. Further, the starting nucleotide molecule HP may be reacted with and linked to the open primer OP under the action of a DNA ligase, and further, the purpose of extending the DNA-encoded sequence is achieved under the action of the DNA ligase.

In some specific embodiments, the active functional group R9 of the starting nucleotide molecule HP is selected as amino, and the active functional group, complementarily paired and reacted with R9, of the linker molecule L1 or of the solid-phase carrier G is carboxyl. The carboxyl reacts with R9 (amino) to form an amide bond, thereby completing the assembly of the linker molecule L1 or solid-phase carrier G with the starting nucleotide molecule HP. The carboxyl may be under the protection of tert-butyl ester; and the carboxyl is deprotected from the tert-butyl ester under a deprotection reaction condition of 95% trifluoroacetic acid. After the protective group is removed, the resulting active functional group carboxyl reacts with the active functional group R9 (amino) of the starting nucleotide molecule HP (HDNA) to form amide bond linkage, where the carboxyl first reacts with N,N'-diisopropylcarbodiimide (DIC) and N-hydroxysuccinimide (NHS) in an organic solvent such as dichloromethane or N,N-dimethylformamide for 1-12 h, preferably 1-6h, and then reacts with the starting nucleotide molecule HP (HDNA) in an HEPES buffer solution for l-48 h, preferably 12-24 h, at the reaction temperature of 15-30°C, preferably 20-25°C.

In some specific embodiments, the starting nucleotide molecule HP is linked to the open primer OP under the action of T4 DNA ligase to extend the DNA sequence, making preparation for linkage of DNA tags.

In the first technical solution, in some specific embodiments, the active functional group R8 of the linker molecule L1 is amino protected by the Fmoc protective group. The Fmoc protective group may be removed under the condition with the foregoing piperidine-containing organic solvent, to subsequently make preparation for linkage of building blocks.

In the second and third technical solutions, the active functional groups R7 and R8 of the linker molecule L1 have protective groups with different deprotection reaction mechanisms. In some specific embodiments, R1 is carboxyl protected by allyl hydroxyl (AllO-), and R8 is amino protected by fluorene methoxycarbonyl (Fmoc).

In the three technical solutions provided by the present invention, the complete assembly of one building block with its corresponding DNA tag is referred to as one extension step. By repeating the extension step, the building blocks are linked and the DNA tags are linked, to expand the number of compounds in the compound library. Each building block has a unique DNA tag corresponding thereto, the DNA tags corresponding to different building blocks are different, and the building blocks and their corresponding DNA tags constitute a list. The extension step is repeated by arbitrarily selecting more building blocks and their corresponding DNA tags from the list, such that each building block is sequentially linked to the building block of the previous extension step, and each DNA tag is sequentially linked to the DNA tag of the previous extension step. That is, the chain of the building blocks and the chain of the DNA tags are extended, respectively.

In the first technical solution, the extension step is repeated to obtain G-M-A-L1(-HP-OP-tag1-······-tagn)-C₁-······-Cₙ. Depending on the size of the ring required, the number n of the linked building blocks and their corresponding DNA tags is determined. In general, 2 ≤ n ≤ 7 and n is a positive integer.

In the second technical solution, the extension step is repeated to obtain tagₙ-······ -tag₁-OP-HP-G-M-L1-C₁-Cₙ and tagₙ₊ₘ- ··· ··· -tag₁-OP-HP-G-M-L1(-C₁······-Cₙ-A)-Cₙ₊₁······-Cₙ₊ₘ, respectively, in which 0 ≤ n ≤ 7, 0 ≤ m ≤ 7, n and m are integers, and 2 ≤ n+m ≤ 7.

In the third technical solution, the extension step is repeated to obtain G-M-L1(-HP-OP-tag₁-tagₙ)-C₁-Cₙ and G-M-L1(-HP-OP-tag₁ ··· ··· -tagₙ₊ₘ)(-C₁...-Cₙ-A)-Cₙ₊₁······-Cₙ₊ₘ, respectively, in which 0 ≤ n ≤ 7,0 ≤ m ≤ 7, n and m are integers, and 2 ≤ n+m ≤ 7.

The building block is a small molecule compound having double active functional groups. The first and second active functional groups of the building block do not simultaneously exist in a form unprotected by a protective group, and the first active functional group and the second active functional group do not interfere with each other. The building block may also comprise a skeleton structure (for example, a skeleton structure unit), which is linked into a ring or may be linked onto a ring in the form of a side chain of the ring. The active functional groups of two adjacent building blocks are complementary. That is, two active functional groups that are complementary to each other react to form covalent bond linkage. The double active functional groups of the building block are any one independently selected from the group consisting of amino, carboxyl, aldehyde, alkyne, alkyne, halogen, azido, hydroxyl and mercapto.

In a preferred embodiment, the first active functional group is in a form protected or unprotected by a protective group, and the second active functional group is in a form protected by a protective group. When the first active functional group exists in the form protected by the protective group, the deprotection mechanism of the first active functional group is different from the deprotection mechanism of the second active functional group.

In a preferred embodiment, the first active functional group exists in a form unprotected by a protective group, and the second active functional group exists in a form protected by a protective group. In the assembly reaction of the building blocks, the first active functional group unprotected by the unprotected group is first used directly for an assembly reaction; then, the protective group of the second active functional group needs to be removed; and the assembly reaction of the building blocks is carried out in a next step.

As the extension step is repeated, the individual building blocks are sequentially assembled. In the first technical solution, each of the building blocks is sequentially assembled as follows: a first active functional group of the building block C₁ is responsible for assembly with the linker molecule L1, a first active functional group of each of the subsequent building blocks is sequentially assembled with a second active functional group of a previous building block, and a second active functional group of the building block Cₙ is responsible for assembly with the B-end ring-closure molecule B; the first active functional groups and the second active functional groups do not simultaneously exist in a form unprotected by a protective group, and the first active functional group and the second active functional group do not interfere with each other. In the second and third technical solutions, the first active functional group of the building block C₁ is responsible for assembly with an active functional group of the linker molecule L1, a first active functional group of each of the building blocks between C₁ and Cₙ and a first active functional group of the building block Cₙ are each sequentially assembled with a second active functional group of a previous building block, and a second active functional group of the building block Cₙ is responsible for assembly with the A-end ring-closure molecule A; a first active functional group of the building block Cₙ₊₁ is responsible for assembly with another active functional group of the linker molecule L1, a first active functional group of each of the building blocks between Cₙ₊₁ and Cₙ₊ₘ and a first active functional group of the building block Cₙ₊ₘ are each sequentially assembled with a second active functional group of a previous building block, and a second active functional group of the building block Cₙ₊ₘ is responsible for assembly with the B-end ring-closure molecule B; and the first active functional groups and the second active functional groups do not simultaneously exist in a form unprotected by a protective group, and the first active functional groups and the second active functional groups do not interfere with each other.

In a preferred embodiment, the adjacent building blocks are linked by the following chemical bonds: an amide bond, or an ester bond.

In a preferred embodiment, the first active functional group of each building block is the same active functional group, for example, carboxyl; the second active functional group of each building block is also the same active functional group, for example, amino; and the first active functional group is not the same as the second active functional group.

In a preferred embodiment, the double functional groups of the building block are carboxyl and amino, respectively, and the carboxyl and the amino independently exist in a form protected or unprotected by a protective group, respectively.

In a preferred embodiment, the building block is a compound having double active functional groups comprising both amino and carboxyl. In some embodiments, the amino and carboxyl in the building block are linked to the same carbon atom, such as α-amino acid. In other embodiments, the amino and carboxyl in the building block are linked to different atoms, which may be non-amino acid compounds having both amino and carboxyl, or N-substituted amino acids. In other embodiments, the building block is selected from the group consisting of substituted or unsubstituted dicarboxylic acid, substituted or unsubstituted diamine, substituted or unsubstituted glycol, substituted or unsubstituted olefin, substituted or unsubstituted alkyne, and substituted or unsubstituted aldehyde.

In a preferred embodiment, the first active functional group is carboxyl, which exists in a form unprotected by a protective group.

In a preferred embodiment, the first active functional group is amino, which exists in a form protected by a protective group, for which an Fmoc protection group is used.

In a preferred embodiment, the building block is an Fmoc- amino acid.

In a preferred embodiment, at least one of the individual building blocks comprises a skeleton structure, which has an E3 ligase substrate structure and can bind to an E3 ligase. The compound library having an E3 ligase substrate structure can be applied in PROTAC to enable multipurpose screening for the library.

In some specific embodiments, the skeleton structure is selected from:

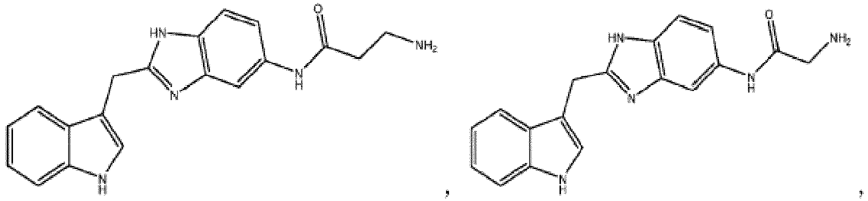

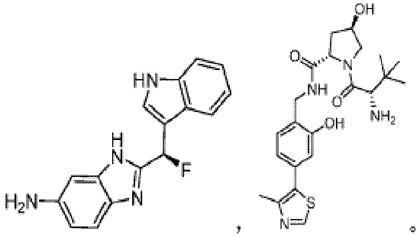

In a preferred embodiment, each of the building blocks comprises at least one extra-ring side chain in total. The extra-ring side chain provides an extended function for further derivatization of library compounds, in order to expand the diversity of the compound library.

In a preferred embodiment, each of the building blocks comprises at least two extra-ring side chains in total, and the at least two extra-ring side chains are linked by means of a chemical reaction to form a bicyclic structure. The ring-closure reaction of the two extra-ring side chains may be performed before or after the ring-closure reaction under cyclase. The method of forming a bicyclic structure by two extra-ring side chains can be performed in three technical solutions provided by the present invention.

The DNA tags are sequentially linked to each other by the DNA ligase. In one extension step, an assembly reaction is completed for one DNA tag and the building block corresponding to the DNA tag, such that the chain of the building blocks and the chain of the DNA tags are extended, respectively. In one extension step, the assembly reaction of the DNA tags may be first performed to extend the chain of the DNA tags, and then the assembly reaction of the building blocks corresponding to the DNA tags may be performed to extend the chain of the building blocks. Or, the assembly reaction with the building blocks is first performed to extend the chain of the building blocks, and then the assembly reaction of the DNA tags corresponding to the building blocks is performed to extend the chain of the DNA tags.

In the three technical solutions provided by the present invention, when the assembly of the DNA tags is completed, the closed primer CP is linked by a DNA ligase, thereby forming a complete DNA-encoded sequence. The closed primer CP refers to a end chain in which DNA stops extending in the DNA assembly technology.

In the three technical solutions provided by the present invention, the B-end ring-closure molecule B is a compound having double active functional groups. The B-end ring-closure molecule B may be represented by a general formula R10-B0-R11. R10 is an active functional group used for reaction and assembly with the second active functional group of the last building block, and R11 is an active functional group used for the ring-closure reaction. R10 and R11 may exist independently in a form protected or unprotected by a protective group, respectively, and R10 and R11 do not interfere with each other in linkage reaction. Since R10 is first assembled with the second active functional group of the last building block, R11 is mainly required not to interfere with the reaction process of linking R10 to the second active functional group of the last building block, or R1 1 is in a form protected by the protective group when R10 is reacted with and linked to the second active functional group of the last building block.

In a preferred embodiment, R10 exists in a form unprotected by a protective group and R11 exists in a form protected by a protective group. R10 first reacts with the second active functional group of the last building block to complete assembly. When R11 is used for the ring-closure reaction, the protective group of R11 is removed before the ring-closure reaction is performed.

R10 and the second active functional group of the last building block are complementarily paired reaction groups. In some preferred embodiments, R10 and the second active functional group of the last building block may be selected from the following combinations: amino and carboxyl, hydroxyl and carboxyl, phosphate and hydroxyl, amino and alkyl halide or aryl halide, etc.

In a new preferred embodiment, one of the compounds R10 and R11 of the two active functional groups of the B-end ring-closure molecule B is amino, and the other is carboxyl.

In the first technical solution, the closed primer OP and the B-end ring-closure molecule B are assembled, isolated and purified to obtain a compound G-M-A-L1(-DNA)-C₁-······ -C₁-B, which in itself may constitute a compound library S1". The compound library S1" contains the solid-phase carrier G, but has no cyclic structure formed as desired. R10 reacts with the second active functional group of Cₙ, and the other active functional group R11 is at a free end of the B-end ring-closure molecule B.

In the second technical solution, the closed primer OP and the B-end ring-closure molecule B are assembled, isolated and purified to obtain DNA-G-M-L1(-C₁······-Cₙ₋A)-Cₙ₊₁ ······-Cₙ₊ₘ-_{B}, i.e., a compound library S2'. R10 reacts with the second active functional group of Cₙ, and the other active functional group R11 is at a free end of the B-end ring-closure molecule B.

In the third technical solution, the closed primer OP and the B-end ring-closure molecule B are assembled, isolated and purified to obtain G-M-L1(-DNA)(-C₁······-Cₙ-A)-Cₙ₊₁······ -Cₙ₊ₘ-B, i.e., a compound library S3'. R10 reacts with the second active functional group of Cₙ, and the other active functional group R11 is at a free end of the B-end ring-closure molecule B.

In a preferred embodiment, R11 is located at the free end of the B-end ring-closure molecule B, and R11 is amino. Preferably, the active functional group R11 at the free end of the B-end ring-closure molecule B is primary amino.

In a preferred embodiment, the B-end ring-closure molecule B is a peptide chain consisting of 2-10 amino acids.

In some specific embodiments, the B-end ring-closure molecule B is a dipeptide protected by Fmoc.

In some specific embodiments, the molecular structure of the B-end ring-closure molecule B has amino acid residues GL(Gly—Leu—), LL(Leu—Leu—), QL(Gln—Leu—), KL(Lys—Leu—), GF(Gly-Phe-), Gl(Gly-Ile-), FSA, VGAG, GV, GF, and GM.

Among the technical solutions provided by the present invention, in the first technical solution, the linkage between M and A in G-M-A-L1(-DNA)-C1-······-Cₙ-B is cleaved and split under the illumination condition to obtain a compound A-L1(-DNA)-C₁-······-Cₙ-B, which in itself may constitute a compound library S1'. The compound library S1' does not contain the solid-phase carrier G, and has no cyclic structure formed as desired.

In the technical solution provided by the present invention, the compound library S1', S2' or S3' is subjected to a ring-closure reaction under the action of a cyclase to react the A-end ring-closure molecule A with the B-end ring-closure molecule B to form a peptide bond for ring formation. In a ring formation reaction, a free-end active functional group of the B-end ring-closure molecule B reacts with the A-end ring-closure molecule A to remove part or all of the molecular fragments from the A-end ring-closure molecule A, and a free-end active functional group of the B-end ring-closure molecule B is linked to a remaining part after removal to form a cyclic structure.

The cyclase described in the present invention refers to an enzyme that allows two end amino acid residues in a molecule to react to form a peptide bond for linkage. Preferably, the cyclase is a cyclic peptide synthetase, which catalyzes a dehydration reaction between α amino at the N-end of one termina amino acid residue and α carboxyl at the N-end of the other end amino acid residue in the molecule to form a cyclic polypeptide.

The cyclase is selected from a ligase VyPAL2, Butelase1, PatG, PagG, ominiligase-1, PCY1, or OaAEP1B&3-5.

Butelase1 is a specific aspartate/aminoacyl ligase, which can efficiently catalyze the intramolecular or intermolecular cyclization of a linear polypeptide with an Asx(Asp/Asn)-His-Val tripeptide sequence at the carboxyl end. Butelase1 cuts off a -His-Val dipeptide at the carboxyl end of the linear polypeptide, allowing the linear polypeptide to link to an amino end to form a cyclic structure.

In some specific embodiments, the ring-closure reaction under the cyclase occurs at a temperature of 25-45°C, preferably 30-45°C, and more preferably 35-40°C.

In some specific embodiments, the ring-closure reaction under the cyclase occurs in a pH range of 4.5-6.0, preferably 4.8-5.5, and more preferably 4.9-5.3.

In some specific embodiments, the ring-closure reaction under the cyclase has a reaction time of 12-48 hours, preferably 18-36 hours, and more preferably 20-24 hours.

In some specific embodiments, in the ring-closure reaction under the cyclase, a pH value is adjusted by using a sodium acetate buffer solution.

In some specific embodiments, disodium ethylenediamine tetraacetate or its solution (0.05M) is added to the ring-closure reaction system under the cyclase.

In some specific embodiments, sodium chloride or its solution (0.25M) is added to the ring-closure reaction system under the cyclase.

In some specific embodiments, TCEP is added as a reducing agent to the ring-closure reaction system under the cyclase.

According to the present invention, the linker molecule L1 is used to provide at least three active functional groups for linking 1) the DNA-encoded sequence, 2) the A-end ring-closure molecule A, and 3) the building blocks and the B-end ring-closure molecule B, respectively, and the amino acid residue structures of the A-end ring-closure molecule A and the B-end ring-closure molecule B are used to complete ring closure under the action of cyclase, showing mild ring closure conditions and higher universality, which expands the chemical reaction types and the diversity space for the encoding compound library.

The present invention provides a construction method for a cyclic compound library, the number and type of atoms provided by each building block in the cyclic structure may be diversified and adjusted, and the individual building blocks may be assembled using an amide bond (—CO—NH—) to provide more hydrogen bond binding potential for the cyclic structure, which is beneficial to improve the binding force between the cyclic structure and proteins.

The present invention provides a method for cutting under illumination, which has mild reaction conditions and effectively prevents the stability of DNA-encoded sequences from being adversely affected by the solid-phase removal synthesis under other harsh conditions.

The present invention provides a synthesis method for a DNA-encoded compound loaded with a solid-phase carrier, whereby the post-processing and purification operations after the reaction are simple and only several simple filtrating and washing operations are needed, which simplifies the post-processing, isolation and purification procedures of each step of the reaction, such that the synthesis period of the DNA-encoded compound library is significantly shortened, and the cost is greatly saved.

According to the synthesis method for the compound library in the present invention, the linker molecule for linking the DNA-encoded sequence to the compound library is first linked to the compound library, and then linked to the DNA-encoded sequence, and the ratio of the starting nucleotide molecule HP to the docking compound during synthesis is about 1:250, such that the DNA use level is greatly reduced, and the cost is saved.

In the synthesis method for the compound library in the present invention, it is equivalent that, after each step of DNA tag assembly, 249 residual unlinked DNAs need to be removed; one of the double active functional groups of each building block has a protective group, which needs to be removed during one alternate linkage reaction between the building block and its corresponding DNA tag; and as the protective group is removed, the reaction point of the compound library of unlinked DNAs can be closed, which is useful to remove the excess DNAs and reduce the interference to achieve a purification effect and improve the efficiency and uniqueness of DNA encoding and the purity of a final product.

The present invention further provides a construction method for a bicyclic-structure compound library, where the linker molecule L1 in the aforementioned three technical solutions has at least five functional groups; one functional group of linker molecule L1 is used to directly or indirectly link the DNA-encoded sequence, and the other four functional groups of the linker molecule L1 are linked to two A-end ring-closure molecules A, A' and two B-end ring-closure molecules B, B', respectively, with or without linkage to the building blocks; and finally, two ring-closure reactions are carried out respectively under the actions of cyclases 1 and 2, such that the two pairs, namely, the pair of A-end ring-closure molecule A and B-end ring-closure molecule B and the pair of A-end ring-closure molecule A' and B-end ring-closure molecule B', are subjected to ring closure respectively to obtain a bicyclic-structure compound library.

The corresponding relationship between the cyclases and the ring-closure A-end and B-end is as follows: when the cyclase is OaAEP1B&3-5, the A-end can be selected from: NFL, NAL, NGL, NHL, DFL, DAL, DGL, and DHL, and the B-end can be selected from: GL(Gly-Leu-), LL(Leu-Leu-), QL(Gln-Leu-), KL(Lys-Leu-), and GF(Gly-Phe-); when the cyclase is VyPAL2, the A-end can be selected from NSL, and the B-end can be selected from GI; when the cyclase is Butelase1, the A-end can be selected from NHV, and the B-end can be selected from GI; when the cyclase is PatG, the A-end can be selected from AYDGE, and the B-end can be selected from P or thiazoline ring; when the cyclase is PagG, the A-end can be selected from FAGDDAE; when the cyclase is ominiligase-1, the A-end can be selected from OCam-Leu and OCam-; when the cyclase is PCY1, the A-end can be selected from FQA and IQT, and the B-end can be selected from FSA and VGAG. Specifically, in the aforementioned three technical solutions, step f2 or f3 is performed twice to link one A-end ring-closure molecule A and one B-end ring-closure molecule B respectively before the reaction with the closed primer CP.

Taking the aforementioned first technical solution as an example, the construction method for the bicyclic-structure compound library includes:
the linker molecule L1 is a linker molecule having at least five functional groups;
after step el, the following steps are performed:
   e1-1, reacting the resulting product from step el with the B-end ring-closure molecule B to link the B-end ring-closure molecule B to Cₙ and link tags to tagₙ;
   e1-2. reacting the resulting product from the previous step sequentially with building blocks Cₙ₊₁, ······, Cₙ₊ₘ and DNA tag tagₙ₊₁, ······,tagₙ₊ₘ corresponding thereto according to the extension step, to link the building block Cₙ₊₁ to L1 and to link the DNA tag tagₙ₊₁ to the tags, thereby obtaining G-M-A-L1(-HP-OP-tag₁- ··· ··· -tagₙ₊ₘ)(-C₁- ··· ··· -Cₙ-B)-Cₙ₊₁ ······-Cₙ₊ₘ;
   e1-3. reacting the resulting product from the previous step with the A-end ring-closure molecule A' to link the A-end ring-closure molecule A' to the Cₙ₊ₘ and link tag_{A'} to tagₙ₊ₘ;
   e1-4. reacting the resulting product from the previous step sequentially with the building blocks Cₙ₊ₘ₊₁, ······,Cₙ₊ₘ₊ₓ and the DNA tags tagₙ₊ₘ₊₁, ······, tagₙ₊ₘ₊ₓ corresponding thereto according to the extension step, to link the building block Cₙ₊₁ to L1 and link the DNA tag tagₙ₊ₘ₊₁ to tag_{A'}, thereby obtaining G-M-A1-L1(-HP-OP-tag₁- ...... -tagₙ₊ₘ₊ₓ)(-C₁-······-Cₙ-B)(-Cₙ₊₁ ······-Cₙ₊ₘ-A')-Cₙ₊ₘ₊₁- ······-Cₙ₊ₘ₊ₓ;
   e1-5. reacting the resulting product from the previous step with the B-end ring-closure molecule B' and tagB' to link the B-end ring-closure molecule B' with Cₙ₊ₘ₊ₓ and link tag_{B'} to tagn+m+x;
   e1-6. reacting the resulting product from the previous step with the closed primer CP to link the closed primer CP to tagB' to obtain G-M-A-L1(-DNA)(-C₁ ··· ··· -Cₙ-B)(-Cₙ₊₁- ··· ··· -Cₙ₊ₘ-A')-Cₙ₊ₘ₊₁ ··· ··· -Cₙ₊ₘ₊ₓ-B', wherein HP-OP-tag₁-...-tagₙ-tag_{B}-tagₙ₊₁-...-tagₙ₊ₘ-tag_{A'}-tagₙ₊ₘ₊₁-...-tagₙ₊ₘ₊ₓ-tag_{B'} —CP forms a completed DNA-encoded sequence;
   e1-7. decomposing the resulting product from the previous step under a light source to cleave M from A to obtain A-L1(-DNA)(-C₁ ······Cₙ-B)(-Cₙ₊₁ ······Cₙ₊ₘ-A')-Cₙ₊ₘ₊₁ ··· ···-Cₙ₊ₘ₊ₓ-B', wherein 0 ≤ n ≤ 7, 0 ≤ m ≤ 7, n, m and x are integers, and 2 ≤ n + m + x ≤ 7;
   e1-8. subjecting the resulting product from the previous step to a ring-closure reaction under the action of a cyclase 1 to react the A-end ring-closure molecule A with the ring B-end ring-closure molecule B to form a ring, thereby obtaining 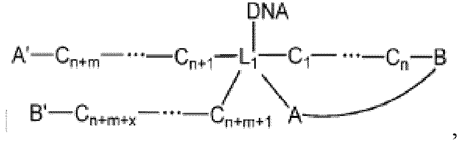
   e1-9. subjecting the resulting product from the previous step to a ring-closure reaction under the action of a cyclase 2 to allow the two pairs of A-end ring-closure molecule A' and B-end ring-closure molecule B' to react to form a ring, thereby obtaining 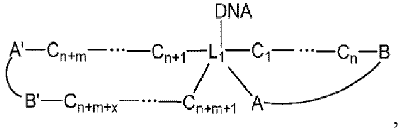 i.e., a bicyclic compound library.

**Taking the aforementioned second technical solution as an example,** the construction method for the bicyclic-structure compound library includes:
the linker molecule L1 is a linker molecule having at least five functional groups;
after step e2, the following steps are performed:
   e2-1. reacting the resulting product from step e2 and a DNA tag tag_{A} corresponding to an A-end of the ring-closure molecule to tagₙ;
   e2-2. reacting the resulting product from step e2-1 with the B-end ring-closure molecule B to link the B-end ring-closure molecule B to Cₙ₊ₘ, and linking tags at a corresponding DNA tag position to obtain tag_{B}-tagₙ₊ₘ- ······ -tag₁-OP-HP-G-M-L1(-C₁ ······-Cₙ₋A)-Cₙ₊₁ ······-Cₙ₊ₘ-B;
   e2-3. reacting the resulting product from the previous step sequentially with the building blocks Cₙ₊ₘ₊₁, ······,Cₙ₊ₘ₊ₓ and the DNA tags tagₙ₊ₘ₊₁, ······, tagₙ₊ₘ₊ₓ corresponding thereto according to the extension step, to link the building block Cₙ₊ₘ₊₁ to L1 and link the DNA tag tagₙ₊ₘ₊₁ to tag_{B}, thereby obtaining tagₙ₊ₘ₊ₓ- ······ -tag₁-OP-HP-G-M-L1(-C₁ ······ -Cₙ-A)(-Cₙ₊₁ ······-Cₙ₊ₘ-B)-Cₙ₊ₘ₊₁ ······-Cₙ₊ₘ₊ₓ;
   e2-4. reacting the resulting product from the previous step with an A-end ring-closure molecule A' to link the A-end ring-closure molecule A' to the Cₙ₊ₘ₊ₓ, and linking tag_{A'} at a corresponding DNA tag position;
   e2-5. reacting the resulting product from the previous step sequentially with the building blocks Cₙ₊ₘ₊ₓ₊₁, ······,C_{n+m+x+y} and the DNA tags tagₙ₊ₘ₊ₓ₊₁, ······ tag_{n+m+x+y} corresponding thereto according to the extension step, to link the building block Cₙ₊ₘ₊ₓ₊₁ to L1 and link the DNA tag tagₙ₊ₘ₊ₓ₊₁ to tag_{A'}, thereby obtaining tag_{n+m+x+y}-······-tag₁-OP-HP-G-M-L1(-C₁······ -Cₙ-A)(-Cₙ₊₁······-Cₙ₊ₘ-B)(-Cₙ₊ₘ₊₁······-Cₙ₊ₘ₊ₓ-A')-Cₙ₊ₘ₊ₓ₊₁······C_{n+m+x+y};
   e2-6. reacting the resulting product from the previous step with the B-end ring-closure molecule B and the DNA tag tag_{B'} to link the B-end ring-closure molecule B' to C_{n+m+x+y} and link tag_{B'} to tag_{n+m+x+y};
   e2-7. reacting the resulting product from the previous step with the closed primer CP to link the closed primer CP to tagB' to obtain DNA-G-M-L1(-C₁······-Cₙ-A)(-Cₙ₊₁······ -Cₙ₊ₘ-B)(-Cₙ₊ₘ₊₁ ··· ··· -Cₙ₊ₘ₊ₓ-A')-Cₙ₊ₘ₊ₓ₊₁ ··· ··· -C_{n+m+x+y}-B', wherein UP-OP-tag₁-······-tag_{n+m+x+y}-tag_{B'}-CP forms a complete DNA-encoded sequence, and wherein 0 ≤ n ≤ 7, 0 ≤ m ≤ 7, 0 ≤ x ≤ 7, 0 ≤ y ≤ 7, n, m, x and y are integers, and 2 ≤ n+m+x+y ≤ 7;
   e2-8. subjecting the resulting product from the previous step to a ring-closure reaction under the action of a cyclase 1 to react the A-end ring-closure molecule A with the ring B-end ring-closure molecule B to form a ring, thereby obtaining 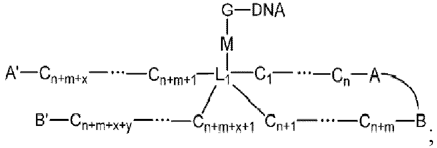 and
   e2-9. subjecting the resulting product from the previous step to a ring-closure reaction under the action of a cyclase 2 to react the A-end ring-closure molecule A' with the B-end ring-closure molecule B' to form a ring, thereby obtaining 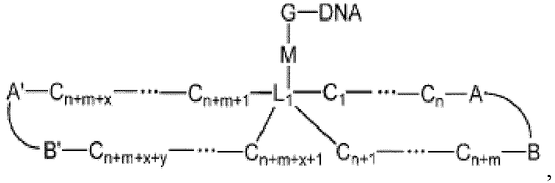 i.e., a bicyclic compound library.

**Taking the aforementioned third technical solution as an example,** the construction method for the bicyclic-structure compound library includes:
the linker molecule L1 is a linker molecule having at least six functional groups;
after step e3, the following steps are performed:
   e3-1. reacting the resulting product from step e3 and a DNA tag tag_{A} corresponding to an A-end of the ring-closure molecule to tagₙ;
   e3-2. reacting the resulting product from step e3-1 with the B-end ring-closure molecule B to link the B-end ring-closure molecule B to Cₙ₊ₘ, and linking tag_{B} at a corresponding DNA tag position to obtain G-M-L1(-HP-OP-tag₁······-tagₙ₊ₘ-tag_{B})(-C1······-Cₙ-A)-Cₙ₊₁ ······-Cₙ₊ₘ-B;
   e3-3. reacting the resulting product from the previous step sequentially with the building blocks Cₙ₊ₘ₊₁, ······,Cₙ₊ₘ₊ₓ and the DNA tags tagₙ₊ₘ₊₁, ······, tagₙ₊ₘ₊ₓ corresponding thereto according to the extension step, to link the building block Cₙ₊ₘ₊₁ to L1 and link the DNA tag tagₙ₊ₘ₊₁ to tag_{B}, thereby obtaining G-M-L1(-HP-OP-tag₁······-tagₙ₊ₘ₊ₓ)(-C₁······ -Cₙ-A)(-Cₙ₊₁······Cₙ₊ₘ-B)-Cₙ₊ₘ₊₁······-Cₙ₊ₘ₊ₓ;
   e3-4. reacting the resulting product from the previous step with an A-end ring-closure molecule A' to link the A-end ring-closure molecule A' to the Cₙ₊ₘ₊ₓ, and linking tag_{A'} at a corresponding DNA tag position;
   e3-5. reacting the resulting product from the previous step sequentially with the building blocks Cₙ₊ₘ₊ₓ₊₁, ······,C_{n+m+x+y} and the DNA tags tagₙ₊ₘ₊ₓ₊₁, ······, tag_{n+m+x+y} corresponding thereto according to the extension step, to link the building block Cₙ₊ₘ₊ₓ₊₁ to L1 and link the DNA tag tagₙ₊ₘ₊ₓ₊₁ to tag_{A'}, thereby obtaining G-M-L1(-HP-OP-tag₁······-tag_{n+m+x+y})(-C1······ -Cₙ-A)(-Cₙ₊₁······-Cₙ₊ₘ-B)(-Cₙ₊ₘ₊₁······-Cₙ₊ₘ₊ₓ-A')-Cₙ₊ₘ₊ₓ₊₁······-C_{n+m+x+y}
   e3-6. reacting the resulting product from the previous step with the B-end ring-closure molecule B' and the DNA tag tag_{B'} to link the B-end ring-closure molecule B' to C_{n+m+x+y} and link the DNA tag tag_{B'} to tag_{n+m+x+y};
   e3-7. reacting the resulting product from the previous step with the closed primer CP to link the closed primer CP to tag_{B'} to obtain G-M-L1(-DNA)(-C1······-Cₙ-A)(-Cₙ₊₁······ -Cₙ₊ₘ-B)(Cₙ₊ₘ₊₁······-Cₙ₊ₘ₊ₓ-A')-Cₙ₊ₘ₊ₓ₊₁······-C_{n+m+x+y}-B', wherein HP-OP-tag₁ ······-tag_{n+m+x+y}-tag_{B'}-CP forms a complete DNA-encoded sequence, and wherein 0 ≤ n ≤ 7, 0 ≤ m ≤ 7, 0 ≤ x ≤ 7, 0 ≤ y ≤ 7, n, m, x and y are integers, and 2 ≤ n+m+x+y ≤ 7;
   e3-8. subjecting the resulting product from the previous step to a ring-closure reaction under the action of a cyclase 1 to react the A-end ring-closure molecule A1 with the ring B-end ring-closure molecule B 1 to form a ring, thereby obtaining 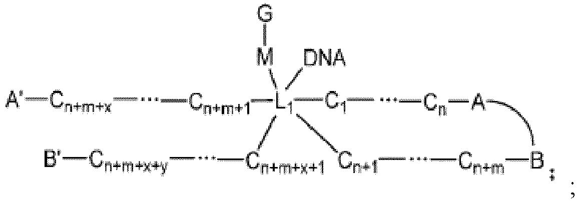 and
   e3-9. subjecting the resulting product from the previous step to a ring-closure reaction under the action of a cyclase 2 to react the A-end ring-closure molecule A2 with the B-end ring-closure molecule B2 to form a ring, thereby obtaining 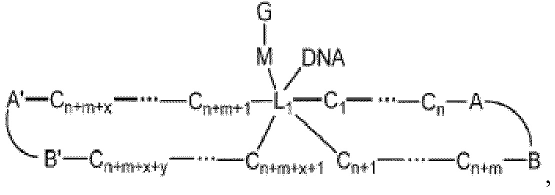 i.e., a bicyclic compound library.

In a preferred embodiment, the linker molecule L1 having at least five functional groups is assembled from three linker molecules each having three functional groups.

In a preferred embodiment, the linker molecule L1 having at least five functional groups is assembled from one linker molecule having four functional groups and one linker molecule having three functional groups.

The present invention further provides a cyclic compound library constructed by the aforementioned method, with the structural formula of: 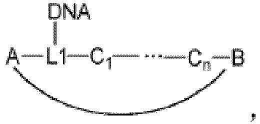
wherein 2 ≤ n ≤ 7 and n is a positive integer,
L1 is a linker molecule having at least three functional groups, and a DNA-encoded sequence is linked to the L1 by means of an amide bond; C1 to Cₙ are building blocks that are linked end to end and each have double active functional groups; A represents an A-end ring-closure molecule, which is an amino acid residue; B represents a B-end ring-closure molecule, which is an amino acid residue; the L1 and the A are linked by means of an amide or ester bond; the building block Cₙ and the B are linked by means of an amide or ester bond; and the A and the B react under the action of a cyclase to form a peptide bond and are then linked to form a ring.

The present invention further provides a second cyclic compound library constructed by the aforementioned method, with the structural formula of: 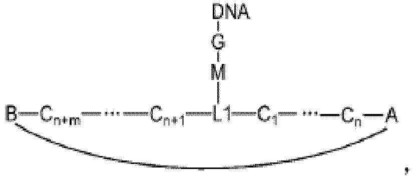
wherein 0 ≤ n ≤ 7, 0 ≤ m ≤ 7, n and m are integers, and 2 ≤ n+m ≤ 7;
L1 is a linker molecule having at least three functional groups, and a DNA-encoded sequence is linked to a solid-phase carrier G by means of an amide bond; C₁ to Cₙ are building blocks that are linked end to end and each have double active functional groups, and Cₙ₊₁ to Cₙ₊ₘ are building blocks that are linked end to end and each have double active functional groups; G represents a solid-phase carrier, and M represents a molecule containing a photocleavable group; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

The present invention further provides a third cyclic compound library constructed by the aforementioned method, with the structural formula of: 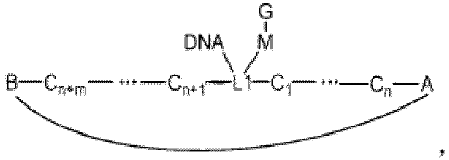
wherein 0 ≤ n ≤ 7, 0 ≤ m ≤ 7, n and m are integers, and 2 ≤ n+m ≤ 7;
L1 is a linker molecule having at least four functional groups, and a DNA-encoded sequence is linked to the L1 by means of an amide bond; C₁ to Cₙ are building blocks that are linked end to end and each have double active functional groups, and Cₙ₊₁ to Cₙ₊ₘ are building blocks that are linked end to end and each have double active functional groups; G represents a solid-phase carrier, and M represents a molecule containing a photocleavable group; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

The present invention further provides a fourth cyclic compound library constructed by the aforementioned method, with the structural formula of: 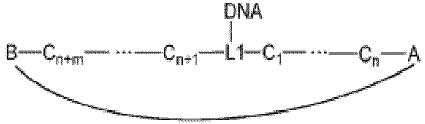
wherein 0 ≤ n ≤ 7, 0 ≤ m ≤ 7, n and m are integers, and 2 ≤ n+m ≤ 7;
L1 is a linker molecule having at least four functional groups, and a DNA-encoded sequence is linked to the L1 by means of an amide bond; C₁ to Cₙ are building blocks that are linked end to end and each have double active functional groups, and Cₙ₊₁ to Cₙ₊ₘ are building blocks that are linked end to end and each have double active functional groups; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

The present invention further provides a first bicyclic-structure compound library constructed by the aforementioned method, with the general structural formula of: 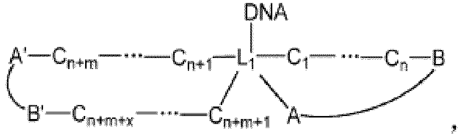
wherein 0 ≤ n ≤ 7, 0 ≤ m ≤ 7, 0 ≤ x ≤ 7, n, m and x are all integers, and 2 ≤ n+m+x ≤ 7;
L1 is a linker molecule having at least five functional groups, and a DNA-encoded sequence is linked to the L1 by means of an amide bond; C₁ to Cₙ are building blocks that are linked end to end and each have double active functional groups, Cₙ₊₁ to Cₙ₊ₘ are building blocks that are linked end to end and each have double active functional groups, and Cₙ₊ₘ₊₁ to Cₙ₊ₘ₊ₓ are building blocks that are linked end to end and each have double active functional groups; A and B represent an A-end ring-closure molecule and a B-end ring-closure molecule which are subjected to ring closure under the action of a cyclase, respectively, A' and B' represent an A-end ring-closure molecule and a B-end ring-closure molecule which are subjected to ring closure under the action of another different cyclase, respectively, and A, B, A' and B' are all amino acid residues; the building block Cₙ and the B are linked by means of an amide or ester bond; the building block Cₙ₊ₘ and the A' are linked by means of an amide or ester bond; the building block Cₙ₊ₘ₊ₓ and the B' are linked by means of an amide or ester bond; and the A and the B, or the A' and the B', react under the action of a cyclase to form a peptide bond and are then linked to form a bicyclic structure.

The present invention further provides a second bicyclic-structure compound library constructed by the aforementioned method, with the general structural formula of: 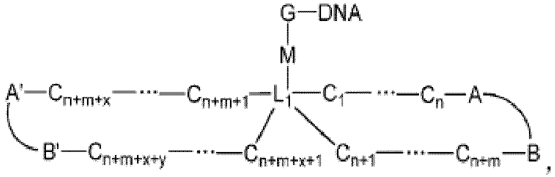
wherein 0 ≤ n ≤ 7, 0 ≤ m ≤ 7, 0≤x≤7,0≤y≤7, n, m, x and y are all integers, and 2 ≤ n+m+x+y ≤ 7;
L1 is a linker molecule having at least five functional groups, and a DNA-encoded sequence is linked to the solid-phase carrier G by means of an amide bond; C₁ to Cₙ are building blocks that are linked end to end and each have double active functional groups, Cₙ₊₁ to Cₙ₊ₘ are building blocks that are linked end to end and each have double active functional groups, Cₙ₊ₘ₊₁ to Cₙ₊ₘ₊ₓ are building blocks that are linked end to end and each have double active functional groups, and Cₙ₊ₘ₊ₓ₊₁ to C_{n+m+x+y} are building blocks that are linked end to end and each have double active functional groups; A and B represent an A-end ring-closure molecule and a B-end ring-closure molecule which are subjected to ring closure under the action of a cyclase, respectively, A' and B' represent an A-end ring-closure molecule and a B-end ring-closure molecule which are subjected to ring closure under the action of another different cyclase, respectively, and A, B, A' and B' are all amino acid residues; the building block Cₙ and the A are linked by means of an amide or ester bond; the building block Cₙ₊ₘ and the B are linked by means of an amide or ester bond; the building block Cₙ₊ₘ₊ₓ and the A' are linked by means of an amide or ester bond; the building block C_{n+m+x+y} and the B' are linked by means of an amide or ester bond; and the A and the B, or the A' and the B', react under the action of a cyclase to form a peptide bond and are then linked to form a bicyclic structure.

The present invention further provides a third bicyclic-structure compound library constructed by the aforementioned method, with the general structural formula of: 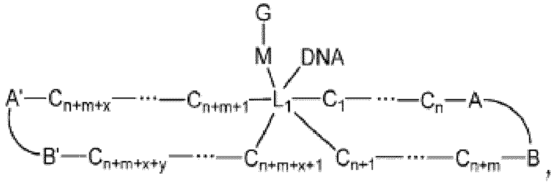
wherein 0 ≤ n ≤ 7, 0 ≤ m ≤ 7, 0≤x≤7,0≤y≤7, n, m, x and y are all integers, and 2 ≤ n+m+x+y≤7;
L1 is a linker molecule having at least six functional groups, and a DNA-encoded sequence is linked to the L1 by means of an amide bond; C₁ to Cₙ are building blocks that are linked end to end and each have double active functional groups, Cₙ₊₁ to Cₙ₊ₘ are building blocks that are linked end to end and each have double active functional groups, Cₙ₊ₘ₊₁ to Cₙ₊ₘ₊ₓ are building blocks that are linked end to end and each have double active functional groups, and Cₙ₊ₘ₊ₓ₊₁ to C_{n+m+x+y} are building blocks that are linked end to end and each have double active functional groups; G represents a solid-phase carrier, and M represents a molecule containing a photocleavable group; A and B represent an A-end ring-closure molecule and a B-end ring-closure molecule which are subjected to ring closure under the action of a cyclase, respectively, A' and B' represent an A-end ring-closure molecule and a B-end ring-closure molecule which are subjected to ring closure under the action of another different cyclase, respectively, and A, B, A' and B' are all amino acid residues; the building block Cₙ and the A are linked by means of an amide or ester bond; the building block Cₙ₊ₘ and the B are linked by means of an amide or ester bond; the building block Cₙ₊ₘ₊ₓ and the A' are linked by means of an amide or ester bond; the building block C_{n+m+x+y} and the B' are linked by means of an amide or ester bond; and the A and the B, or the A' and the B', react under the action of a cyclase to form a peptide bond and are then linked to form a bicyclic structure.

The present invention further provides a fourth bicyclic-structure compound library constructed by the aforementioned method, with the general structural formula of: 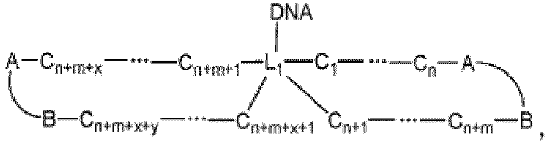
wherein 0 ≤ n ≤ 7, 0 ≤ m ≤ 7, 0≤x≤7,0≤y≤7, n, m, x and y are all integers, and 2 ≤ n+m+x+y≤7;
L1 is a linker molecule having at least six functional groups, and a DNA-encoded sequence is linked to the L1 by means of an amide bond; C₁ to Cₙ are building blocks that are linked end to end and each have double active functional groups, Cₙ₊₁ to Cₙ₊ₘ are building blocks that are linked end to end and each have double active functional groups, Cₙ₊ₘ₊₁ to Cₙ₊ₘ₊ₓ are building blocks that are linked end to end and each have double active functional groups, and Cₙ₊ₘ₊ₓ₊₁ to C_{n+m+x+y} are building blocks that are linked end to end and each have double active functional groups; A and B represent an A-end ring-closure molecule and a B-end ring-closure molecule which are subjected to ring closure under the action of a cyclase, respectively, A' and B' represent an A-end ring-closure molecule and a B-end ring-closure molecule which are subjected to ring closure under the action of another different cyclase, respectively, and A, B, A' and B' are all amino acid residues; the building block Cₙ and the A are linked by means of an amide or ester bond; the building block Cₙ₊ₘ and the B are linked by means of an amide or ester bond; the building block Cₙ₊ₘ₊ₓ and the A' are linked by means of an amide or ester bond; the building block C_{n+m+x+y} and the B' are linked by means of an amide or ester bond; and the A and the B, or the A' and the B', react under the action of a cyclase to form a peptide bond and are then linked to form a bicyclic structure. The fourth bicyclic-structure compound library may be obtained from the aforementioned third bicyclic-structure compound library by cleaving the molecule M containing the photocleavable group under illumination.

Specifically, each building block (C₁, ······,Cₙ, Cₙ₊₁, ······,Cₙ₊ₘ, Cₙ₊ₘ₊₁, ······, Cₙ₊ₘ₊ₓ, Cₙ₊ₘ₊ₓ₊₁, ··· ···, C_{n+m+x+y}) is independently selected from the group consisting of substituted or unsubstituted amino acid, substituted or unsubstituted dicarboxylic acid, substituted or unsubstituted diamine, substituted or unsubstituted glycol, α,β-unsaturated aldehyde, α,β-unsaturated ketone, α,β-unsaturated acid, a natural amino acid and an unnatural amino acid.

Specifically, at least one of the individual building blocks comprises a skeleton structure, which has an E3 ligase substrate structure and can bind to an E3 ligase. The skeleton structure is linked into a ring or linked onto a ring in the form of a side chain of the ring.

Specifically, the skeleton structure is selected from:

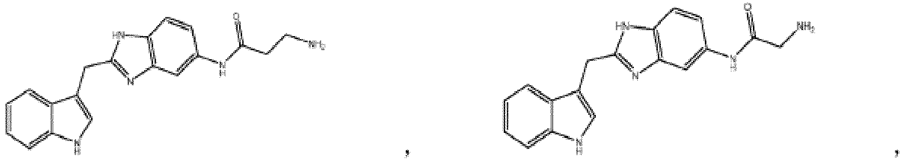

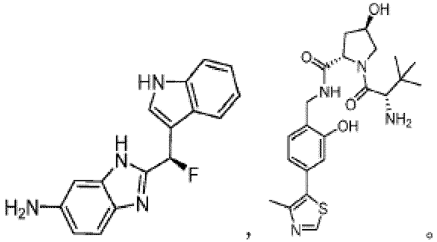

Specifically, each of the building blocks comprises at least one extra-ring side chain in total. The extra-ring side chain provides the extended function of further derivatization of library compounds, and at the same time, some functional modifications similar to lipidation may be performed on the side chain, such that the membrane permeability and infiltration characteristics of molecules can be further improved on the basis of the membrane permeability of the molecules themselves to expand the diversity of the compound library.

Specifically, each of the building blocks comprises at least two extra-ring side chains in total, and the at least two extra-ring side chains are linked by means of a chemical reaction to form a bicyclic structure. The bicyclic or polycyclic structure can stabilize the conformation of macrocyclic molecules, improve the rigidity of cyclic structures, improve the stability of cyclic-structure molecules, and prolong the half-life of cyclic-structure molecular drugs.

The compound library of the present invention provides a cyclic compound library, which increases the diversity of the compound library and expands its application range in new drug screening.

In the compound library of the present invention, the number and type of atoms in the cyclic structure can diversified and adjusted by different building blocks, which expands the diversity of the compound library.

In the compound library of the present invention, the adjacent building blocks can be assembled using an amide bond (—CO—NH—) to provide more hydrogen bond binding potential for the cyclic structure, which is beneficial to improve the binding force between the cyclic structure and proteins.

In the cyclic compound library of the present invention, the building blocks on the ring may comprise a skeleton structure, such as an E3 ligase substrate structure, which increases the application of the compound library in PROTAC.

### BRIEF DESCRIPTION OF THE DRAWINGS

FIG. 1 is a first diagram showing detection results of agarose gel electrophoresis according to Example 29;
FIG. 2 is a second diagram showing detection results of agarose gel electrophoresis according to Example 29;
FIG. 3 is a third diagram showing detection results of agarose gel electrophoresis according to Example 29;
FIG. 4 is a diagram showing detection results of agarose gel electrophoresis according to step 5 in Example 31;
FIG. 5 shows statistical results on the abundance of Tag sequences according to Example 32;
FIG 6 is a graph showing test results according to Example 33;
FIG. 7 is a diagram showing detection results of agarose gel electrophoresis according to step 5 in Example 34;
FIG. 8 shows statistical results on the abundance of Tag sequences according to Example 35;
FIG. 9 shows a flow cytometry analysis result of Example 36.

### DETAILED DESCRIPTION OF THE PREFERRED EMBODIMENTS

The technical solutions of the present invention will be described clearly and completely below. Obviously, the embodiments described are part of rather than all of the embodiments of the present invention. Based on the embodiments in the present invention, all other embodiments obtained by those ordinarily skilled in the art without making inventive efforts shall fall within the protection scope of the present invention.

In the reaction roadmap of the following embodiments, 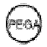 represents a solid-phase carrier (PEGA resin), and in addition, the rest 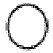 represents a building block. English characters L, N, A, I, E, P, etc. inside 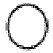 are one-letter symbol abbreviations of amino acids. For example, L represents leucine (Leu), N represents aspartic acid (Asn), A represents alanine (Ala), I represents isoleucine (Ile), E represents glutamic acid (Glu), and P represents proline (Pro). 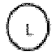 indicates that the building block is leucine, and other one-letter symbol abbreviations are also interpreted as the common one-letter symbol abbreviations of amino acids in the art. The characters AA₁, ······ and AA₄ inside 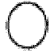 all indicate that these building blocks are amino acids, which are distinguished only by the serial number 1, ······, 4, etc. The characters C₁, ······, Cₙ, Cₙ₊₁, ······, Cₙ₊ₘ inside 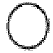 all indicate that these building blocks are any types of building blocks involved in the present application and are distinguished only by the serial number 1······n, n+1······ₙ₊ₘ. Photo linker represents the photocleavable group. Ahx represents 6-aminocaproic acid.

### Example 1: Synthesis of Cyclic Compound Library

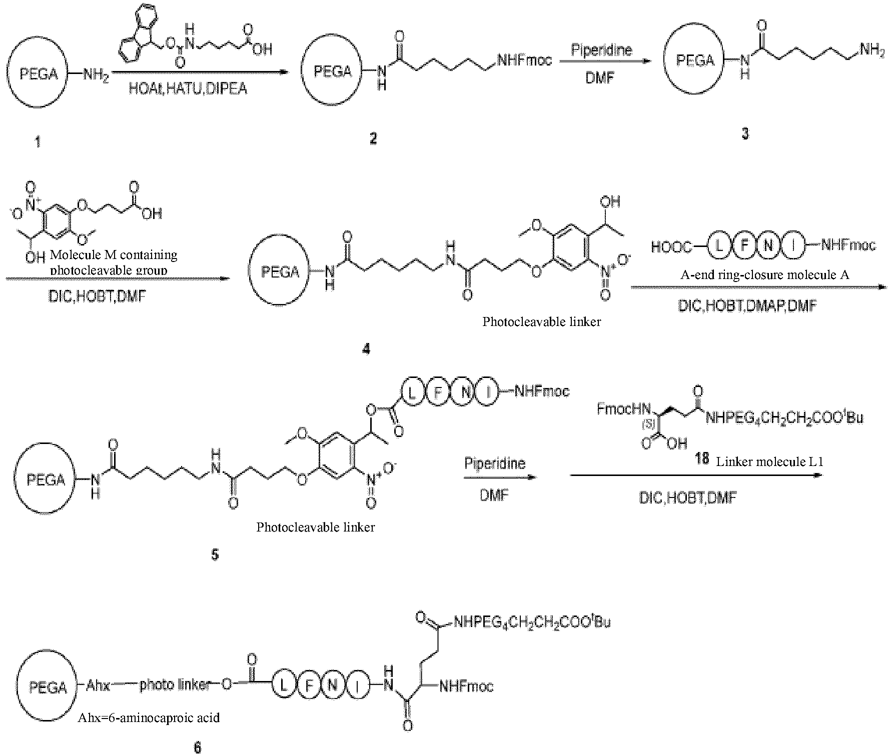

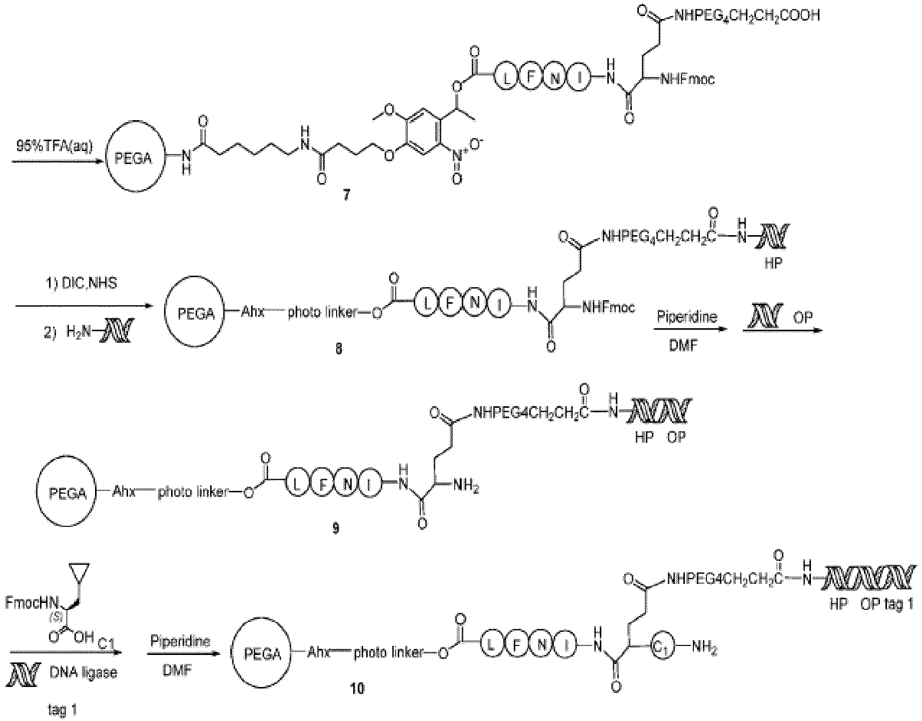

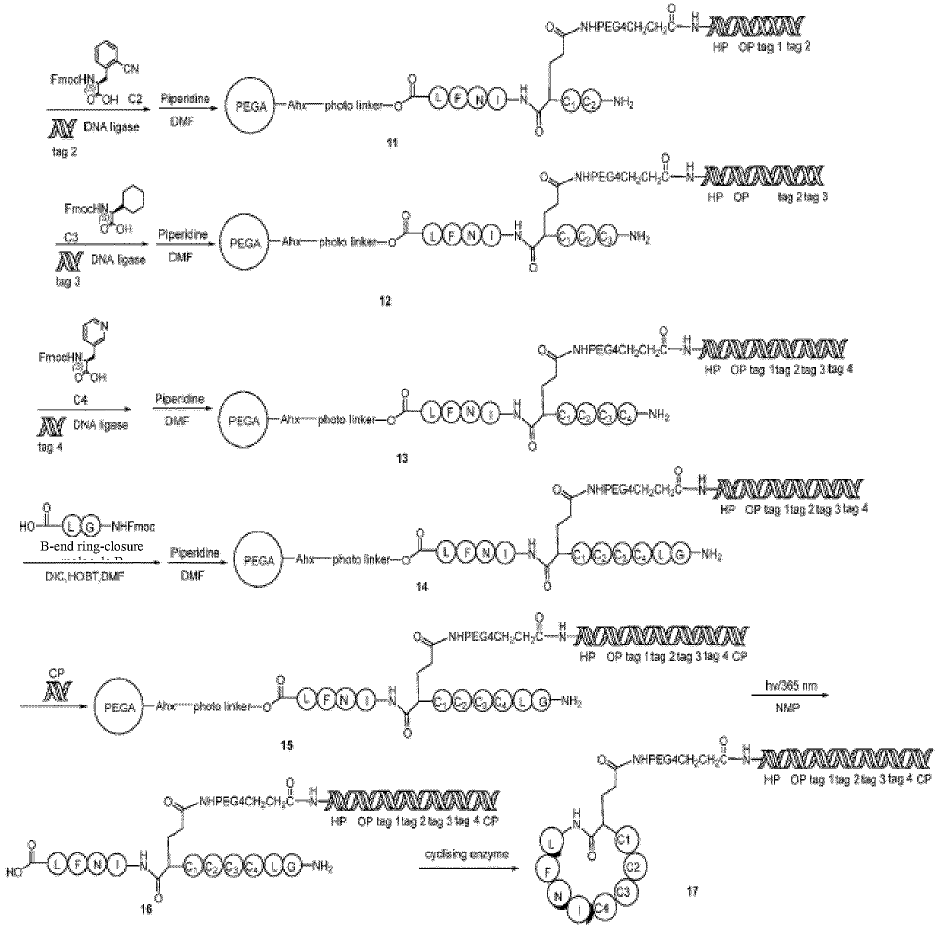

In step 1, an amino-modified PEGA resin (1 g, 0.5 mmol/g) was swollen in N,N-dimethylformamide for 1 h, and the resin was washed with 10% N,N-diisopropylethylamine in N,N-dimethylformamide and with N,N-dimethylformamide, respectively, and then dried by draining for later use. 10 mL of N,N-dimethylformamide, acetic anhydride (26 mg, 0.25 mmol) and N,N-diisopropylethylamine (129 mg, 1 mmol) were added to the ready resin and stirred for 30 min at room temperature. The resin was washed with N,N-dimethylformamide (3 × 3 mL), dichloromethane (3 × 3 mL), and N,N-dimethylformamide (3 × 5 mL), respectively, and dried by draining for later use. The resin was dissolved in 5 mL of N,N-dimethylformamide, and Fmoc-ε-Ahx-OH (397 mg, 4.5 eq.), HOAt (153 mg, 4.5 eq.), HATU (428 mg, 4.5 eq.) and DIEA (323 mg, 10 eq.) were added respectively and then stir for 2 hours at room temperature. The resin was washed with N,N-dimethylformamide (3 × 3 mL), dichloromethane (3 × 3 mL), and N,N-dimethylformamide (3 × 5 mL), respectively, and then dried by draining to obtain 1 g of resin for later use.

In step 2, 10 mL of 20% piperidine in N,N-dimethylformamide solution was added to the 1 g of resin, and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 980 mg of resin for later use.

In step 3, DIC (126 mg, 4 eq.), HOBt (135 mg, 4 eq.) and the molecule M containing photocleavage group (225 mg, 3 eq.) was stirred in 5 ml of N,N-dimethylformamide for 3 min; then, 980 mg of the swollen resin was added; the mixture was stirred for 2 hours at room temperature; and the resin was washed with N,N-dimethylformamide (3 × 3 mL), dichloromethane (3 × 3 mL), and N,N-dimethylformamide (3 × 5 mL), respectively, and then dried by draining to obtain 1 g of resin for later use.

In step 4, DIC (126 mg, 4 eq.), HOBt (135 mg, 4 eq.), DMAP (15 mg, 0.5 eq.) and Fmoc-protected tetrapeptide (225 mg, 3 eq.) were stirred in 10 ml of N,N-dimethylformamide for 10 min; then, 1 g of swollen resin was added; the mixture was stirred for 16 hours at room temperature; and the resin was washed with N,N-dimethylformamide (3 × 3 mL), dichloromethane (3 × 3 mL), and N,N-dimethylformamide ( 3 × 5 mL), respectively, and then dried by draining to obtain 1 g of resin for later use.

In step 5, referring to the method of removing Fmoc in step 2, 950 mg of resin was obtained from the compound 18 according to the condensation conditions in step 3.

In step 6, 950 mg of resin was added to 25 ml of 95% trifluoroacetic acid in water to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining; and then the resulting resin was added to 25 ml of 20% DIEA in N,N-dimethylformamide solution and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining to obtain 900 mg of resin for later use.

In step 7, DIC (40 mg, 5 eq.) and NHS (36 mg, 5 eq.) were dissolved in 5 ml of N,N-dimethylformamide, and then added to 250 mg of resin to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining for later use; the resin was swollen in 4 mL of HEPES (100 mM) buffer solution at pH 8.0; and HDNA (250 nmol, 1 mM) was added to react overnight at room temperature to obtain 240 mg of resin.

In step 8, 10 mL of 20% piperidine in N,N-dimethylformamide solution was added to the 240 mg of resin, and then stirred for 1 h at room temperature. The resin was washed with N,N-dimethylformamide (3 × 5 mL) and water (3 × 5 mL), respectively, and then dried by draining for later use; 3400 µl of water, 400 µl of 10 × T4 DNA ligase buffer, 8 µl of T4 DNA ligase (40 U/µl) and 175 µl of OP (AAATCGATGTG) (300 nm, 1.74 nm/µl) were added to the resin to react overnight at room temperature; and the resin was washed with water (3 × 10 mL) and T4 DNA ligase buffer solution (3 × 3 mL), respectively, and then dried by draining to obtain 230 mg of resin for later use. 3300 µl of water, 400 µl of 10 × T4 DNA ligase buffer solution, 8 µl of T4 DNA ligase (40 U/µl) and 294 µl of DNA tag1 (ATCTGACA) (300 nm, 1.7 nm/µl) were added to 230 mg of resin to react overnight at room temperature; and the resin was washed with T4 DNA ligase buffer solution (3 × 3 mL), water (3 × 10 mL), and N,N-dimethylformamide (3 × 10 mL), respectively, and then dried by draining for later use. DIC (40 mg, 5 eq.), HOBt (42 mg, 5 eq.), and the amino acid AA1 (110 mg, 5 eq.) were stirred in 5 ml of N,N-dimethylformamide for 10 min; then, 230 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining for later use. 5 mL of 20% piperidine in N,N-dimethylformamide solution was added to the resin, and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 220mg of resin for later use. GTTACACGT

In step 9, the DNA tag2 (GTTACACGT) and the amino acid AA2 were treated according to the operation procedures of step 9 to obtain 210 mg of resin.

In step 10, the DNA tag3 (CTGTAACGA) and the amino acid AA3 were treated according to the operation procedures of step 9 to obtain 200 mg of resin.

In step 11, the DNA tag4 (TTCGAACTT) and the amino acid AA4 were treated according to the operation procedures of step 9 to obtain 190 mg of resin.

In step 12, DIC (40 mg, 5 eq.), HOBt (42 mg, 5 eq.), and Fmoc-protected dipeptide (128 mg, 5 eq.) were stirred in 5 ml of N,N-dimethylformamide for 10 min; then, 190mg of swollen resin was added; the mixture was stirred for 2 hours at room temperature; the resin was washed with N,N-dimethylformamide (3 × 3 mL), dichloromethane (3 × 3 mL), and N,N-dimethylformamide (3 × 5 mL), respectively, and then dried by draining for later use; and 5 mL of 20% piperidine in N,N-dimethylformamide solution was added to the resin and stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 185mg of resin for later use.

In step 13, 3400 µl of water, 400 µl of 10 × T4 DNA ligase buffer solution, 8 µl of T4 DNA ligase (40 U/µl), and 175 µl of CP (TAGCCTATTGTCAGACAAGCTTCACCTGC) (300 nm, 1.74 nm/µl) were added to 185 mg of resin to react overnight at room temperature; and the resin was washed with water (3 × 10 mL) and T4 DNA ligase buffer solution (3 × 3 mL), respectively, and then dried by draining to obtain 180 mg of resin for later use.

In step 14, 180 mg of resin was added to 1 ml of N-methylpyrrolidone, and illuminated for 5 h under UV light at 365 nm; the resin was filtered, dialyzed with a solvent, and then lyophilized to obtain 5 mg of a compound 16.

In step 15, 2 mg of the compound 16 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of ring-closure enzyme OaAEP3 (1.34 mg/mL) were added respectively; and the mixture underwent reaction overnight at 37 °C to prepare 1.2 mg of product.

In this example, the synthesis method for the linker molecule compound 18 was as follows.

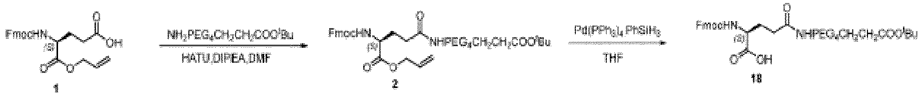

In step 1, the compound Fmoc-Asp(Alloc)-OH (750 mg, 1.83 mmol), NH2-PEG4CH₂CH₂COO^{t}Bu (588 mg, 1.83 mmol) and DIPEA(472 mg, 3.66 mmol) were dissolved in 10 ml N,N-dimethylformamide; HATU (836 mg, 2.2 mmol) was added at 0 degree centigrade; the mixture was stirred for 2 h at room temperature, and the reaction was quenched with water; 10 ml of ethyl acetate was added; the aqueous phase was extracted three times with ethyl acetate; the organic phases were combined, dried over anhydrous sodium sulfate, filtered, and spun dry to obtain a crude product. The crude product was let to flow through the column with ethyl acetate as an eluent to obtain colorless oil (1.2 g, 92.3%).

In step 2, the compound 2 (1.2 g, 1.68 mmol) and Pd(PPh3)4 (97 mg, 0.084 mmol) were dissolved in 20 ml of tetrahydrobaran solution; phenylsilane (363 mg, 3.36 mmol) was added at 0 degree centigrade in the presence of nitrogen; the mixture was stirred for 1 h at room temperature and spun dry, and then flows through the column for purification (6% methanol in dichloromethane solution as an eluent) to obtain a white solid (1 g, 88.5%). LCMS:673.7(M+H)⁺.

### Example 2: Verification of Ring Closure Method under Cyclase

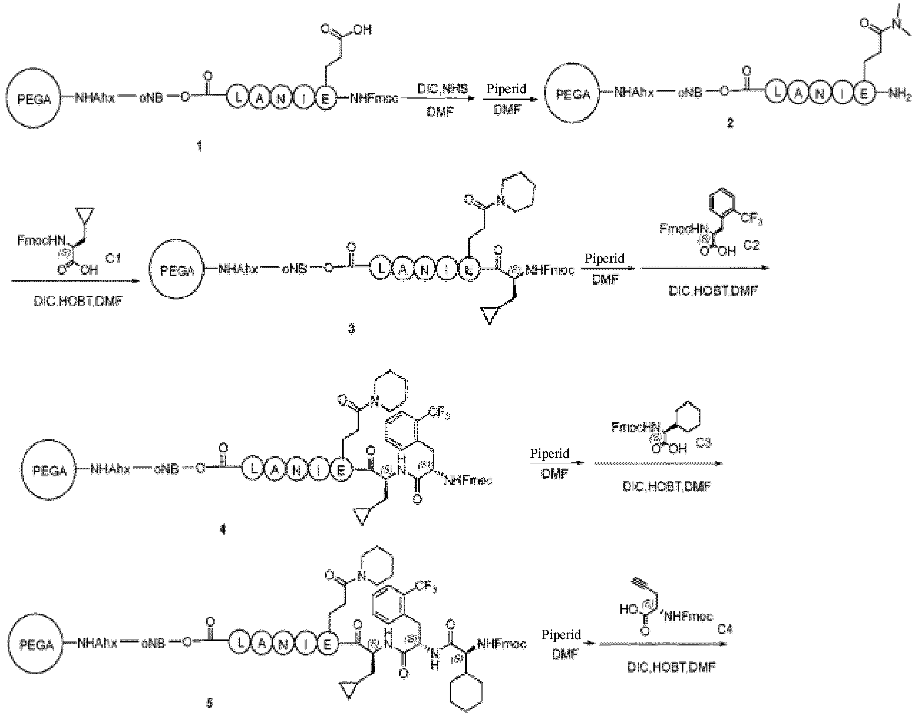

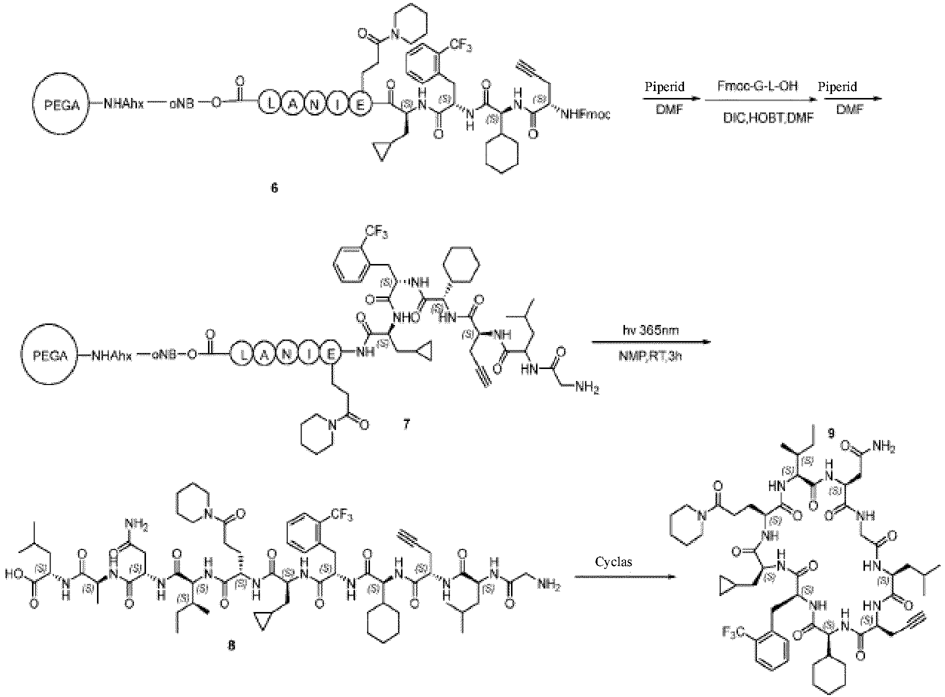

In step 1, DIC (40 mg, 5 eq.) and NHS (36 mg, 5 eq.) were dissolved in 5 ml of N,N-dimethylformamide, and then added to 250 mg of resin to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining for later use; 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 240 mg of resin for later use.

In step 2, DIC (40 mg, 5 eq.), HOBt (42 mg, 5 eq.), and the amino acid AA1 (110 mg, 5 eq.) were stirred in 5 ml of N,N-dimethylformamide for 10 min; then, 230 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 240 mg of resin for later use.

In step 3, with 5 mL of 20% piperidine in N,N-dimethylformamide solution, DIC (40 mg, 5 eq.), HOBt (42 mg, 5 eq.) and amino acid AA2 (142 mg, 5 eq.), Fmoc was removed by referring to step 1 and the condensation operation was carried out by referring to step 2, to obtain 235 mg of resin.

In step 4, with 5 mL of 20% piperidine in N,N-dimethylformamide solution, DIC (40 mg, 5 eq.), HOBt (42 mg, 5 eq.) and amino acid AA3 (119 mg, 5 eq.), Fmoc was removed by referring to step 1 and the condensation operation was carried out by referring to step 2, to obtain 230 mg of resin.

In step 5, with 5 mL of 20% piperidine in N,N-dimethylformamide solution, DIC (40 mg, 5 eq.), HOBt (42 mg, 5 eq.) and amino acid AA4 (105 mg, 5 eq.), Fmoc was removed by referring to step 1 and the condensation operation was carried out by referring to step 2, to obtain 220 mg of resin.

In step 6, with 5 mL of 20% piperidine in N,N-dimethylformamide solution, DIC (40 mg, 5 eq.), HOBt (42 mg, 5 eq.) and dipeptide (128 mg, 5 eq.), Fmoc was removed by referring to step 1, the condensation operation was carried out by referring to step 2, and then Fmoc was removed by referring to step 1, to obtain 215 mg of resin.

In step 7, 215mg of resin was added to 1 ml of N-methylpyrrolidone, and illuminated for 5 h under UV light at 365 nm; the resin was filtered, dialyzed with a solvent, and then lyophilized to obtain 25mg of a compound 8.

In step 8, 0.3 mg of the compound 8 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively; and the mixture underwent reaction overnight at 37°C to obtain a cyclic compound 9. LCMS:1154.72(M+H)⁺.

### Example 3: Verification of Construction Method 3 for Library

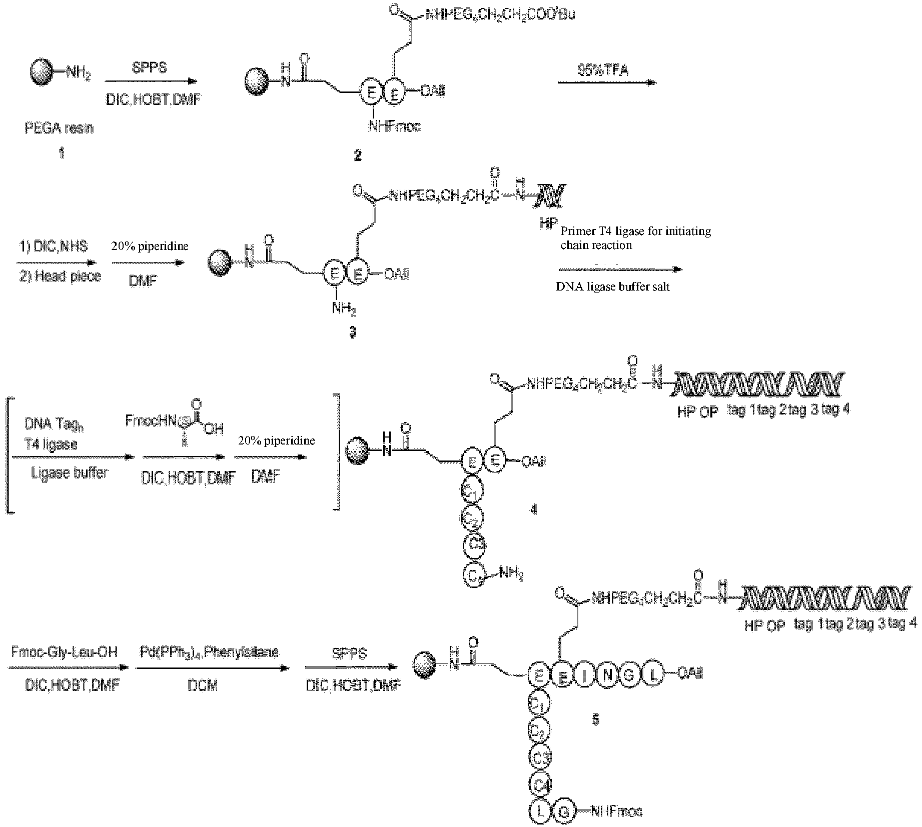

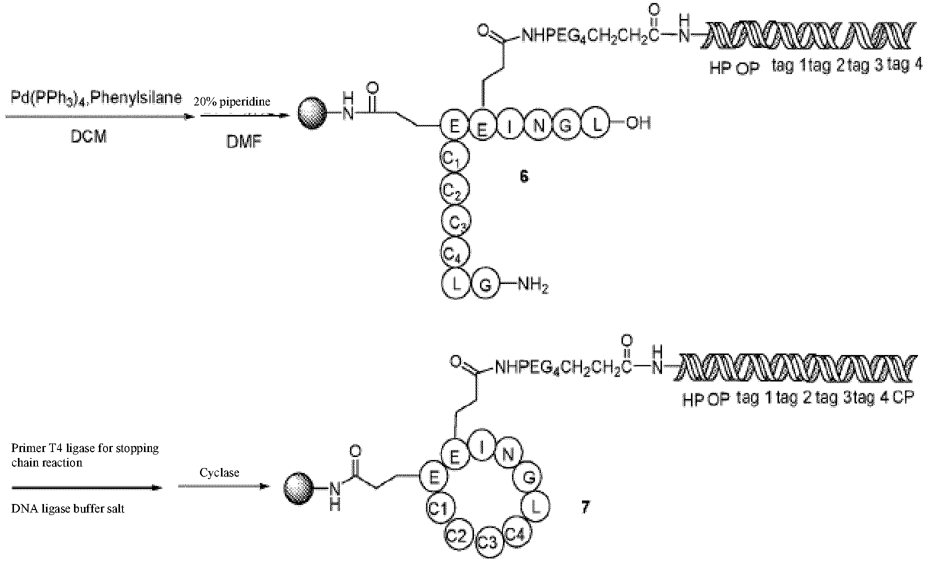

Synthesis method:
1. A PEGA resin (100 mg, 25 µmol) was swollen for 2 h in 3 ml of N,N-dimethylformamide, and the standard solid-phase polypeptide synthesis operation was carried out to obtain 95 mg of resin.
2. 95% of trifluoroacetic acid, 2.5% of water and 2.5% of triisopropylsilane were added to 95 mg of resin and stirred for 2 h at room temperature. 10% N,N-diisopropylethylamine in N,N-dimethylformamide solution (3 mL) was added to the resin and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining. DIC (32 mg, 10 eq.) and NHS (28 mg, l0 eq.) were stirred for 1 min in 3 ml of N,N-dimethylformamide, and then added to the resin to react for 4 h at 37°C. The resin was washed with N,N-dimethylformamide (3 × 2 mL), dichloromethane (3 × 2 mL), and N,N-dimethylformamide (3 × 2 mL), respectively, and then dried by draining; and HDNA (100 nmol) was dissolved in 3 ml of 100 mM HEPES buffer at pH 8.0 and then added to the resin to react for 16 h at 37 °C . 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 85 mg of resin for later use.
3. 2095 µl of water, 250 µl of 10 × T4 DNA ligase buffer solution, 25 µl of T4 DNA ligase (10000 U), and 58 µl of OP (AAATCGATGTG) (100 nm, 1.74 nm/µl) were added to 85mg of resin to react overnight at room temperature; and the resin was washed with water (3 × 5 mL) and T4 DNA ligase buffer solution (3 × 3 mL), respectively, and then dried by draining to obtain 75 mg of resin for later use. 2095 µl of water, 250 µl of 10 × T4 DNA ligase buffer solution, and 25 µl of T4 DNA ligase (10000 U) were added to 75 mg of resin; the mixture was divided into 96 portions; the DNA tag (1.35 nm, 1 nm/µl) was added to each well to react overnight at room temperature; and the resin was washed with T4 DNA ligase buffer solution (3 × 3 mL), water (3 × 3 mL), and N,N-dimethylformamide (3 × 3 mL), respectively, and then dried by draining for later use. DIC (16 mg, 5 eq.) and HOBt (17 mg, 5 eq.) were dissolved in 5 mL of N,N-dimethylformamide and then divided into 96 portions; each portion together with the amino acid (5 eq.) were added to the resin in each well; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining for later use. The resins were put together, and 5 mL of 20% piperidine in N,N-dimethylformamide solution was added to the resins and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 70mg of resin for later use. All condensation reactions and DNA linkage reactions were repeated 4 times in total. After the fourth round of linkage for the amino acid and the DNA tag, the resins were combined, and then washed with N,N-dimethylformamide (3 × 5 mL), dichloromethane (3 × 3 mL), and N,N-dimethylformamide (3 × 5 mL), respectively, and then dried by draining to obtain 70 mg of resin for later use.
4. DIC (16 mg, 5 eq.), HOBt (17 mg, 5 eq.), and Fmoc-Gly-Leu-OH (51 mg, 5 eq.) were stirred for 2 min in 5 ml of N,N-dimethylformamide; then 70 mg of resin was added; the mixture was stirred for 2 h at room temperature; and the resin was washed with N,N-dimethylformamide (3 × 3mL), dichloromethane (3 × 3 mL), and N,N-dimethylformamide (3 × 5 mL), respectively, and then dried by draining; and the resin, tetratriphenylphosphine palladium (2.89 mg, 0.2 eq.) and phenylsilane (54.12 mg, 20 eq.) were stirred in 5 mL of dichloromethane for 1 h at room temperature under a nitrogen protection condition. The resin was washed with dichloromethane (3 × 3 mL) and N,N-dimethylformamide (3 × 3 mL), respectively, and then dried by draining for later use. The resulting resin was treated according to the standard solid-phase synthesis operation to obtain 65 mg of resin.
5. 65 mg of resin, tetratriphenylphosphine palladium (2.89 mg, 0.2 eq.) and phenylsilane (54.12 mg, 20 eq.) were stirred in 5 ml dichloromethane for 1 h at room temperature under a nitrogen protection condition. The resin was washed with dichloromethane (3 × 3 mL) and N,N-dimethylformamide (3 × 3 mL), respectively, and then dried by draining for later use; and then the resulting resin was added to 5 ml of 20% piperidine in N,N-dimethylformamide solution and stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 60mg of resin.
6. 2095 µl of water, 250 µl of 10 × T4 DNA ligase buffer solution, 25 µl of T4 DNA ligase (10000 U) and 57 µl of CP (100 nm, 1.74 nm/µl) were added to 60mg of resin to react overnight at room temperature; the resin was washed with T4 DNA ligase buffer solution (3 × 3 mL) and water (3 × 5 mL), respectively, and then dried by draining for later use; 50mg of resin was swollen in 830 µl of water; 100 µl of sodium acetate buffer solution (PH=5.0, 0.5 M), 2 µl of sodium chloride solution (0.25 M), 20 µl of disodium ethylenediamine tetraacetate solution (0.05 M), 10 µl of TCEP solution (0.05 M) and 30 µl of OaAEP3 (1.34 mg/mL) were added respectively; and the mixture underwent reaction overnight at 37 degrees centigrade to obtain a DNA-encoded cyclic peptide library.

### Example 4: Library Construction Based on Different A-end Ring Closure

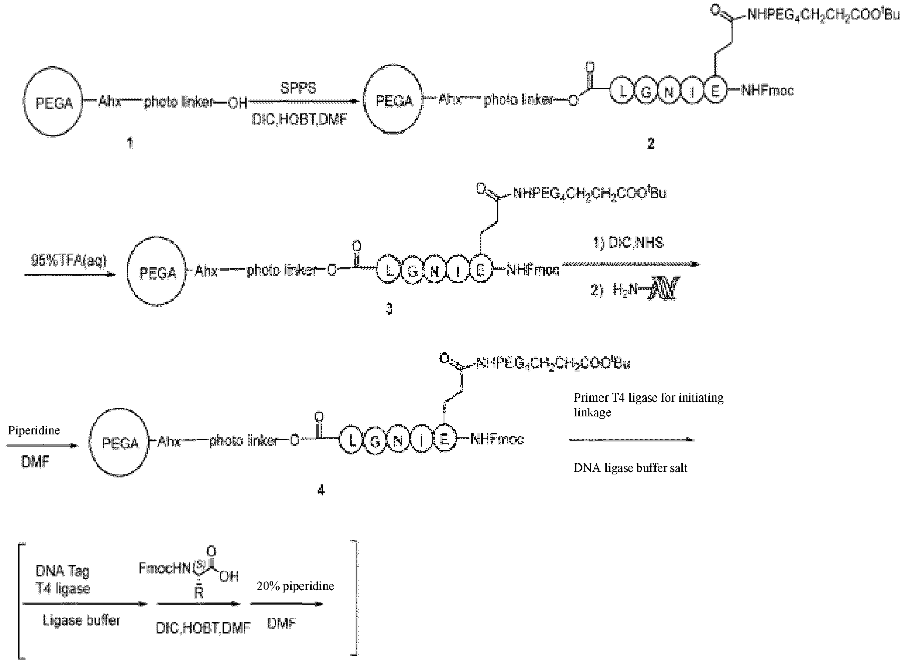

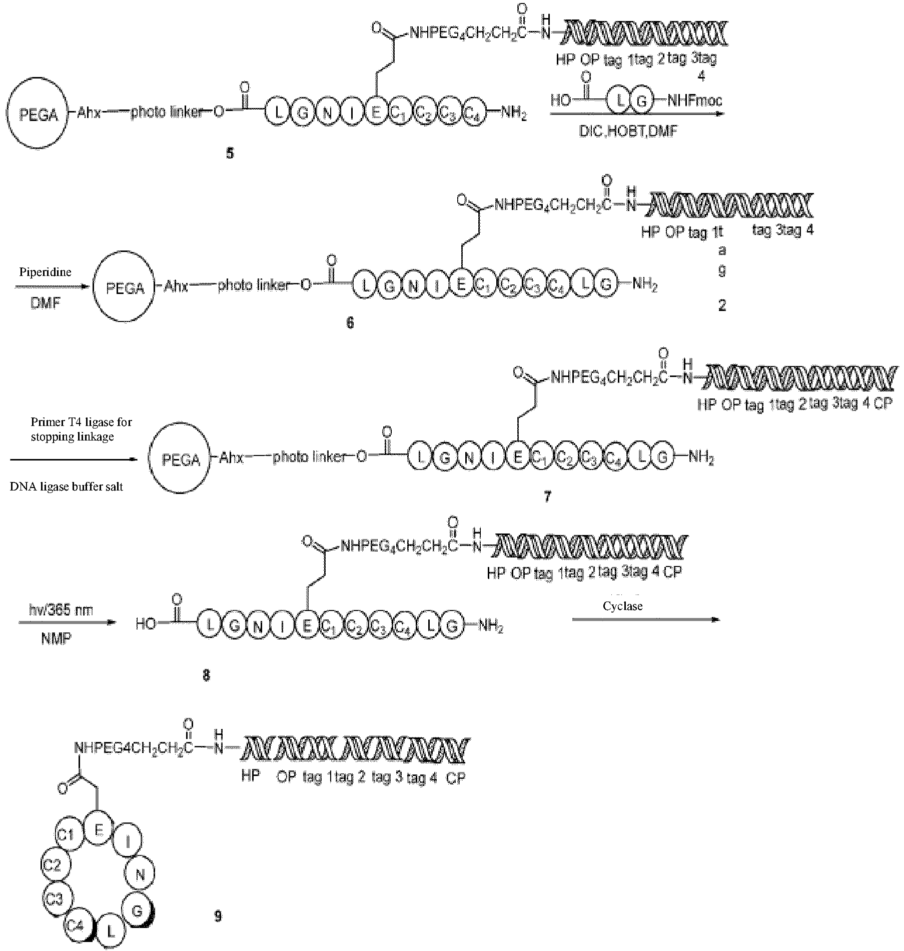

Synthesis method:
1. 100 mg of a resin 1 was treated according to the standard solid-phase synthesis operation by referring to the above synthesis method to obtain 90 mg of resin.
2. 90 mg of resin was added to 5 mL of 95% trifluoroacetic acid in water to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining; and then the resulting resin was added to 5 ml of 20% DIEA in N,N-dimethylformamide solution and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining to obtain 85mg of resin for later use.
3. DIC (16 mg, 5 eq.) and NHS (15 mg, 5 eq.) were dissolved in 5 ml of N,N-dimethylformamide, and then added to 85 mg of resin to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining for later use; the resin was swollen in 2 mL of HEPES (100 mM) buffer solution at pH 8.0; and HDNA (100 nmol, 1 mM) was added to react overnight at room temperature to obtain 80mg of resin. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 75mg of resin for later use.
4. 2095 µl of water, 250 µl of 10 × T4 DNA ligase buffer solution, 25 µl of T4 DNA ligase (10000 U), and 58 µl of OP (AAATCGATGTG) (100 nm, 1.74 nm/µl) were added to 75mg of resin to react overnight at room temperature; and the resin was washed with water (3 × 5 mL) and T4 DNA ligase buffer solution (3 × 3 mL), respectively, and then dried by draining to obtain 75 mg of resin for later use. 2095 µl of water, 250 µl of 10 × T4 DNA ligase buffer solution, and 25 µl of T4 DNA ligase (10000 U) were added to 75 mg of resin; the mixture was divided into 96 portions; the DNA tag (1.35 nm, 1 nm/µl) was added to each well to react overnight at room temperature; and the resin was washed with T4 DNA ligase buffer solution (3 × 3 mL), water (3 × 10 mL), and N,N-dimethylformamide (3 × 3 mL), respectively, and then dried by draining for later use. DIC (16 mg, 5 eq.) and HOBt (17 mg, 5 eq.) were dissolved in 5 mL of N,N-dimethylformamide and then divided into 96 portions; each portion together with the Fmoc-amino acid (5 eq.) were added to the resin in each well; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining for later use. The resins were put together, and 5 mL of 20% piperidine in N,N-dimethylformamide solution was added to the resins and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 70mg of resin for later use. After the fourth round of linkage for the amino acid and the DNA tag, the resin was combined to obtain 65 mg of resin.
5. DIC (16 mg, 5 eq.), HOBt (17 mg, 5 eq.), and Fmoc-Gly-Leu-OH (51 mg, 5 eq.) were stirred in 5 ml of N,N-dimethylformamide for 2 min; then, 65 mg of the resin was added; the mixture was stirred for 2 hours at room temperature; the resin was washed with N,N-dimethylformamide (3 × 3 mL), dichloromethane (3 × 3 mL), and N,N-dimethylformamide ( 3 × 5 mL), respectively, and then dried by draining for later use; the resin was washed with dichloromethane (3 × 3 mL) and N,N-dimethylformamide ( 3 × 3 mL), respectively, and then dried by draining; and 5 mL of 20% piperidine in N,N-dimethylformamide solution was added to the resin and stirred for 1 h at room temperature. The resin was washed with N,N-dimethylformamide (3 × 3 mL) and water (3 × 5 mL), respectively, and then dried by draining to obtain 60 mg of resin for later use.
6. 2095 µl of water, 250 µl of 10 × T4 DNA ligase buffer solution, 25 µl of T4 DNA ligase (10000 U), and 57 µl of CP (100 nm, 1.74 nm/µl) were added to 60mg of resin to react overnight at room temperature; and the resin was washed with T4 DNA ligase buffer solution (3 × 3 mL) and water (3 × 5 mL), respectively, and then dried by draining for later use.
7. 55 mg of resin was added to 1 ml of N-methylpyrrolidone, and illuminated for 5 h under UV light at 365 nm; the resin was filtered, dialyzed with a solvent, and then lyophilized to obtain 5 mg of a compound 8.
8. 5 mg of the compound 8 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively; and the mixture underwent reaction overnight at 37 °C, and the aqueous phase was lyophilized to obtain 3 mg of a DNA-encoded cyclic peptide library. When the ring-closure reaction was performed, a fragment -GL was removed from the A-end ring-closure molecule.

### Example 5: Library Construction Based on Different A-end Ring Closure

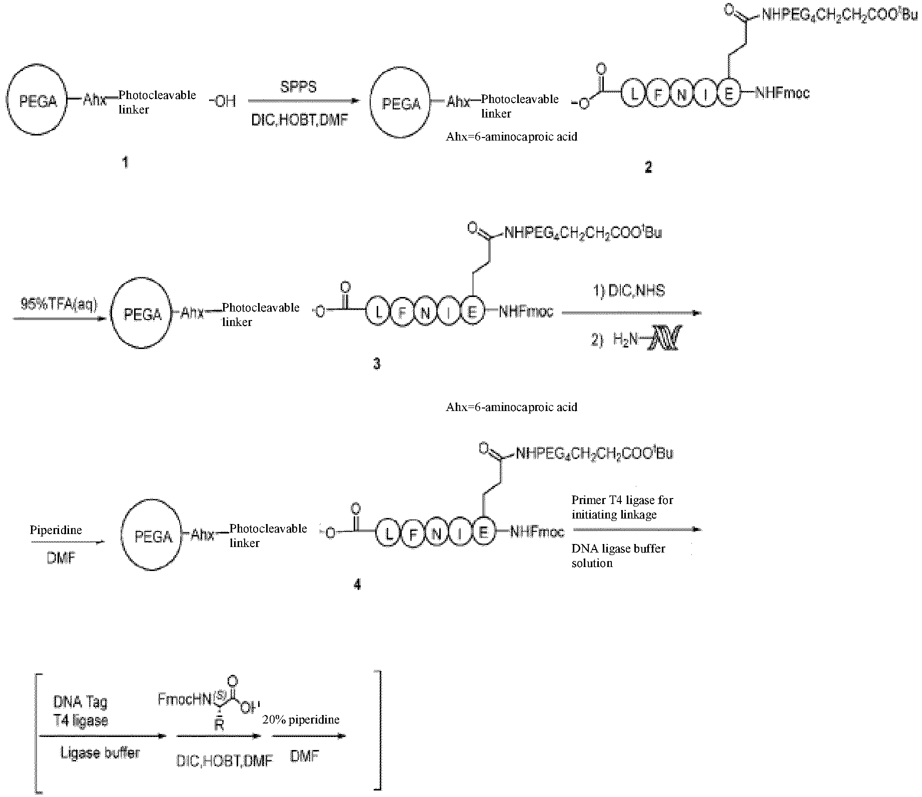

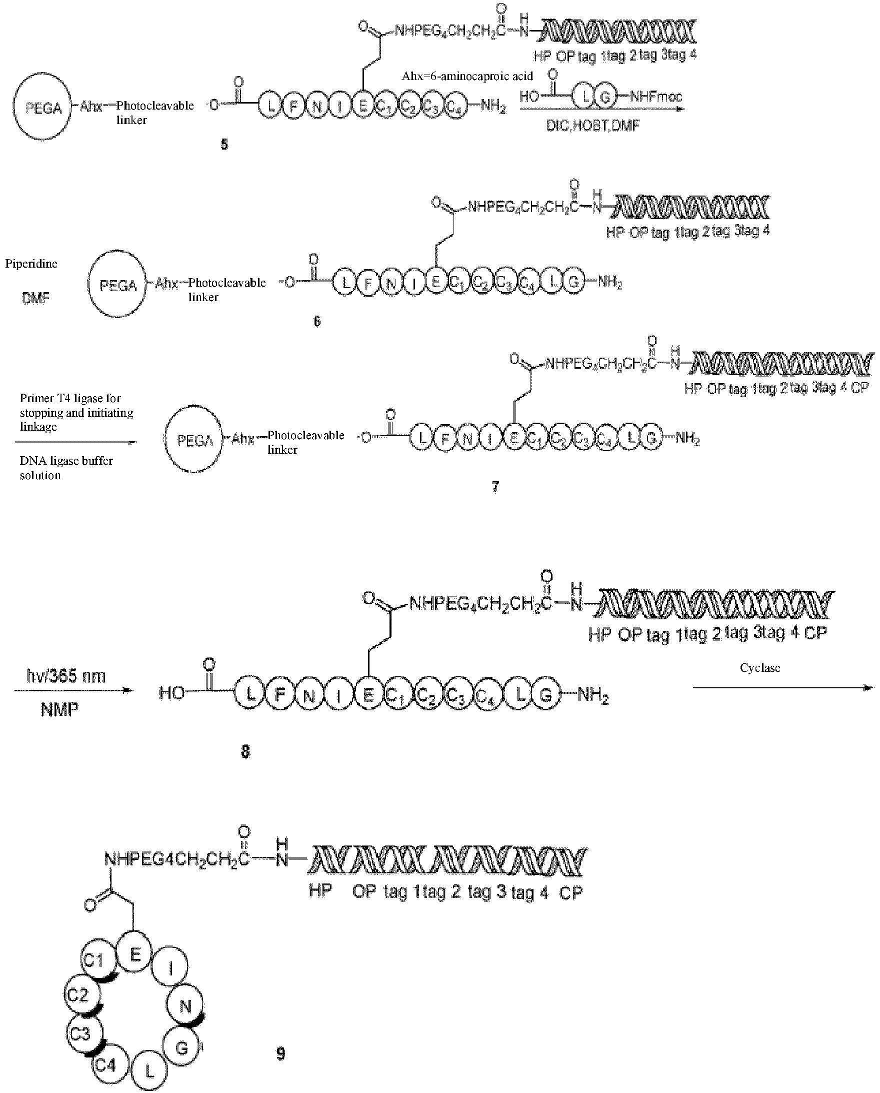

Synthesis method:
1. 100 mg of a resin 1 was treated according to the standard solid-phase synthesis operation by referring to the above synthesis method to obtain 90 mg of resin.
2. 90mg of resin was added to 5 ml of 95% trifluoroacetic acid in water to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining; and then the resulting resin was added to 5 ml of 20% DIEA in N,N-dimethylformamide solution and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining to obtain 85mg of resin for later use.
3. DIC (16 mg, 5 eq.) and NHS (15 mg, 5 eq.) were dissolved in 5 ml of N,N-dimethylformamide, and then added to 85mg of resin to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining for later use; the resin was swollen in 2 mL of HEPES (100 mM) buffer solution at pH 8.0; and HDNA (100 nmol, 1 mM) was added to react overnight at room temperature to obtain 80 mg of resin. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 75 mg of resin for later use.
4. 2095 µl of water, 250 µl of 10 × T4 DNA ligase buffer solution, 25 µl of T4 DNA ligase (10000 U), and 58 µl of OP (AAATCGATGTG) (100 nm, 1.74 nm/µl) were added to 75mg of resin to react overnight at room temperature; and the resin was washed with water (3 × 5 mL) and T4 DNA ligase buffer solution (3 × 3 mL), respectively, and then dried by draining to obtain 75 mg of resin for later use. 2095 µl of water, 250 µl of 10 × T4 DNA ligase buffer solution, and 25 µl of T4 DNA ligase (10000 U) were added to 75 mg of resin; the mixture was divided into 96 portions; the DNA tag (1.35 nm, 1 nm/µl) was added to each well to react overnight at room temperature; and the resin was washed with T4 DNA ligase buffer solution (3 × 3 mL), water (3 × 3 mL), and N,N-dimethylformamide (3 × 3 mL), respectively, and then dried by draining for later use. DIC (16 mg, 5 eq.) and HOBt (17 mg, 5 eq.) were dissolved in 5 mL of N,N-dimethylformamide and then divided into 96 portions; each portion together with the Fmoc-amino acid (5 eq.) were added to the resin in each well; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining for later use. The resins were put together, and 5 mL of 20% piperidine in N,N-dimethylformamide solution was added to the resins and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 70mg of resin for later use. After the fourth round of linkage for the amino acid and the DNA tag, the resin was combined to obtain 65 mg of resin.
5. DIC (16 mg, 5 eq.), HOBt (17 mg, 5 eq.), and Fmoc-Gly-Leu-OH (51 mg, 5 eq.) were stirred in 5 ml of N,N-dimethylformamide for 2 min; then, 65 mg of the resin was added; the mixture was stirred for 2 hours at room temperature; the resin was washed with N,N-dimethylformamide (3 × 3 mL), dichloromethane (3 × 3 mL), and N,N-dimethylformamide ( 3 × 5 mL), respectively, and then dried by draining for later use; the resin was washed with dichloromethane (3 × 3 mL) and N,N-dimethylformamide ( 3 × 3 mL), respectively, and then dried by draining; and 5 mL of 20% piperidine in N,N-dimethylformamide solution was added to the resin and stirred for 1 h at room temperature. The resin was washed with N,N-dimethylformamide (3 × 3 mL) and water (3 × 5 mL), respectively, and then dried by draining to obtain 60 mg of resin for later use.
6. 2095 µl of water, 250 µl of 10 × T4 DNA ligase buffer solution, 25 µl of T4 DNA ligase (10000 U), and 57 µl of CP (100 nm, 1.74 nm/µl) were added to 60mg of resin to react overnight at room temperature; and the resin was washed with T4 DNA ligase buffer solution (3 × 3 mL) and water (3 × 5 mL), respectively, and then dried by draining for later use.
7. 55 mg of resin was added to 1 ml of N-methylpyrrolidone, and illuminated for 5 h under UV light at 365 nm; the resin was filtered, dialyzed with a solvent, and then lyophilized to obtain 5 mg of a compound 8.
8. 5 mg of the compound 8 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively; and the mixture underwent reaction overnight at 37 °C, and the aqueous phase was lyophilized to obtain 3 mg of a DNA-encoded cyclic peptide library. When the ring-closure reaction was performed, a fragment -Fl was removed from the A-end ring-closure molecule.

### Example 6: Library Construction Based on Different A-end Ring Closure

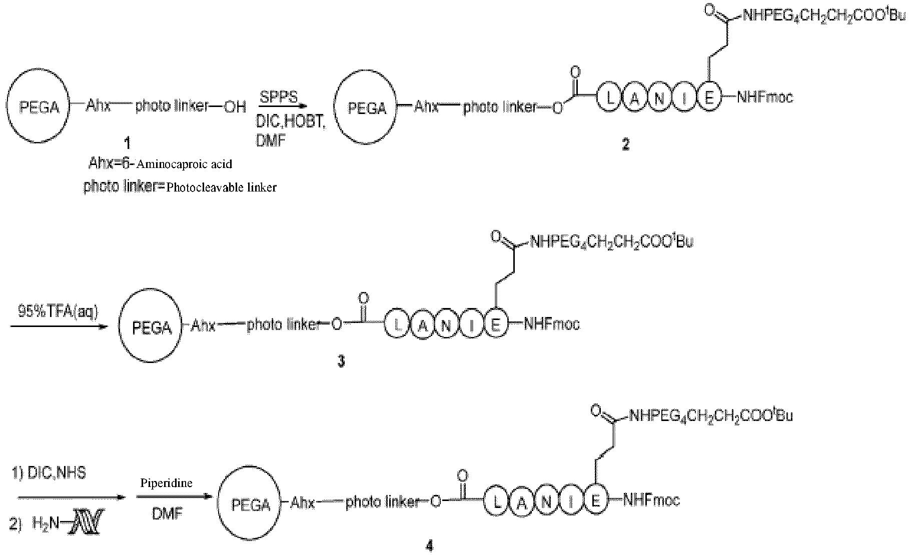

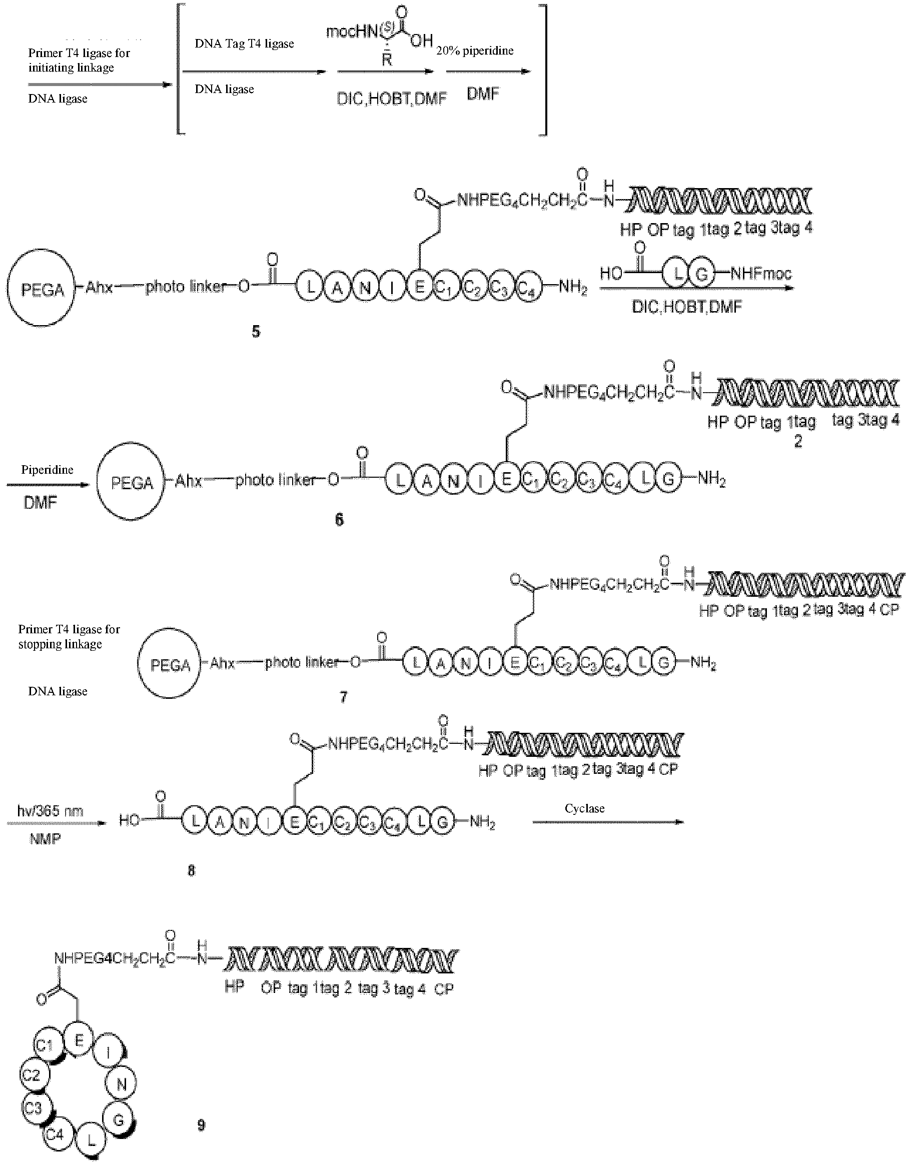

Synthesis method:
Referring to the construction method for the library in Example 4, 3 mg of a DNA-encoded cyclic peptide library was finally obtained from 100 mg of the starting resin. When the ring-closure reaction was performed, a fragment -AL was removed from the A-end ring-closure molecule.

### Example 7: Library Construction Based on Different A-end Ring Closure

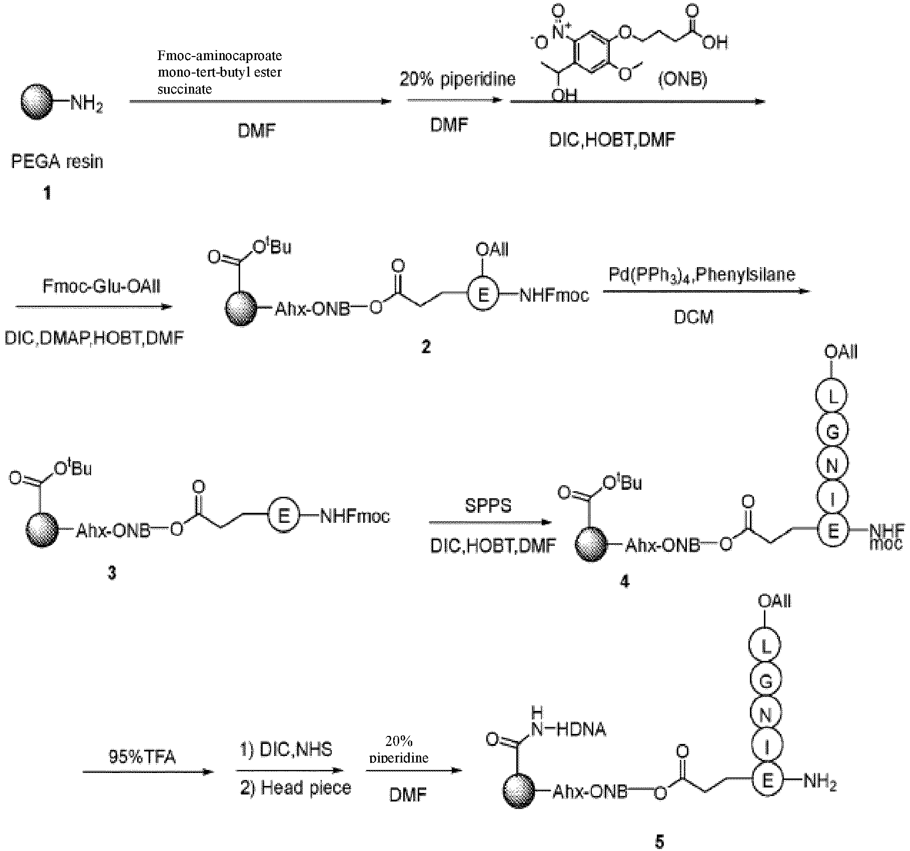

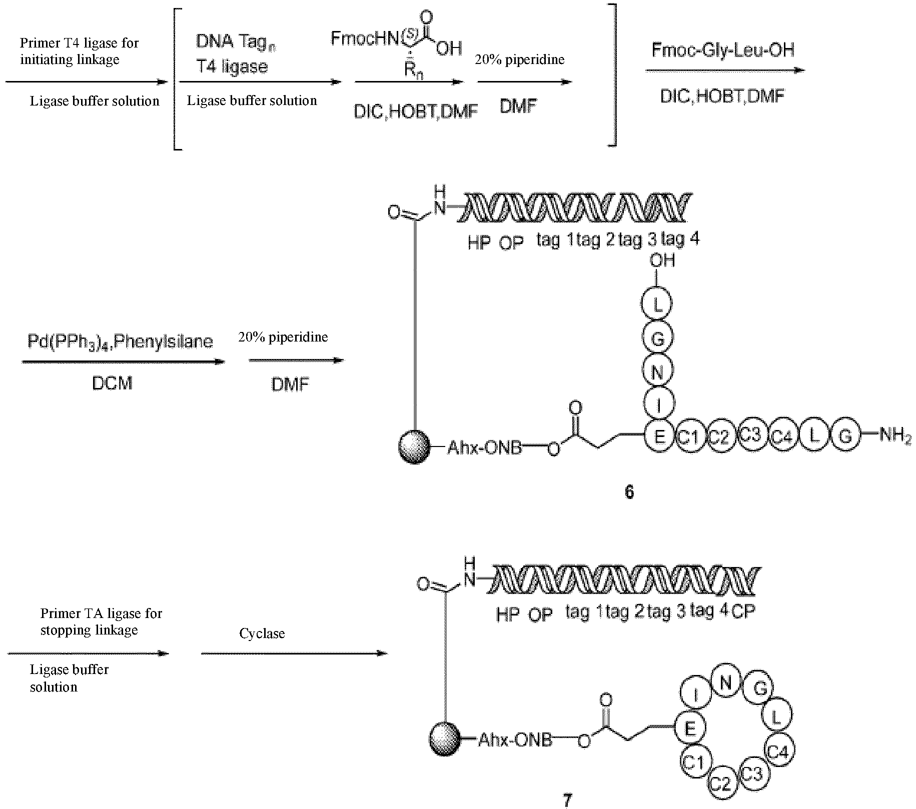

Synthesis method:
1. The PEGA resin (100 mg, 25 µmol) is swollen for 2 h in 3 ml of N,N-dimethylformamide; Fmoc-Ahx-OH (4.77 equiv), mono-tert-butyl succinate (0.23 equiv), HOBt (5 equiv), HBTU (5equiv) and DIPEA (10 equiv) were added; and the mixture was stirred for 2 h at room temperature. The resin was washed with N,N-dimethylformamide (3 × 2 mL), dichloromethane (3 × 2 mL), and N,N-dimethylformamide (3 × 2 mL), respectively, and then dried by draining. 4 mL of 20% piperidine in N,N-dimethylformamide solution was added to the resin and stirred for 1 hour at room temperature; and the resin was washed with N, N-dimethylformamide (3 × 2 mL), dichloromethane (3 × 2 mL), and N, N-dimethylformamide (3 × 2 mL) respectively, and then dried by draining. DIC (16 mg, 5 eq.), HOBt (17 mg, 5 eq.), and 4-(4-(1-hydroxyethyl)-2-methoxy-5-nitrophenoxy)butyric acid (38 mg, 5 eq.) were stirred in 3 ml of N,N-dimethylformamide for 5 min, and then added to the dried resin; the mixture was stirred for 2 hours at room temperature; and the resin was washed with N,N-dimethylformamide (3 × 2 mL), dichloromethane (3 × 2 mL), and N,N-dimethylformamide (3 × 2 mL), respectively, and then dried by draining. DIC (16 mg, 5 eq.), HOBt (17 mg, 5 eq.), DMAP (2 mg, 0.5 eq.) and Fmoc-Glu-OAll (51 mg, 5 eq.) were stirred in 3 ml of N,N-dimethylformamide for 5 min, and then added to the dried resin; the mixture was stirred for 2 hours at room temperature; and the resin was washed with N,N-dimethylformamide (3 × 2 mL), dichloromethane (3 × 2 mL), and N,N-dimethylformamide (3 × 2 mL), respectively, and then dried by draining to obtain 95 mg of resin.
2. 90 mg of resin, tetratriphenylphosphine palladium (2.89 mg, 0.2 eq.) and phenylsilane (54.12 mg, 20 eq.) were stirred in 5 mL of dichloromethane for 1 h at room temperature under a nitrogen protection condition. The resin was washed with N,N-dimethylformamide (3 × 3 mL), dichloromethane (3 × 3 mL), and N,N-dimethylformamide (3 × 5 mL), respectively, and then dried by draining to obtain 85mg of resin for later use.
3. With DIC (16 mg, 5 eq.) and HOBt (17 mg, 5 eq.) as condensing agents and the allyl-protected amino acid as a raw material, the standard operation procedures of solid-phase synthesis was carried out to obtain 80 mg of resin.
4. 95% of TFA, 2.5% of H2O and 2.5% of triisopropylsilane were added to 80 mg of resin, and the mixture was stired for 2 h at room temperature. 10% DIPEA in N,N-dimethylformamide solution (3 mL) was added to the resin and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining. DIC (32 mg, 10 eq.) and NHS (28 mg, l0 eq.) were stirred for 1 min in 3 ml of N,N-dimethylformamide, and then added to the resin to react for 4 h at 37°C. The resin was washed with N,N-dimethylformamide (3 × 2 mL), dichloromethane (3 × 2 mL), and N,N-dimethylformamide (3 × 2 mL), respectively, and then dried by draining; and HDNA (100 nmol) was dissolved in 3 ml of 100 mM HEPES buffer at pH 8.0 and then added to the resin to react for 16 h at 37 °C . 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 75 mg of resin for later use.
5. 2095 µl of water, 250 µl of 10 × T4 DNA ligase buffer solution, 25 µl of T4 DNA ligase (10000 U), and 58 µl of OP (AAATCGATGTG) (100 nm, 1.74 nm/µl) were added to 75mg of resin to react overnight at room temperature; and the resin was washed with water (3 × 5 mL) and T4 DNA ligase buffer solution (3 × 3 mL), respectively, and then dried by draining to obtain 75 mg of resin for later use. 2095 µl of water, 250 µl of 10 × T4 DNA ligase buffer solution, and 25 µl of T4 DNA ligase (10000 U) were added to 75 mg of resin; the mixture was divided into 96 portions; the DNA tag (1.35 nm, 1 nm/µl) was added to each well to react overnight at room temperature; and the resin was washed with T4 DNA ligase buffer solution (3 × 3 mL), water (3 × 3 mL), and N,N-dimethylformamide (3 × 3 mL), respectively, and then dried by draining for later use. DIC (16 mg, 5 eq.) and HOBt (17 mg, 5 eq.) were dissolved in 5 mL of N,N-dimethylformamide and then divided into 96 portions; each portion together with the amino acid (5 eq.) were added to the resin in each well; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining for later use. The resins were put together, and 5 mL of 20% piperidine in N,N-dimethylformamide solution was added to the resins and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 70mg of resin for later use. All condensation reactions and DNA linkage reactions were repeated 4 times in total; after the fourth round of linkage for the amino acid and the DNA tag, the resins were combined; DIC (16 mg, 5 eq.), HOBt (17 mg, 5 eq.), and Fmoc-Gly-Leu-OH (51 mg, 5 eq.) were stirred for 2 min in 5 ml of N,N-dimethylformamide; then 70 mg of resin was added; the mixture was stirred for 2 h at room temperature; and the resin was washed with N,N-dimethylformamide (3 × 3mL), dichloromethane (3 × 3 mL), and N,N-dimethylformamide (3 × 5 mL), respectively, and then dried by draining; and the resin, tetratriphenylphosphine palladium (2.89 mg, 0.2 eq.) and phenylsilane (54.12 mg, 20 eq.) were stirred in 5 mL of dichloromethane for 1 h at room temperature under a nitrogen protection condition. The resin was washed with dichloromethane (3 × 3 mL) and N,N-dimethylformamide (3 × 3 mL), respectively, and then dried by draining; and then the resulting resin was added to 5 ml of 20% piperidine in N,N-dimethylformamide solution and stirred for 1 h at room temperature. The resin was washed with N,N-dimethylformamide (3 × 3 mL) and water (3 × 5 mL), respectively, and then dried by draining to obtain 65 mg of resin for later use.
6. 2095 µl of water, 250 µl of 10 × T4 DNA ligase buffer solution, 25 µl of T4 DNA ligase (10000 U) and 57 µl of CP (100 nm, 1.74 nm/µl) were added to 65 mg of resin to react overnight at room temperature; the resin was washed with T4 DNA ligase buffer solution (3 × 3 mL) and water (3 × 5 mL), respectively, and then dried by draining for later use; 10 mg of resin was swollen in 830 µl of water; 100 µl of sodium acetate buffer solution (PH=5.0, 0.5 M), 2 µl of sodium chloride solution (0.25 M), 20 µl of disodium ethylenediamine tetraacetate solution (0.05 M), 10 µl of TCEP solution (0.05 M) and 30 µl of OaAEP3 (1.34 mg/mL) were added respectively for ring-closure reaction; and the mixture underwent reaction overnight at 37 degrees centigrade to obtain a DNA-encoded cyclic peptide library. When the ring-closure reaction was performed, a fragment -GL was removed from the A-end ring-closure molecule.

### Example 8: Library Construction Based on Different A-end Ring Closure

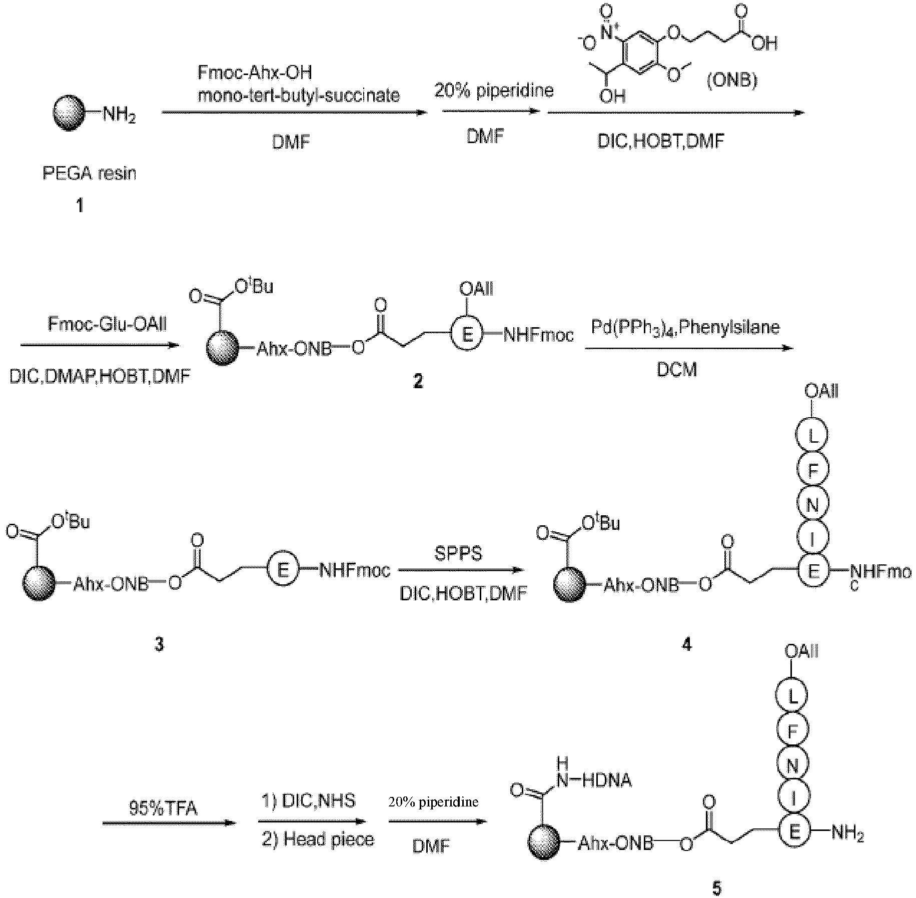

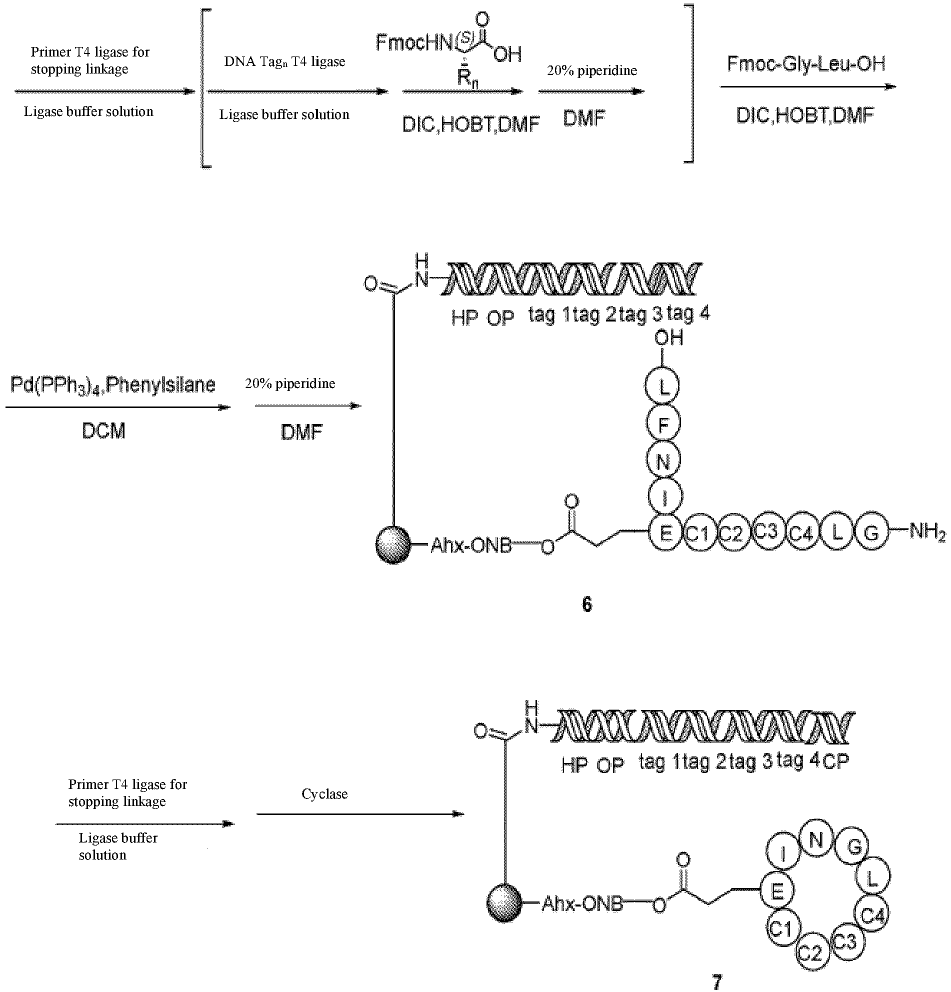

Synthesis method:
Referring to the methods for resin synthesis and database construction in Example 7, 55 mg of a solid-phase DNA-encoded cyclic peptide library was finally obtained from 100 mg of starting resin. When the ring-closure reaction was performed, a fragment -FL was removed from the A-end ring-closure molecule.

### Example 9: Library Construction Based on Different A-end Ring Closure

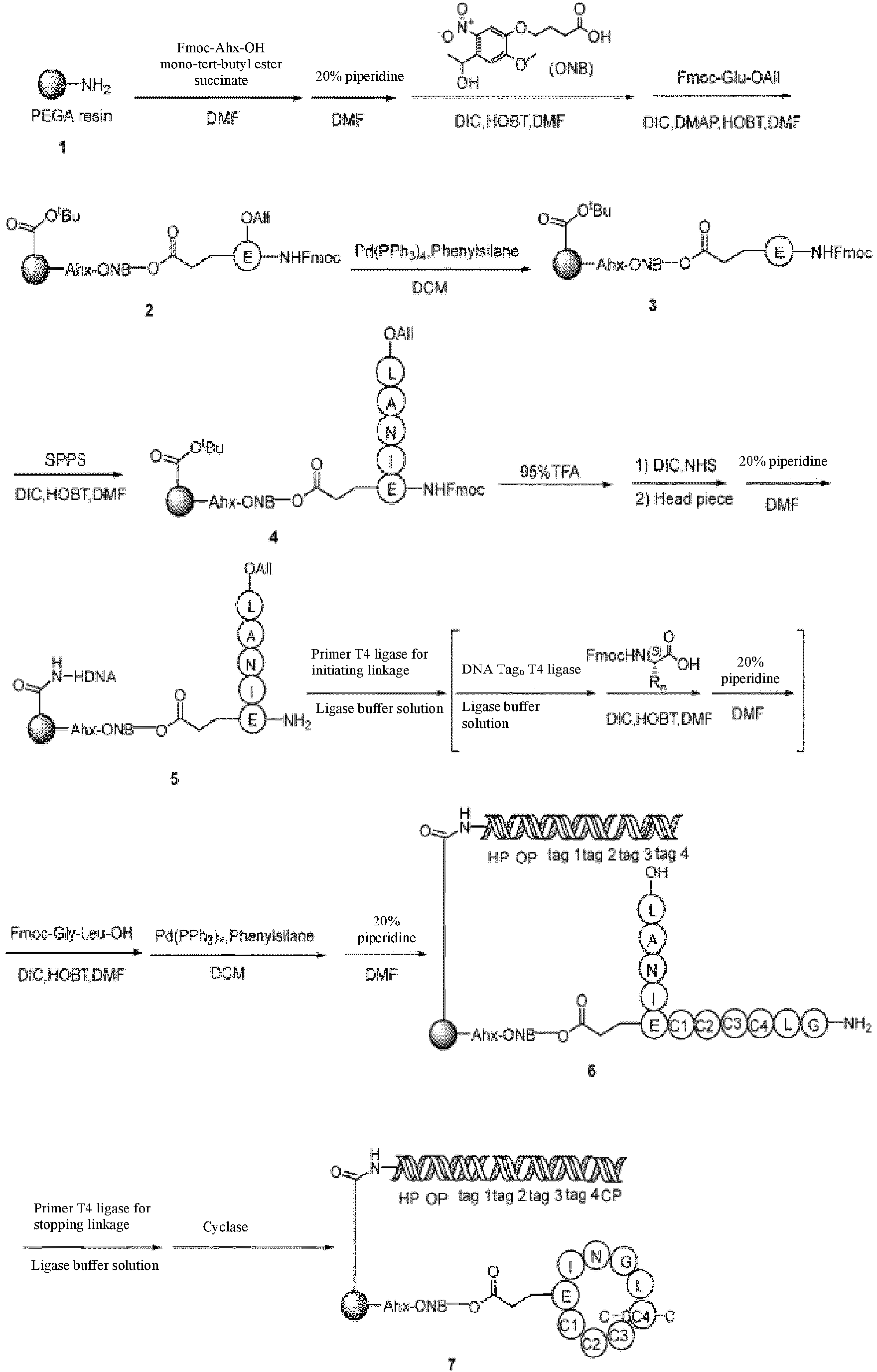

Synthesis method:
Referring to the methods for resin synthesis and database construction in Example 7, 50 mg of a solid-phase DNA-encoded cyclic peptide library was finally obtained from 100 mg of starting resin. When the ring-closure reaction was performed, a fragment -AL was removed from the A-end ring-closure molecule.

### Example 10: Synthesis of DEL of the Present Invention Using ONB-protected Polypeptide

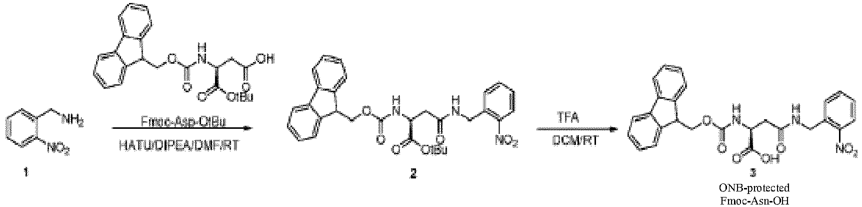

In step 1, ONB-protected Fmoc-Asn-OH was synthesized, and this protective group can be completely removed under UV illumination at 365 nm.
(1) A compound (2-nitrophenyl)methanamine hydrochloride (1.9 g, 10 mmol), Fmoc-Asp-OtBu (5 g, 12 mmol) and DIPEA (3.9 g, 15 mmol) were dissolved in 20 ml of N,N-dimethylformamide; HATU (5.8 g, 15 mmol) was added at 0 degree centigrade; the mixture was stirred for 2 h at room temperature, and the reaction was quenched with water; 10 ml of ethyl acetate was added; the aqueous phase was extracted three times with ethyl acetate; the organic phases were combined, dried over anhydrous sodium sulfate, filtered, and spun dry to obtain a crude product. The crude product was let to flow through the column with ethyl acetate as an eluent to obtain a white solid (4.5 g, 82.6%).
(2) The resulting compound (4.5 g, 8.25 mmol) from the previous step were added to 50 ml of 25% trifluoroacetic acid in dichloromethane solution to react for 2 h at room temperature. The mixture was spun dry and purified (with 6% methanol in dichloromethane solution as an eluent) through the column to obtain a white solid (3.5 g, 86.7%). 1HNMR(400MHz, DMSO) δ 8.50(t,J=5.7Hz,1H), 8.03(d,J=8.1Hz, 1H),7.90(d,J=7.4Hz,2H), 7.71(d,J=7.3Hz,2H), 7.67-7.59(m,2H), 7.59-7.48(m,2H), 7.42(t,J=7.4Hz,2H), 7.33(dd,J=6.9,4.5Hz,2H),4.56(d,J=4.2Hz,2H),4.47-4.36(m,1H),4.26(dt,J=13.7,7.2Hz,3H), 2.74(dd,J=15.1,5.6Hz,1H),2.65-2.58(m,1H).LCMS:490.2(M+H)⁺.

In step 2, the ONB-protected peptide was used for the synthesis of a DEL, and the removal effect of the ONB protective group was tested under ultraviolet illumination at 365 nm.

For the operation method, refer to the synthesis process of the DEL of Example 1. Under ultraviolet illumination at 365 nm, the ONB protective group can be removed from the compound 15, which can be simultaneously isolated from the resin to obtain a compound 16. A preparation method included the following steps.

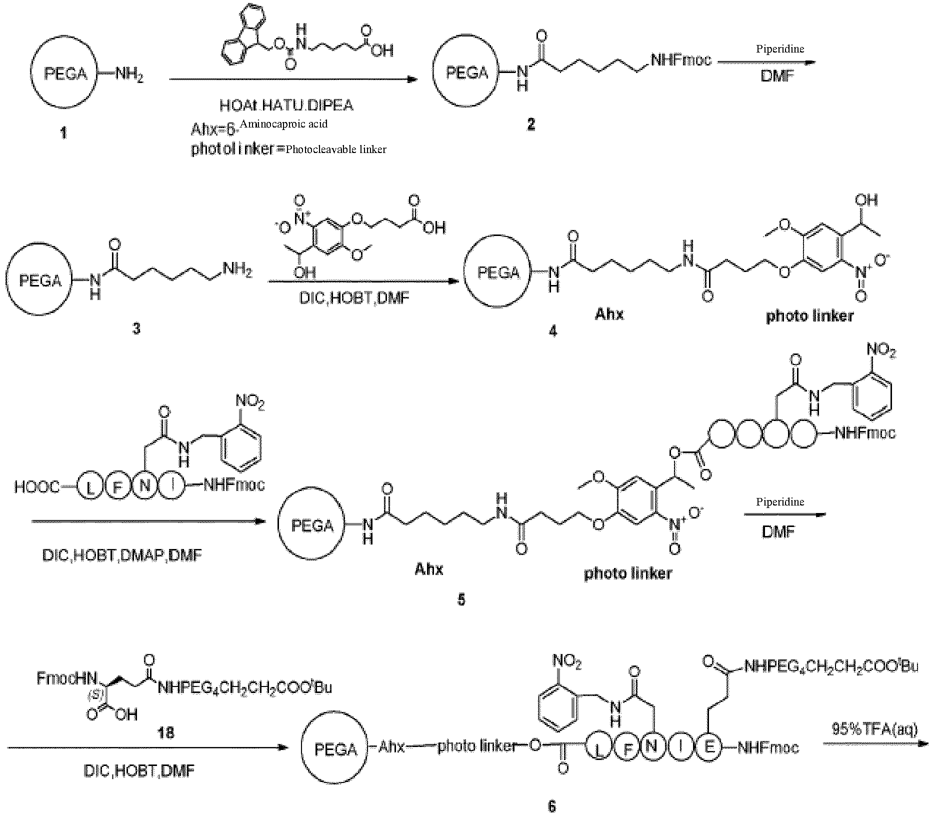

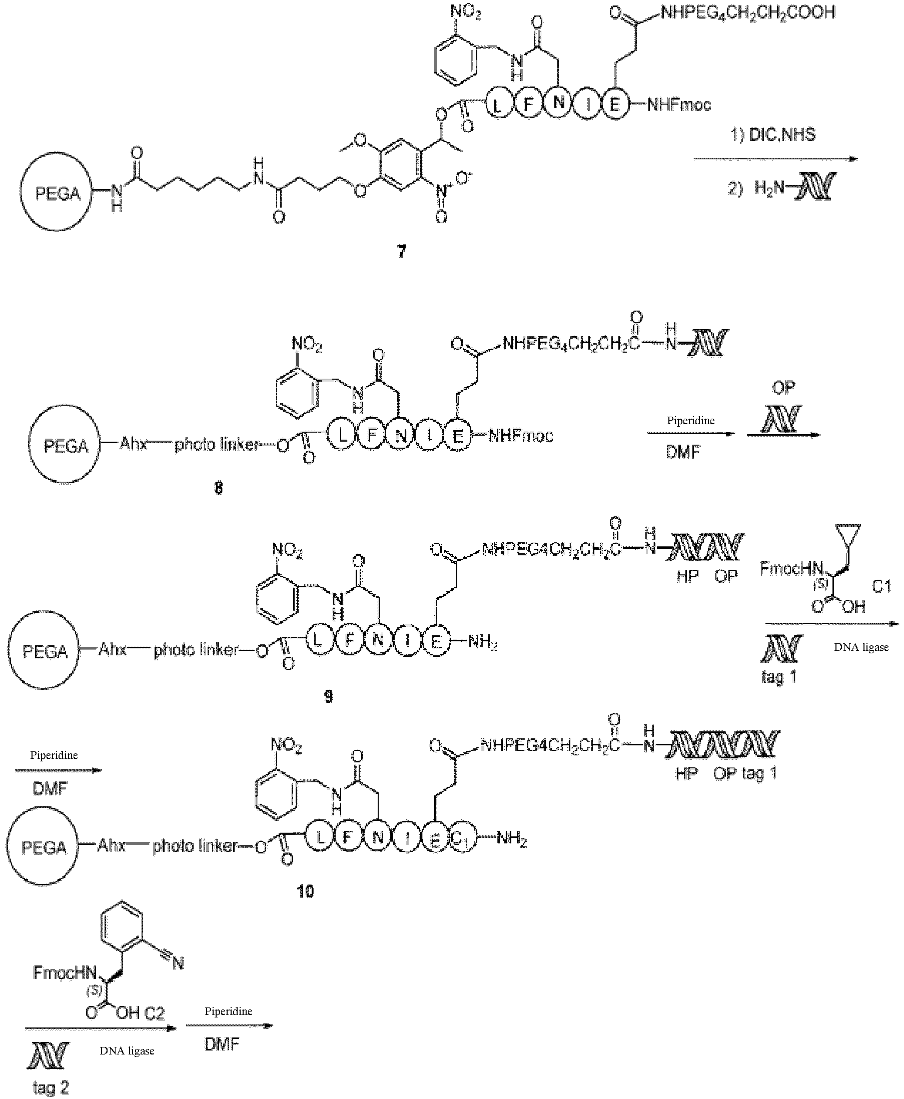

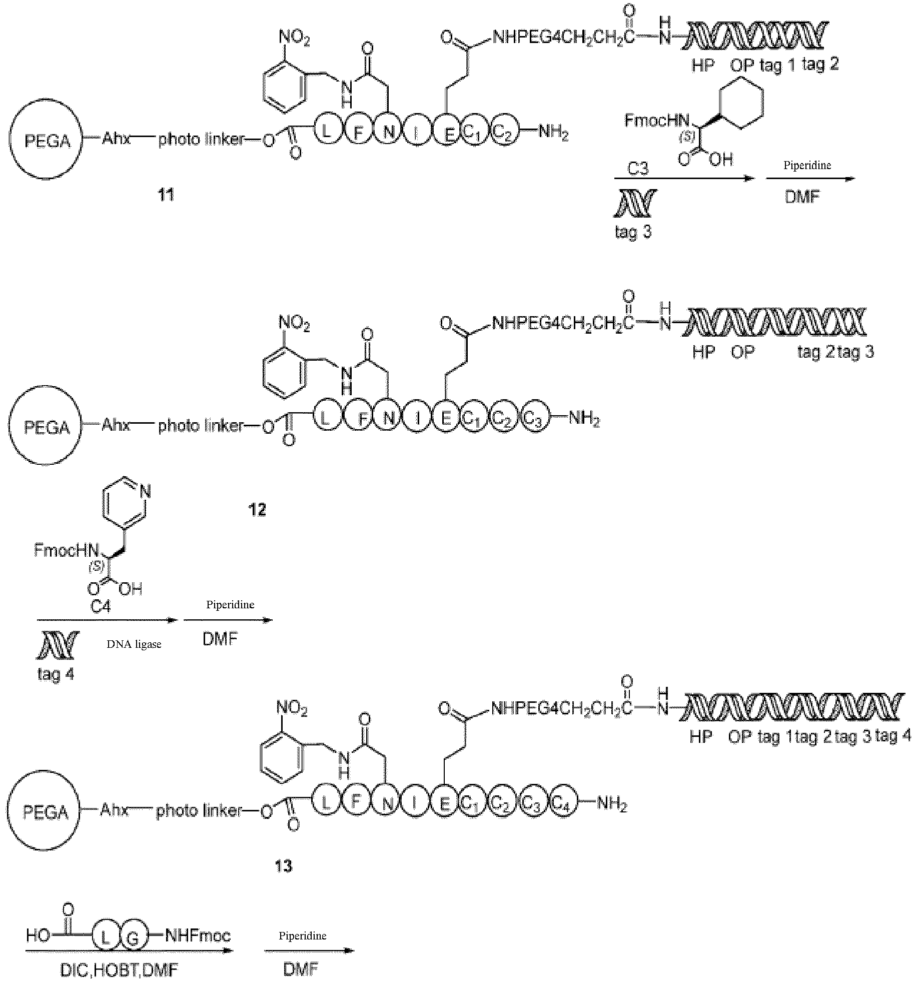

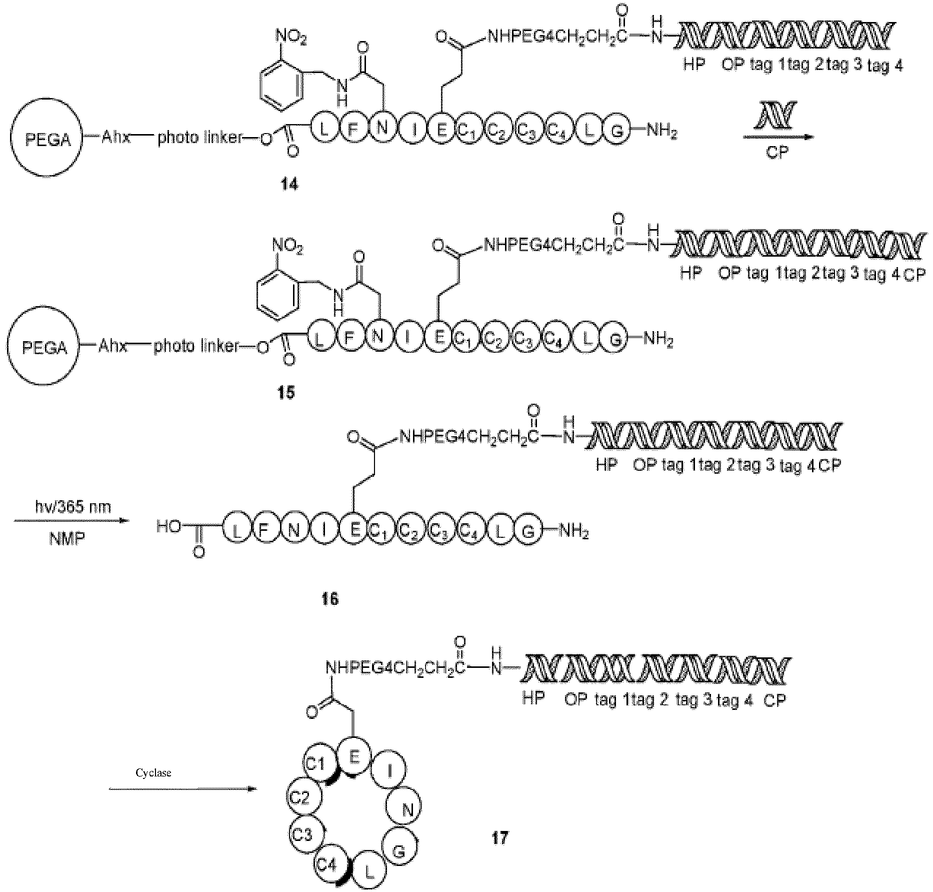

### Example 11: Verification of Ring Closure Method for Compound Having One Side Chain on a Ring

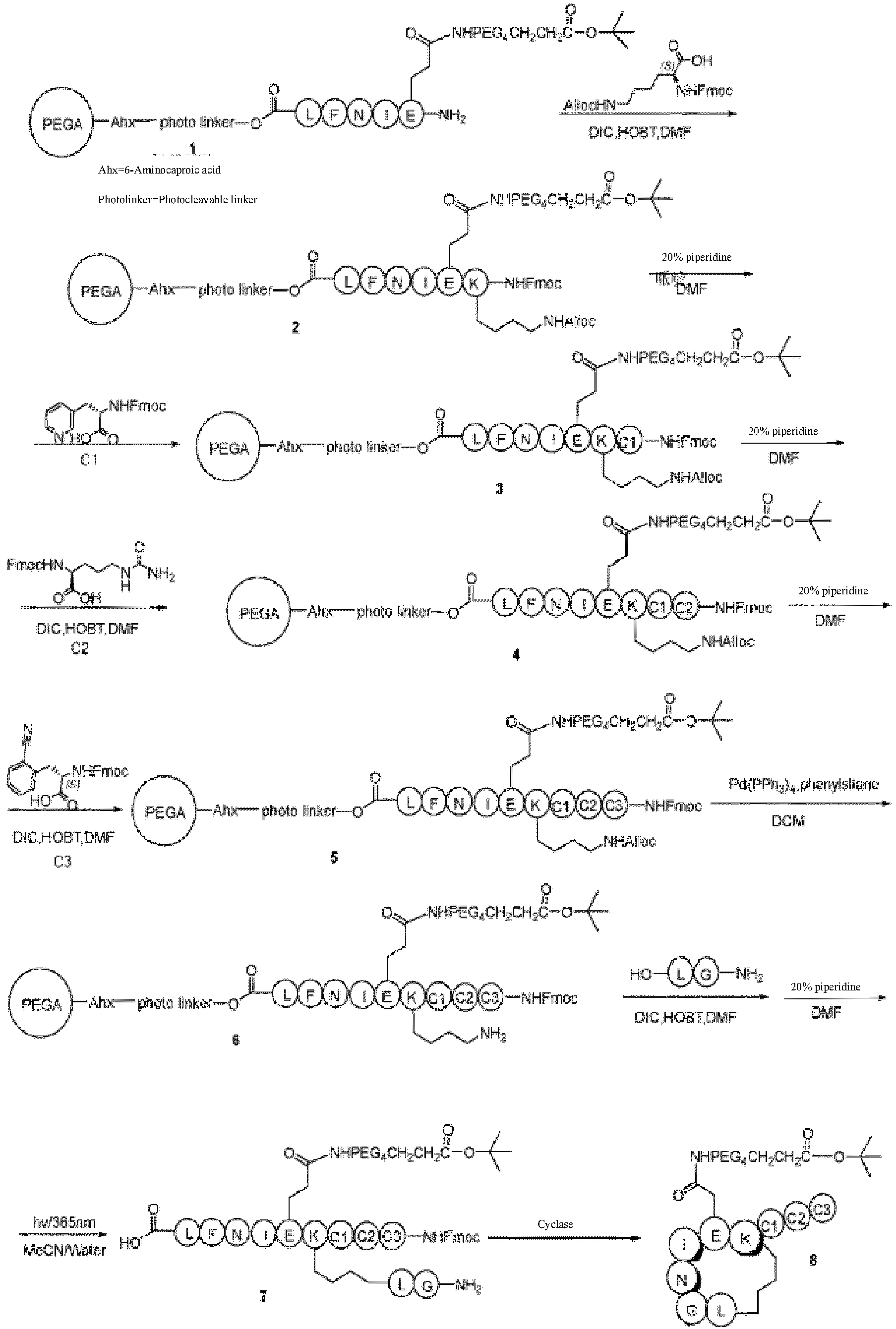

In step 1, DIC (16 mg, 5 eq.), HOBt (17 mg, 5 eq.), and Fmoc-Lys(Alloc)-OH (57 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 100 mg of resin for later use.

In step 2, 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100mg of resin for later use. DIC (16 mg, 5 eq.), HOBt (17 mg, 5 eq.), and a building block Al (49 mg, 5 eq.) were treated by referring to the condensation operation of step 1 to obtain 95 mg of resin.

In step 3, with 5 mL of 20% piperidine in N,N-dimethylformamide solution, DIC (16 mg, 5 eq.), HOBt (17 mg, 5 eq.) and Fmoc-L-citrulline (a building block A2) (50 mg, 5 eq.), 95 mg of resin was obtained by referring to the Fmoc removal and condensation operations in step 1.

In step 4, with 5 mL of 20% piperidine in N,N-dimethylformamide solution, DIC (16 mg, 5 eq.), HOBt (17 mg, 5 eq.) and a building block A3 (52 mg, 5 eq.), 90 mg of resin was obtained by referring to the Fmoc removal and condensation operations in step 1.

In step 5, 90 mg of resin, tetratriphenylphosphine palladium (2.89 mg, 0.2 eq.) and phenylsilane (54.12 mg, 20 eq.) were stirring in 5 mL of dichloromethane for 1 h at room temperature under a nitrogen protection condition. The resin was washed with N,N-dimethylformamide (3 × 3 mL), dichloromethane (3 × 3 mL), and N,N-dimethylformamide (3 × 5 mL), respectively, and then dried by draining to obtain 85mg of resin for later use.

In step 6, DIC (16 mg, 5 eq.), HOBt (17 mg, 5 eq.), Fmoc-Gly-Leu-OH (50 mg, 5 eq.), and 5 mL of 20% piperidine in N, N-dimethylformamide solution were treated by referring to the condensation and Fmoc removal operations in step 1 to obtain 85 mg of resin. 85 mg of resin was added to the mixed solution of 0.5 ml of acetonitrile and 0.5 mL of water, and illuminated for 5 h under UV light at 365 nm; the resin was filtered, dialyzed with a solvent, and then lyophilized to obtain 5 mg of a compound 7.

In step 7, 0.25 mg of the compound 7 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 37 °C to obtain a cyclic compound 8. LCMS:1437.06(M+H)⁺.

### Example 12: Verification of Ring Closure Method for Compound Having One Side Chain on a Ring

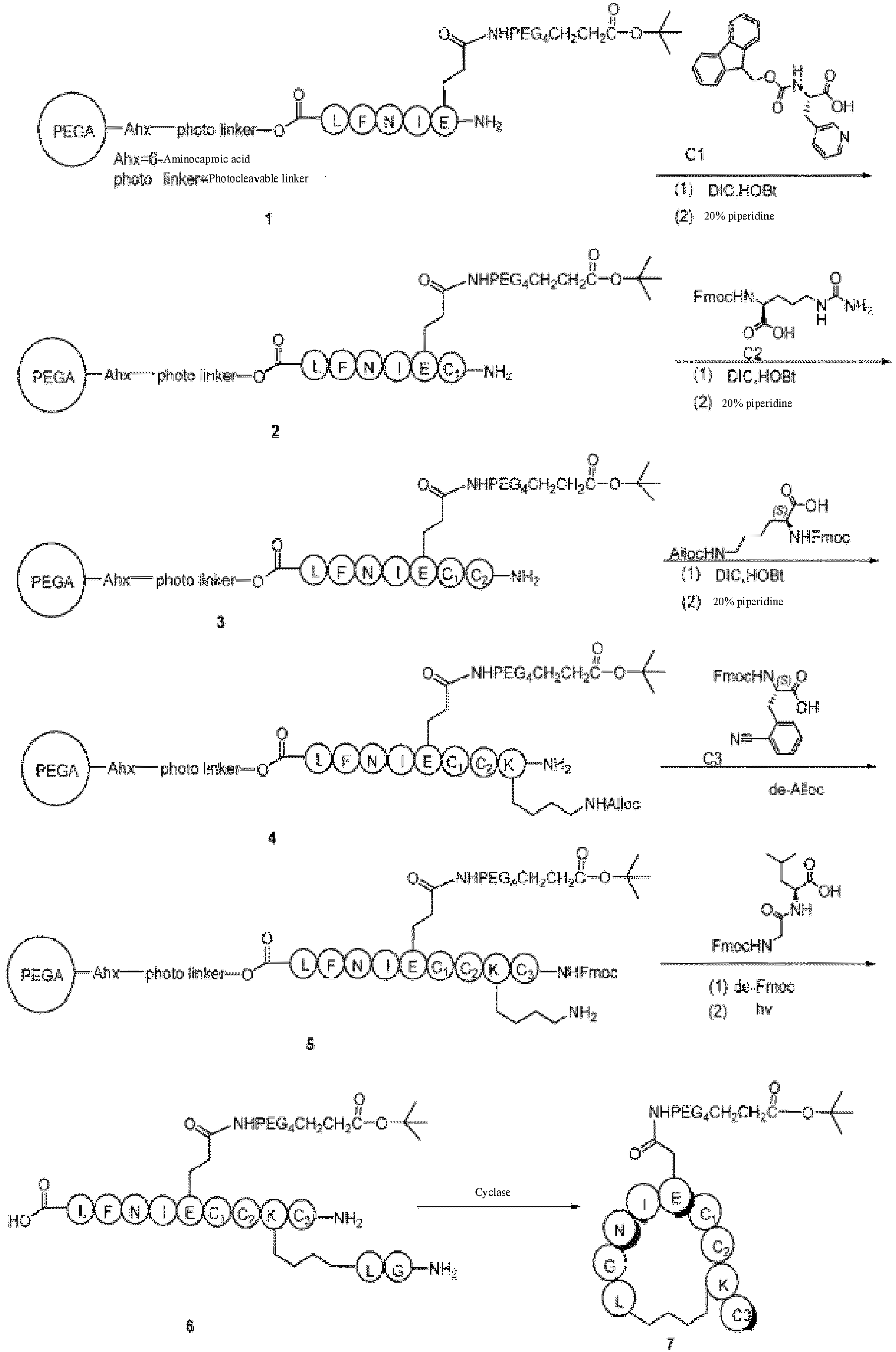

In step 1, DIC (16 mg, 5 eq.), HOBt (17 mg, 5 eq.), and a building block A1 (s) -2-((((9H-fluoren-9-yl)methoxy)carbonyl)amino)3-(pyridin-3-yl)propionic acid (50 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 100mg of resin for later use. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100 mg of resin for later use.

In step 2, DIC (16 mg, 5 eq.), HOBt (17 mg, 5 eq.), and a building block A2 (50 mg, 5 eq.) were treated by referring to the condensation operation in step 1 to obtain 95 mg of resin. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 95 mg of resin for later use.

In step 3, DIC (16 mg, 5 eq.), HOBt (17 mg, 5 eq.), and Fmoc-Lys(Alloc)-OH (57 mg, 5 eq.) were treated by referring to the Fmoc removal and condensation operations in step 1 to obtain 95 mg of resin.

In step 4, DIC (16 mg, 5 eq.), HOBt (17 mg, 5 eq.), and a building block A3 (52 mg, 5 eq.) were treated by referring to the condensation and Fmoc removal operations in step 1 to obtain 90 mg of resin.

In step 5, DIC (16 mg, 5 eq.), HOBt (17 mg, 5 eq.), Fmoc-Gly-Leu-OH (52 mg, 5 eq.), and 5 mL of 20% piperidine in N, N-dimethylformamide solution were treated by referring to the condensation and Fmoc removal operations in step 1 to obtain 85 mg of resin. 85 mg of resin was added to the mixed solution of 0.5 mL of acetonitrile and 0.5 mL of water, and illuminated for 5 h under UV light at 365 nm; and the resin was filtered, dialyzed with a solvent, and then lyophilized to obtain 5 mg of a compound 6.

In step 6, 0.25 mg of the compound 6 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 37°C to obtain a cyclic molecule compound 7. LCMS:1436.11(M+H)⁺.

### Example 13: Application of Cyclic Molecule Compound Library of the Present Invention in Protac

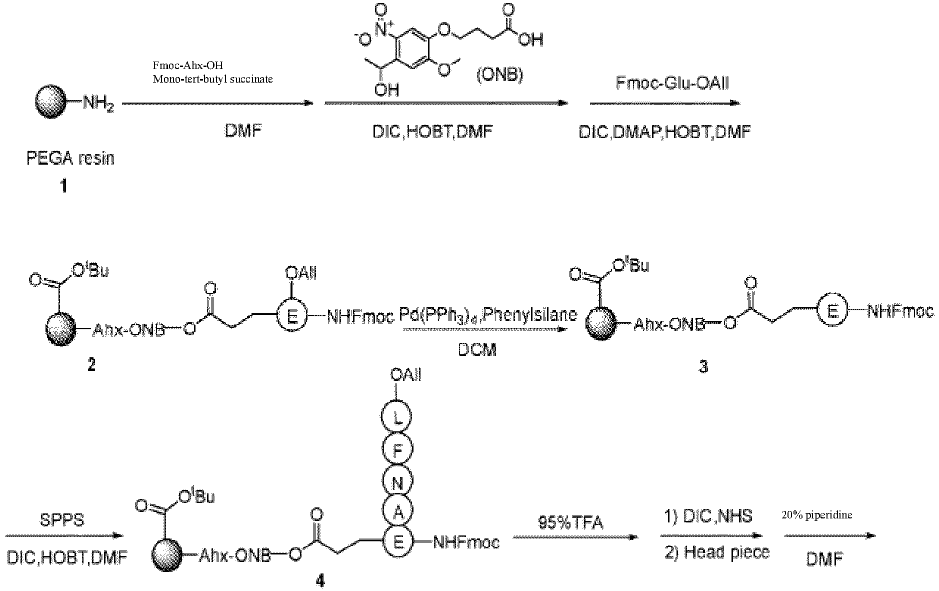

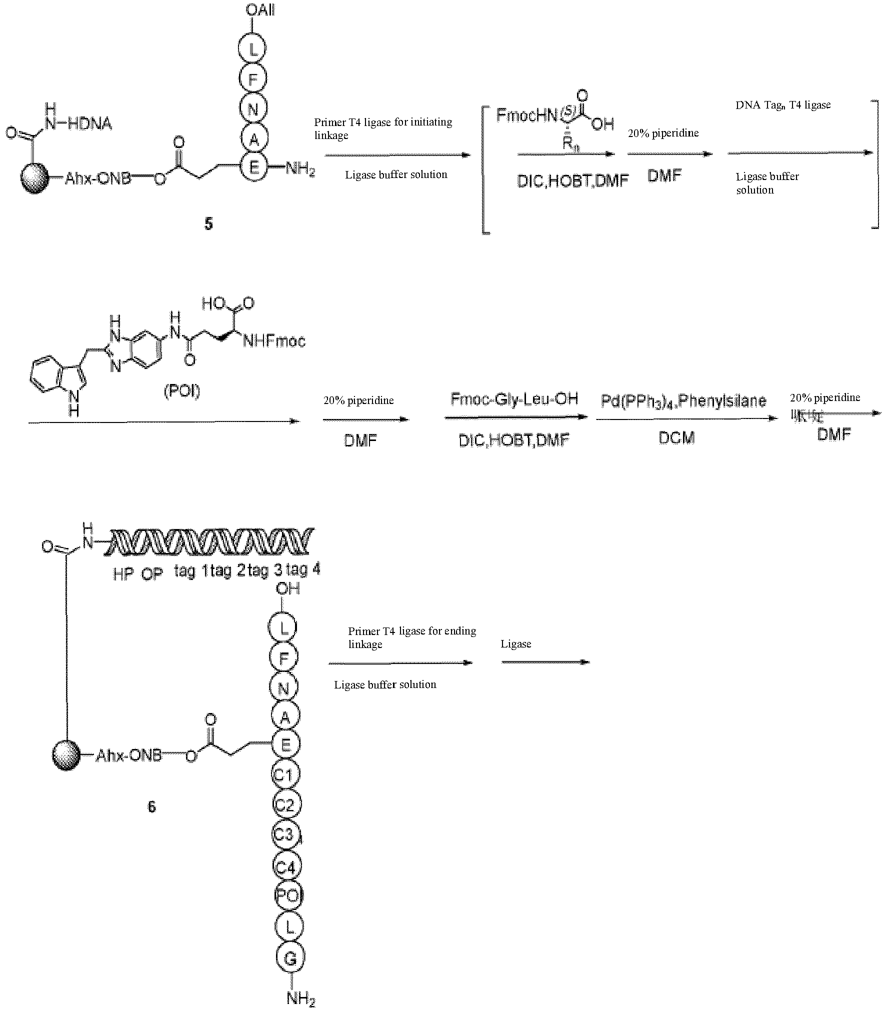

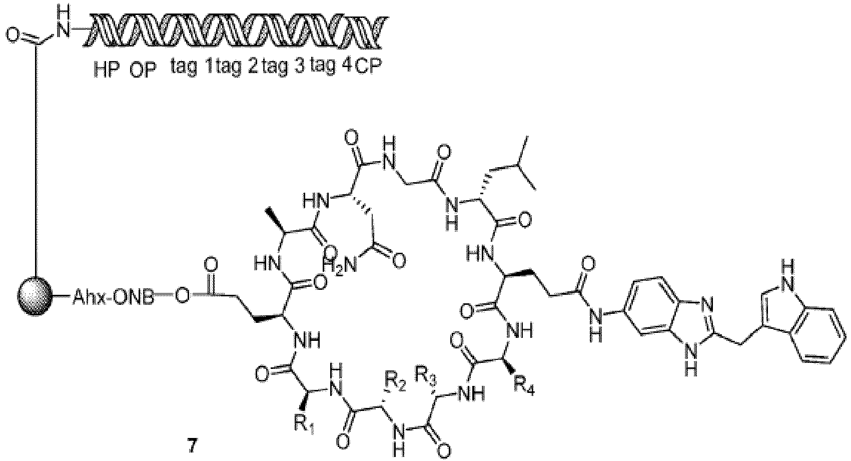

Synthesis method:
Referring to the synthesis process for synthesizing the DEL cyclic molecule library from the above-mentioned resin having double functional groups, POI is added to the compound library as a specific building block to synthesize the DEL cyclic molecular library with specific functions and structures.

### Example 14: Application of DEL Cyclic Molecule Library in PROTAC Technology: (Target Protein Substrate in a Side Chain)

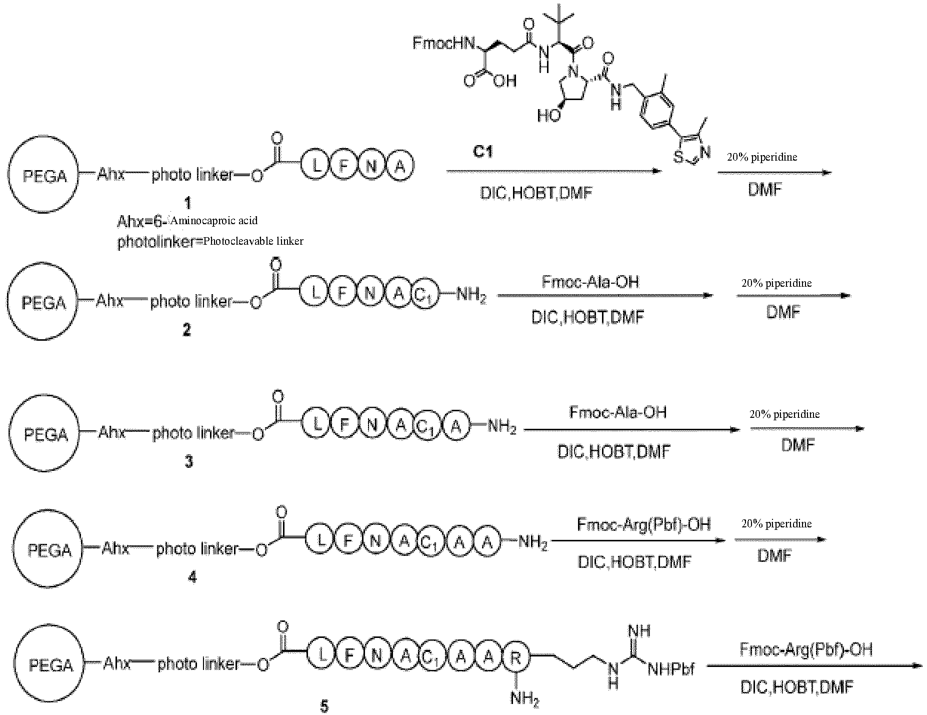

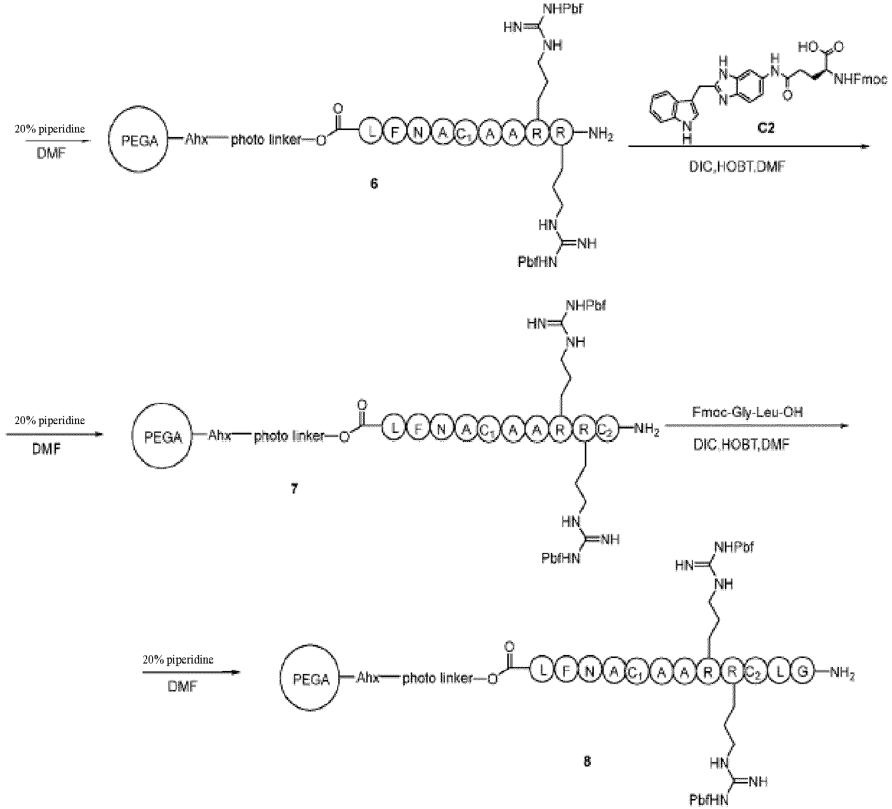

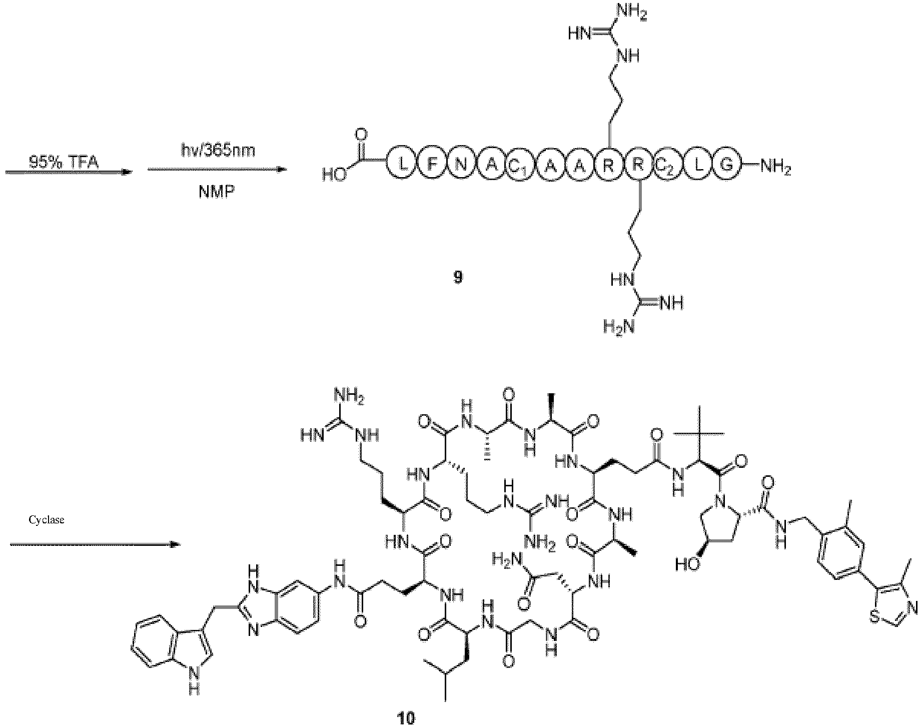

### Method steps:

In step 1, DIC (16 mg, 5 eq.), HOBt (17 mg, 5 eq.), and Fmoc-AAi-OH (100 mg, 5 eq.) were stirred in 3 ml of N,N-dimethylformamide for 10 min; then, 100 mg of the prepared resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100mg of resin for later use.

In step 2, according to the standard solid-phase synthesis operation of step 1, the amino acids were linked sequentially to obtain 85 mg of a compound 8.

In step 3, 95% of trifluoroacetic acid, 2.5% of water and 2.5% of triisopropylsilane was added to 85 mg of resin and stirred for 2 h at room temperature. 10% DIPEA in N,N-dimethylformamide solution (3 mL) was added to the resin and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining. 50 mg of resin was added to 3 mL of NMP solution, and illuminated for 5 h under UV light at 365 nm; and the resin was filtered, dialyzed with a solvent, and then lyophilized to obtain 8mg of a compound 9.

In step 4, 0.6 mg of the compound 9 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 37°C to obtain a cyclic molecule compound 10. LCMS:1740.04(M+H)⁺.

### Example 15: Application of DEL Cyclic Molecule Library in PROTAC: (Target Protein Substrate inside a Ring)

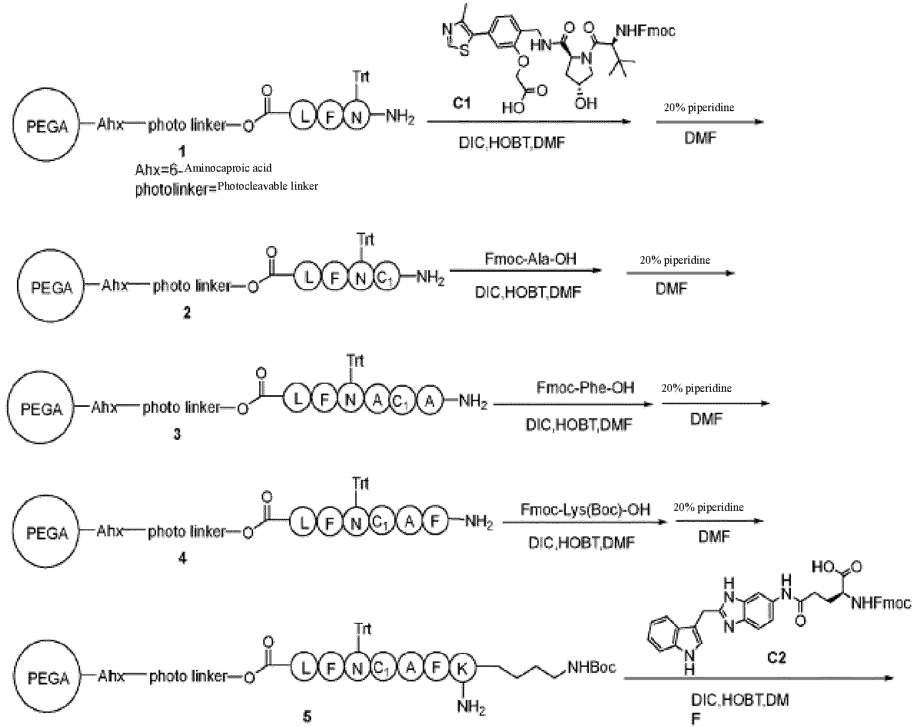

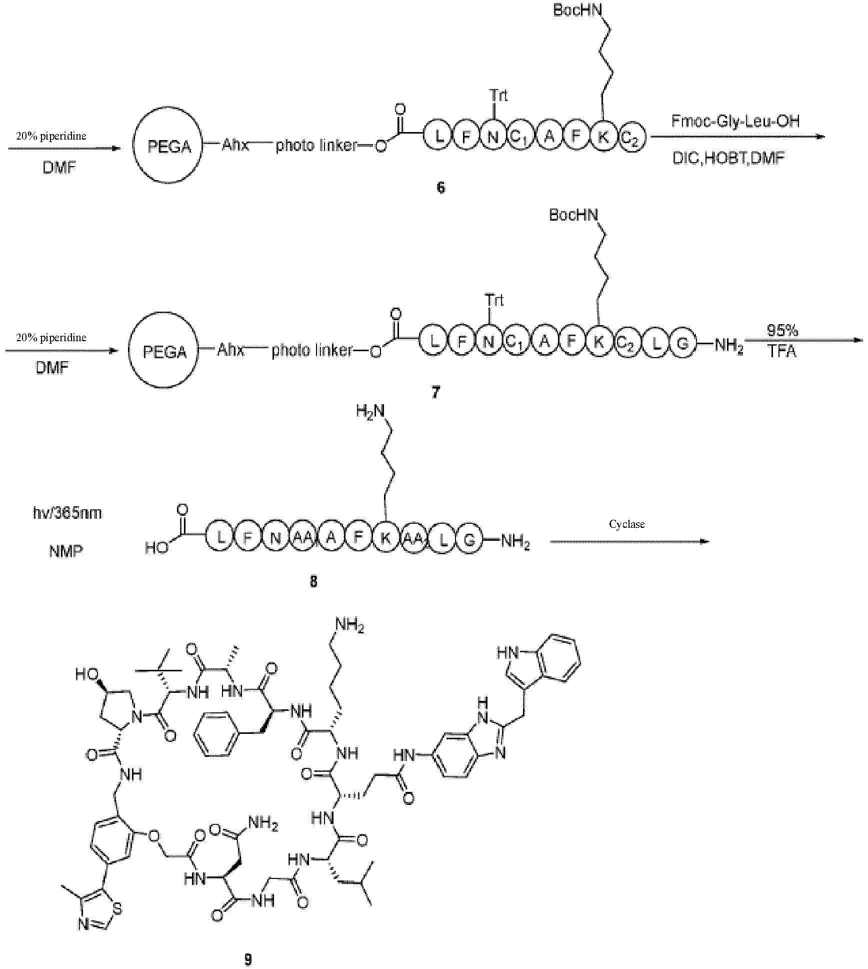

In step 1, DIC (16 mg, 5 eq.), HOBt (17 mg, 5 eq.), and Fmoc-AAi-OH (93 mg, 5 eq.) were stirred in 3 ml of N,N-dimethylformamide for 10 min; then, 100 mg of the prepared resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100 mg of resin for later use.

In step 2, according to the standard solid-phase synthesis operation of step 1, the amino acids were linked sequentially to obtain 80 mg of a compound 7.

In step 3, 95% of trifluoroacetic acid, 2.5% of water and 2.5% of triisopropylsilane were added to 80 mg of resin and stirred for 2 h at room temperature. 10% DIPEA in N,N-dimethylformamide solution (3 mL) was added to the resin and stirred for 2 hours at room temperature. The resin was washed with N,N-dimethylformamide (3 × 3 mL), dichloromethane (3 × 3 mL), and N,N-dimethylformamide (3 × 5 mL), respectively, and then dried by draining. 50 mg of resin was added to 3 mL of NMP solution, and illuminated for 5 h under UV light at 365 nm; and the resin was filtered, dialyzed with a solvent, and then lyophilized to obtain 8mg of a compound 8.

In step 4, 0.5mg of the compound 8 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 37 °C to obtain a cyclic peptide compound 9. LCMS:1490.8(M+H)⁺.

### Example 16: Verification of Ring Closure Method for Double Rings

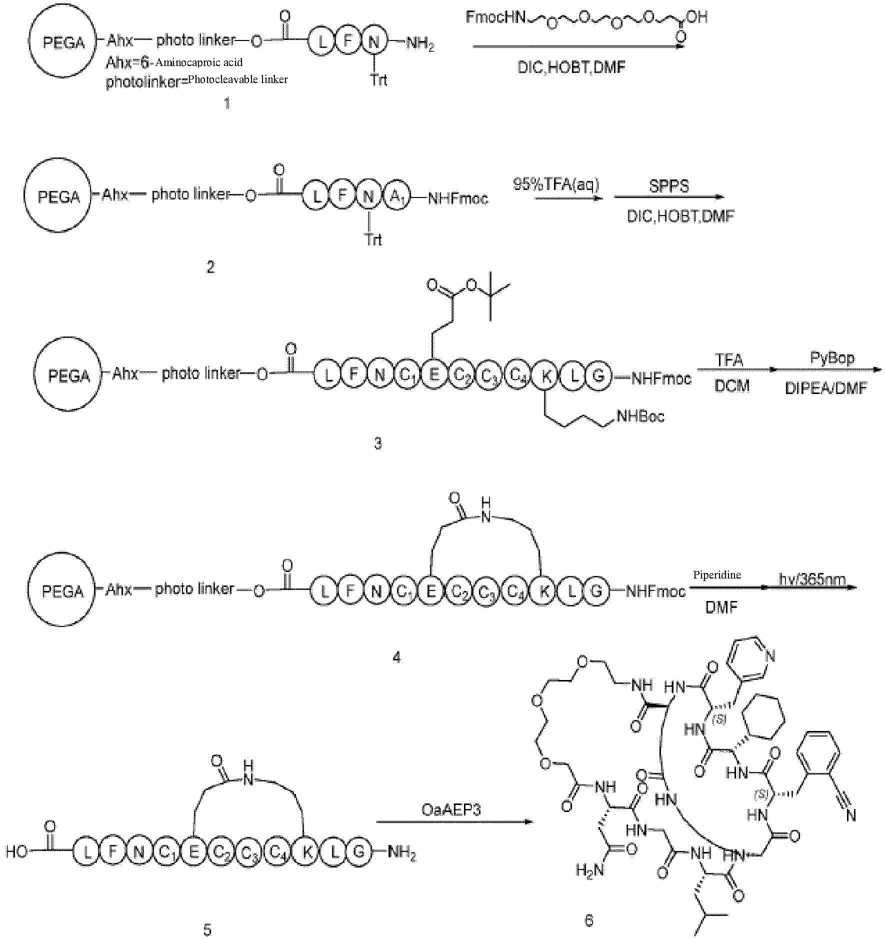

### Synthesis method:

In step 1, DIC (16 mg, 5 eq.), HOBt (17 mg, 5 eq.), and Fmoc-NH-PEG₃CH₂COOH (55 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), N,N-dimethylformamide (3 × 5 mL) and H2O (3 × 3 mL), respectively, and then dried by draining to obtain 100 mg of resin for later use.

In step 2, 2mL of 95% trifluoroacetic acid in water was added to the resin and then stirred for 2 days at room temperature; the resin was washed with H2O (3 × 3 mL), N,N-dimethylformamide (3 × 5 mL), dichloromethane (3 × 3 mL), and N,N-dimethylformamide (3 × 5 mL), respectively, and then dried by draining to obtain 100 mg of resin for later use; 5 mL of 50% DIPEA in N,N-dimethylformamide solution was added to the resin and stirred for 1 h at room temperature; and the resin was washed with N,N-dimethylformamide (3 × 5 mL), dichloromethane (3 × 3 mL), and N, N-dimethylformamide (3 × 5 mL) respectively, and then dried by draining to obtain 95 mg of resin for later use. The resin was treated according to the standard solid-phase polypeptide synthesis operation to obtain 92 mg of resin, in which DIC (16 mg, 5eq.), HOBt (17 mg, 5eq.), and a Fmoc-protected amino acid (60 mg, 5 eq.) were used.

In step 3, 5 mL of 50% trifluoroacetic acid in dichloromethane solution was added to the resin and stirred for 1 hour at room temperature; the resin was washed with N,N-dimethylformamide (3 × 5 mL), dichloromethane (3 × 3 mL), and N,N-dimethylformamide (3 × 5 mL), respectively, and then dried by draining to obtain 87 mg of resin for later use; PyBop (27 mg, 2 eq.) and DIPEA (17 mg, 5 eq.) were stirred for 1 min in 3 ml of N,N-dimethylformamide, and then added to the resin; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 85 mg of resin for later use. In this step of reaction, the side chain of the building block glutamic acid (E) was linked to the side chain of the building block methionine (K) to form a cyclic structure.

In step 4, 85 mg of the resin was added to 5 mL of 20% piperidine in N,N-dimethylformamide solution, and then stirred for 1 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 80 mg of resin for later use. 80 mg of resin was added to the mixed solution of 0.5 mL of acetonitrile and 0.5 mL of water, and illuminated for 5 h under UV light at 365 nm; and the resin was filtered, dialyzed with a solvent, and then lyophilized to obtain 4 mg of a compound 5.

In step 5, 0.25 mg of the compound 5 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 37°C to obtain a cyclic peptide compound 6, which is a bicyclic-structure compound.

LCMS:1172.(M+H)⁺.

From the above verification results, it can be seen that a bicyclic compound library can be constructed by the construction method of the present application. The building block E and the building block K each provide an extra-ring outer side chain, and the two extra-ring side chains are linked by chemical reactions to form a cyclic structure. The bicyclic structure of the library compound obtained in this example is a bridge ring, and one or more synthetic building blocks are shared between two rings.

### Example 17: Ring-closure Verification Test for Double Rings

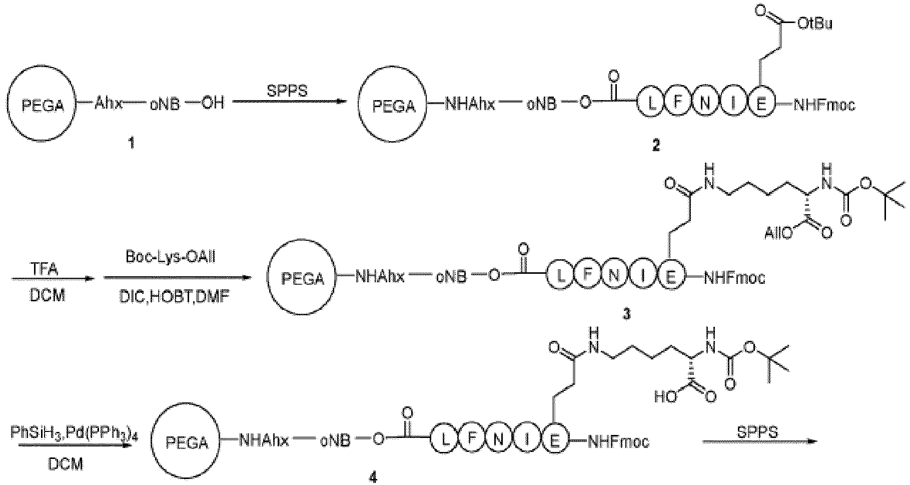

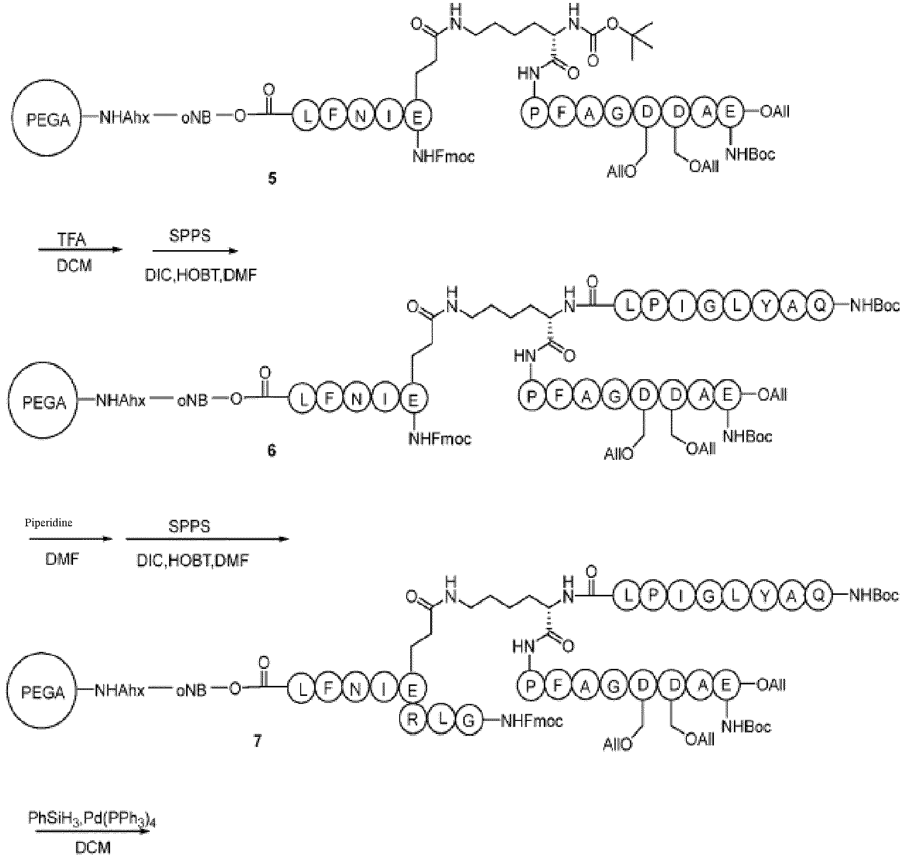

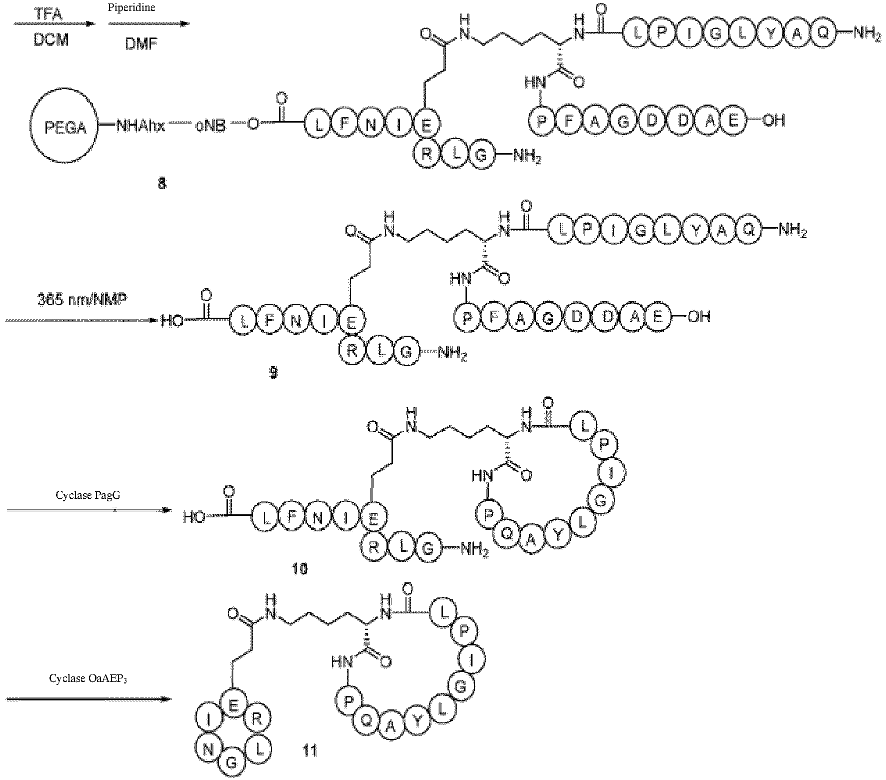

### Synthesis method:

1. The PEGA resin (100 mg, 25 µmol) was swollen for 2 hours in 3 ml of N,N-dimethylformamide. According to the standard solid-phase polypeptide synthesis operation, the assembly with the A-end ring-closure molecule (LFNI tetrapeptide) was first carried out, and then the assembly with Fmoc-O-tert-butyl 1-glutamic acid (i.e., the linker L1' having three functional groups) to obtain 95 mg of resin (a compound 2).
2. 95% of TFA, 2.5% of H₂O and 2.5% of triisopropylsilane were added to 95 mg of the resulting resin from the previous step and stirred for 2 h at room temperature. 10% DIPEA in N,N-dimethylformamide solution (3 mL) was added to the resin and stirred for 2 h at room temperature. The resin was washed with N,N-dimethylformamide (3 × 3 mL), dichloromethane (3 × 3 mL), and N,N-dimethylformamide (3 × 5 mL), respectively, and then dried by draining. DIC (32 mg, 10 eq.), HOBt (35 mg, 10 eq.), and >Boc-Lys-OAll (76 mg, 10 eq., i.e., the linker L1" having three functional groups) were stirred for 1 min in 3 ml of N,N-dimethylformamide and then added to the resin to react for 1 hour, such that the linker L1" having three functional groups was assembled with the linker L1' having three functional groups to form a linker L1 having four functional groups. The resin was washed with N,N-dimethylformamide (3 × 2 mL), dichloromethane (3 × 2 mL), and N,N-dimethylformamide (3 × 2 mL), respectively, and then dried by draining to obtain 85 mg of resin (a compound 3).
3. 85 mg of resin, tetratriphenylphosphine palladium (3 mg, 0.2 eq.) and phenylsilane (60.12 mg, 20 eq.) were stirred in 5 ml of dichloromethane for 1 h at room temperature under a nitrogen protection condition. The resin was washed with dichloromethane (3 × 3 mL) and N,N-dimethylformamide (3 × 3 mL), respectively, and then dried by draining to obtain 80 mg of resin (a compound 4).
4. The allyl ester-protected amino acid was treated according to the standard solid-phase polypeptide synthesis operation to obtain 70 mg of resin (a compound 5).
5. 95% of TFA, 2.5% of H2O and 2.5% of triisopropylsilane were added to 70 mg of resin, and the mixture was stired for 2 h at room temperature. 10% DIPEA in N,N-dimethylformamide solution (3 mL) was added to the resin and stirred for 2 hours at room temperature. The resin was washed with N,N-dimethylformamide (3 × 3mL) respectively and then dried by draining, and the amino acid protected by tert-butoxycarbonyl was treated according to the standard solid-phase polypeptide synthesis operation to obtain 60 mg of resin (a compound 6).
6. 60 mg of the resin was added to 5 mL of 20% piperidine in N,N-dimethylformamide solution, and then stirred for 1 h at room temperature. The resin was washed with N,N-dimethylformamide (3 × 3 mL), dichloromethane (3 × 3 mL) and N,N-dimethylformamide (3 × 5 mL), respectively, and then dried by draining, and the Fmoc-protected amino acid was treated according to the standard solid-phase polypeptide synthesis operation to obtain 55mg of resin (a compound 7).
7. Allyl, tert-butoxycarbonyl and 9-fluorenyl methoxycarbonyl were sequentially removed from the resin according to the above deprotection method to obtain 45 mg of resin (a compound 8).
8. 45 mg of resin was added to 1 mL of NMP solution, and illuminated for 5 h under UV light at 365 nm; and the resin was filtered, dialyzed with a solvent, and then lyophilized to obtain 5mg of a compound 9.
9. 20% DMSO, 100 µM polypeptide compound 9 and 20 µM PagG were added to MES buffer of 10 m MPH=7.0. The reaction occurred in the mixture for 16 h at 25°C. The reactant was boiled for 5 min to quench the reaction, and then lyophilized to obtain a compound 10.
10. The compound 10 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively for ring-closure reaction; and the mixture underwent reaction overnight at 45°C to close the two rings at the same time, thereby obtaining a cyclic peptide compound 10, which is a bicyclic-structure compound. LCMS:1746.1(M+H)⁺.

From the above verification results, it can be seen that the bicyclic compound library can be constructed by the construction method of the present application, which is in particular applied to the screening of bicyclic peptide molecules. The bicyclic or polycyclic structure can stabilize the conformation of macrocyclic molecules, improve the rigidity of cyclic structures, improve the stability of cyclic peptide molecules, and prolong the half-life of cyclic peptide drugs, showing a better application prospect.

### Example 18: Verification Test for Ring Closure

H₂N-GLRINGL-COOH → Cycle(GLRIN)

0.25 mg of a polypeptde was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively for ring-closure reaction; and the mixture underwent reaction overnight at 37°C to obtain a product.

The LC-MS verified that the product had a molecular weight of 554(M+H)⁺, and was available for ring closure.

### Example 19: Verification Test for Ring Closure

H₂N-GLIRIKIRIKNGL-COOH → Cycle(GLIRIKIRIKN)

0.5mg of a polypeptde was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively; and the mixture underwent reaction overnight at 37°C to obtain a product.

The LC-MS verified that the product had a molecular weight of 1306.5(M+H)⁺, and was available for ring closure.

### Example 20: Verification Test for Ring Closure

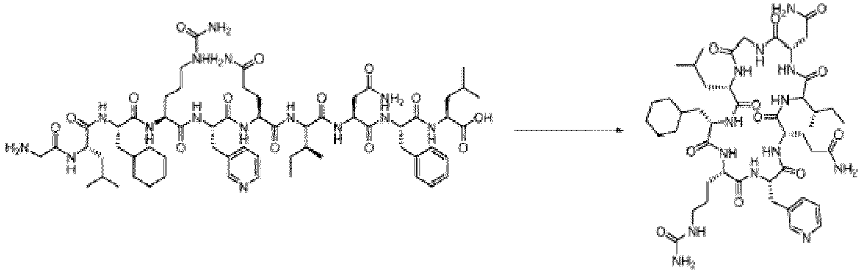

0.5mg of a polypeptde was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively for ring-closure reaction; and the mixture underwent reaction overnight at 37°C to obtain a product.

LCMS:984.98(M+H)⁺.

### Example 21: Verification Test for Ring Closure

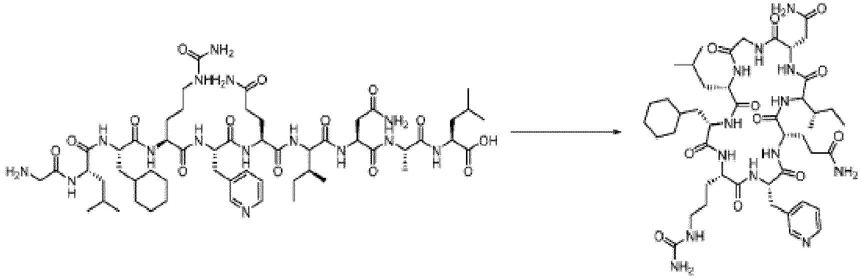

0.5mg of a polypeptde was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (134 mg/mL) were added respectively for ring-closure reaction; and the mixture underwent reaction overnight at 37°C to obtain a product.

LCMS:984.71(M+H)⁺.

### Example 22: Verification of Ring Closure Method for Cyclic Molecules Containing Aromatic Ring

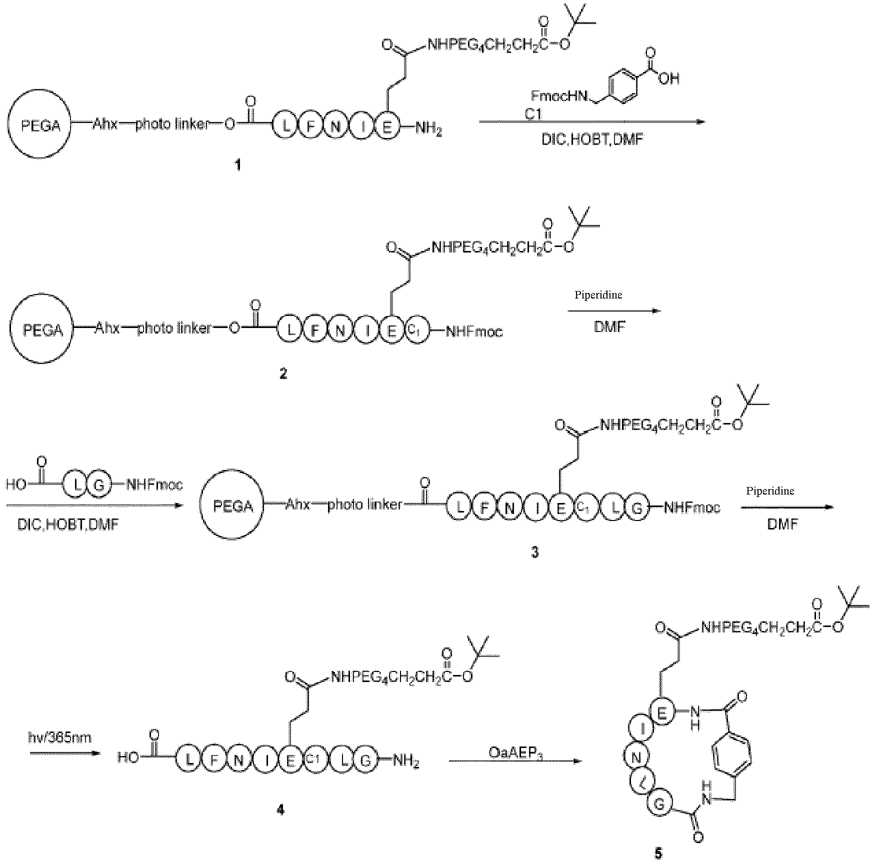

In step 1, DIC (16 mg, 5 eq.), HOBt (17 mg, 5 eq.), and Fmoc-benzylamine benzoic acid (40 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 100mg of resin for later use.

In step 2, 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100 mg of resin for later use. DIC (16 mg, 5 eq.), HOBt (17 mg, 5 eq.) and Fmoc-Gly-Leu-OH (52 mg, 5 eq.) were treated by referring to the condensation operation in step 1 to obtain 95 mg of resin.

In step 3, with 5 mL of 20% piperidine in N,N-dimethylformamide solution, 85 mg of resin was obtained by referring to the Fmoc removal operation in step 2. 85 mg of resin was added to the mixed solution of 0.5 mL of acetonitrile and 0.5 mL of water, and illuminated for 5 h under UV light at 365 nm; and the resin was filtered, dialyzed with a solvent, and then lyophilized to obtain 5 mg of a compound 4.

In step 4, 0.25mg of the compound 5 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively; and the mixture underwent reaction overnight at 37°C to obtain a cyclic peptide compound 5. LCMS:962.53(M+H)⁺.

### Example 23: Verification of Ring Closure Method for Cyclic Molecules Containing Aromatic Ring

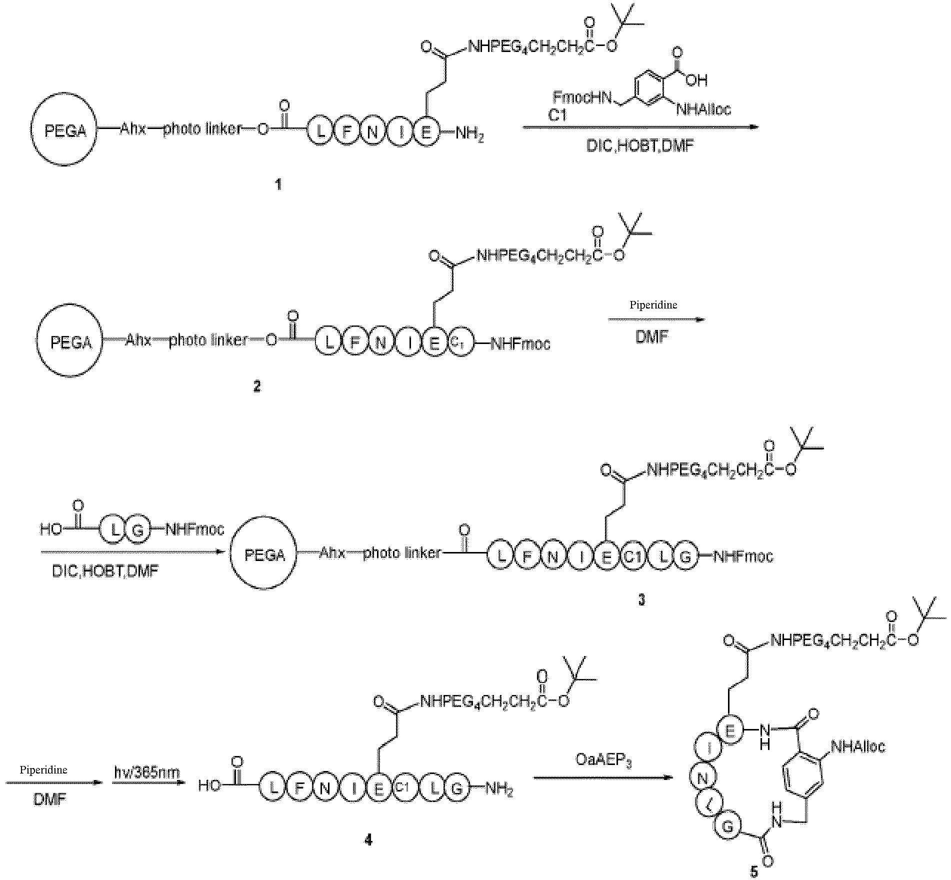

In step 1, DIC (16 mg, 5 eq.), HOBt (17 mg, 5 eq.), and 4-(((((9H-fluoren-9-yl)methoxy)carbonyl)amino)methyl)-2-(((allyloxy)carbonyl)amino)benzoi c acid (45 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining to obtain 100 mg of resin for later use.

In step 2,5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 100mg of resin for later use. DIC (16 mg, 5 eq.), HOBt (17 mg, 5 eq.), and Fmoc-Gly-Leu-OH (52 mg, 5 eq.) were treated by referring to the condensation operation in step 1 to obtain 95 mg of resin.

In step 3, 5 mL of 20% piperidine in N,N-dimethylformamide solution was treated by referring the Fmoc removal operation in step 2 to obtain 85 mg of resin, which was added to the mixed solution of 0.5 ml of acetonitrile and 0.5 ml of water and then illuminated for 5 h under ultraviolet light at 365 nm; the resin was filtered; and the solvent was lyophilized to obtain 5 mg of a compound 4.

In step 4, 0.25mg of the compound 5 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively; and the mixture underwent reaction overnight at 37°C to obtain a cyclic compound 5.

LCMS:1061.56(M+H)⁺.

### Example 24: Compound Library with Increased Membrane Permeability and Construction therefor

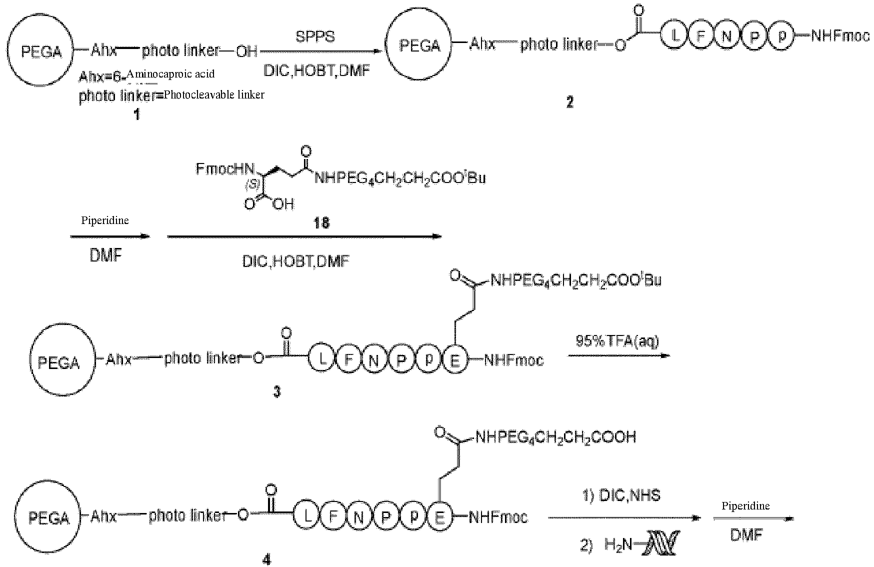

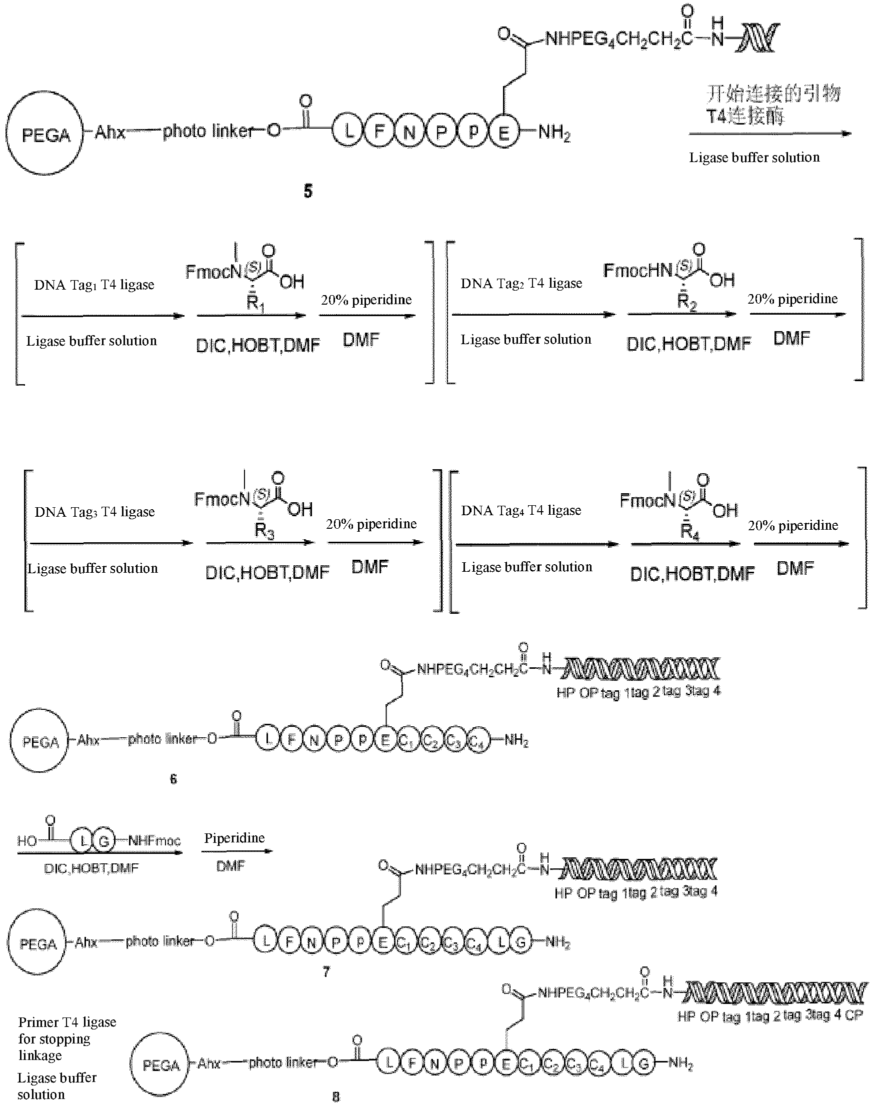

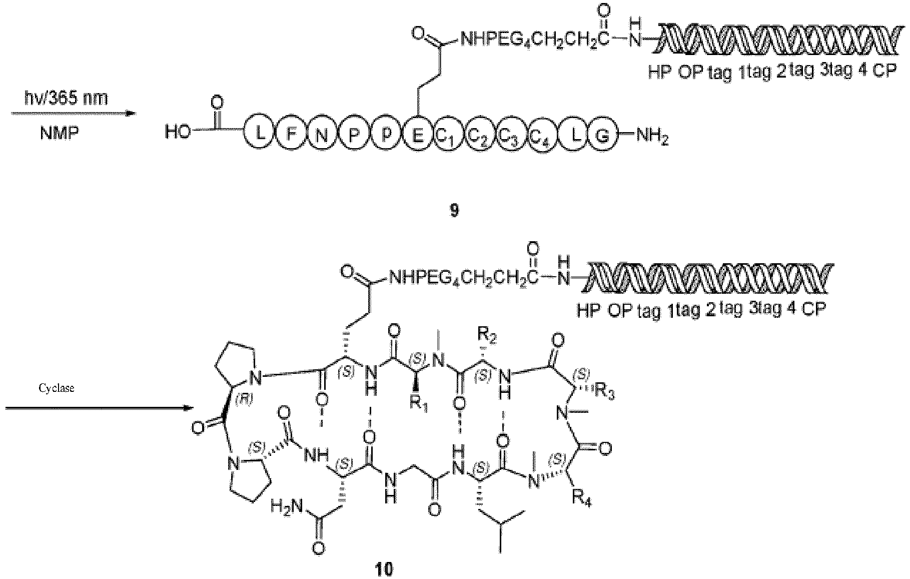

1. 100 mg of PEGA resin 1 was treated by referring to the above synthesis method according to the standard solid-phase synthesis operation to obtain 95 mg of resin. The molecular structure of the product 2 has a molecular fragment consisting of two prolines.
2. 90 mg of resin was obtained by referring to the methods for Fmoc deprotection and amino acid condensation according to the solid-phase synthesis standard.
3. 90 mg of resin was added to 5 ml of 95% trifluoroacetic acid in water to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining; and then the resulting resin was added to 5 ml of 20% DIEA in N,N-dimethylformamide solution and stirred for 2 h at room temperature. The resin was washed with N,N-dimethylformamide (3 × 3 mL), dichloromethane (3 × 3 mL), and N,N-dimethylformamide (3 × 5 mL), respectively, and then dried by draining to obtain 85mg of resin for later use.
4. DIC (16 mg, 5 eq.) and NHS (15 mg, 5 eq.) were dissolved in 5 ml of N,N-dimethylformamide, and then added to 85mg of resin to react for 2 h at room temperature. The resin was washed with N,N-dimethylformamide (3 × 3 mL), dichloromethane (3 × 3 mL), and N,N-dimethylformamide (3 × 5 mL), respectively, and then dried by draining for later use; the resin was swollen in 2 mL of HEPES (100 mM) buffer solution at pH 8.0; and HDNA (100 nmol, l mM) was added to react overnight at room temperature to obtain 80 mg of resin. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 75mg of resin for later use.
5. 2095 µl of water, 250 µl of 10 × T4 DNA ligase buffer solution, 25 µl of T4 DNA ligase (10000 U), and 58 µl of OP (AAATCGATGTG) (100 nm, 1.74 nm/µl) were added to 75mg of resin to react overnight at room temperature; and the resin was washed with water (3 × 5 mL) and T4 DNA ligase buffer solution (3 × 3 mL), respectively, and then dried by draining to obtain 75 mg of resin for later use. 2095 µl of water, 250 µl of 10 × T4 DNA ligase buffer solution, and 25 µl of T4 DNA ligase (10000 U) were added to 75 mg of resin; the mixture was divided into 96 portions; the DNA tag (1.35 nm, 1 nm/µl) was added to each well to react overnight at room temperature; and the resin was washed with T4 DNA ligase buffer solution (3 × 3 mL), water (3 × 3 mL), and N,N-dimethylformamide (3 × 3 mL), respectively, and then dried by draining for later use. DIC (16 mg, 5 eq.) and HOBt (17 mg, 5 eq.) were dissolved in 5 mL of N,N-dimethylformamide and then divided into 96 portions; each portion together with the Fmoc-N-methylamino acid (5 eq.) were added to the resin in each well; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining for later use. The resins were put together; 5 mL of 20% piperidine in N, N-dimethylformamide solution was added; the mixture was stirred for 1 h at room temperature; and the resin was washed with N,N-dimethylformamide (3 × 3 mL), dichloromethane (3 × 3 mL), and N,N-dimethylformamide (3 × 5 mL), respectively, and then dried by draining to obtain 70 mg of resin for later use. All condensation reactions and DNA linkage reactions were repeated 4 times in total, in which the Fmoc amino acid was linked in the second round, the Fmoc-N-methyl amino acid was linked in the first, third and fourth rounds, and after the amino acid and DNA tag in the fourth round were linked, the resins were combined to obtain 65 mg of resin.
6. DIC (16 mg, 5 eq.), HOBt (17 mg, 5 eq.), and Fmoc-Gly-Leu-OH (51 mg, 5 eq.) were stirred in 5 ml of N,N-dimethylformamide for 2 min; then, 65 mg of the resin was added; the mixture was stirred for 2 hours at room temperature; the resin was washed with N,N-dimethylformamide (3 × 3 mL), dichloromethane (3 × 3 mL), and N,N-dimethylformamide ( 3 × 5 mL), respectively, and then dried by draining for later use; the resin was washed with dichloromethane (3 × 3 mL) and N,N-dimethylformamide ( 3 × 3 mL), respectively, and then dried by draining; and 5 mL of 20% piperidine in N,N-dimethylformamide solution was added to the resin and stirred for 1 h at room temperature. The resin was washed with N,N-dimethylformamide (3 × 3 mL) and water (3 × 5 mL), respectively, and then dried by draining to obtain 60 mg of resin for later use.
7. 2095 µl of water, 250 µl of 10 × T4 DNA ligase buffer solution, 25 µl of T4 DNA ligase (10000 U), and 57 µl of CP (100 nm, 1.74 nm/µl) were added to 60mg of resin to react overnight at room temperature; and the resin was washed with T4 DNA ligase buffer solution (3 × 3 mL) and water (3 × 5 mL), respectively, and then dried by draining for later use.
8. 60 mg of resin was added to 1 ml of N-methylpyrrolidone, and illuminated for 5 h under UV light at 365 nm; the resin was filtered, dialyzed with a solvent, and then lyophilized to obtain 5 mg of a compound 9.
9. 5 mg of the compound 16 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 60 µl of OaAEP3 (1.34 mg/mL) were added respectively; and the mixture underwent reaction overnight at 37 °C for ring closure to prepare 3 mg of a product 10.

### Example 25: Compound Library with Increased Membrane Permeability and Construction therefor

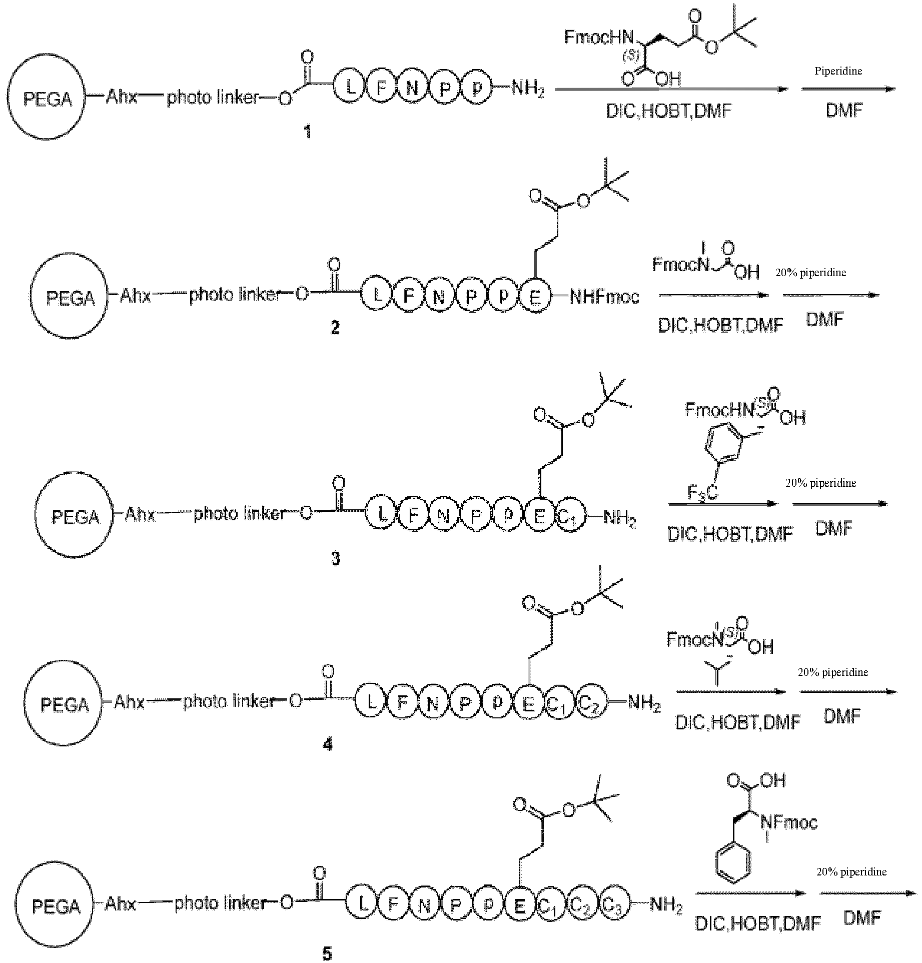

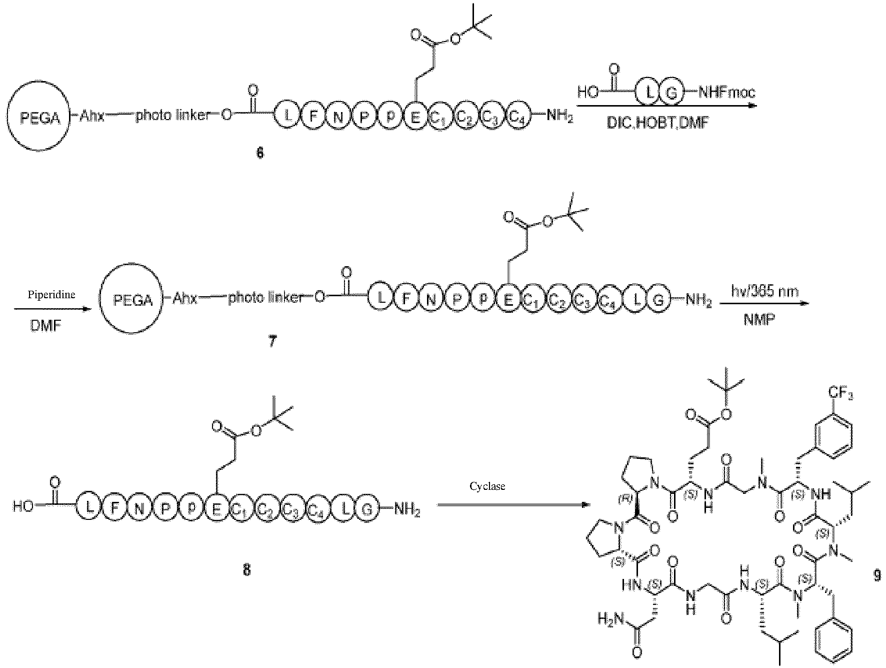

Synthesis method:
1. DIC (16 mg, 5 eq.), HOBt (17 mg, 5 eq.), and Fmoc-Glu(O^{t}Bu)-OH (100 mg, 5 eq.) were stirred in 3 ml of N,N-dimethylformamide for 10 min; then, 100 mg of the prepared resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 95mg of resin for later use.
2. The amino acids were sequentially linked according to the standard solid-phase synthesis operation in step 1 to obtain 80 mg of resin 7.
3. 80 mg of resin was added to 2 mL of NMP solution, and illuminated for 5 h under UV light at 365 nm; and the resin was filtered, dialyzed with a solvent, and then lyophilized to obtain 10mg of a compound 8.
4. 0.5 mg of the compound 8 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively; and the mixture underwent reaction overnight at 37°C to obtain a cyclic peptide compound 9.

LCMS:1238.70(M+H)⁺.

According to the method of the above example, a compound 10, a compound 11, and a compound 12 were synthesized, respectively. The comparison table for compound structure and LC-MS verification is as follows:

| Compound name | Compound structure | LC-MS verification |
|---|---|---|
| Compound 9 | 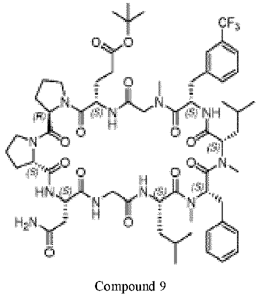 | 1238.70 (M+H)⁺ |
| Compound 10 | 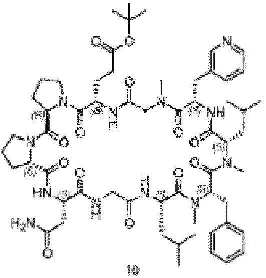 | 1171.75 (M+H)⁺ |
| Compound 11 | 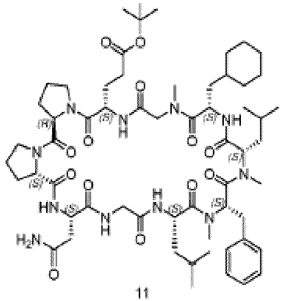 | 1177.04 (M+H)⁺ |
| Compound 12 | 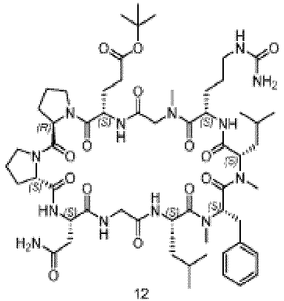 | 1180.44 (M+H)⁺ |

### Example 26: Evaluation of Membrane Permeability

In order to determine the cyclic peptide compounds 9-12 obtained in the above examples, an MDCK model was established to determine their membrane permeability. The MDCK cell line was sourced from Madin Darby canine epithelial cell lines, and the monolayer MDCK cell was the best cytological model to study the in vivo processes such as absorption and reabsorption of drugs in renal tubules. The MDCK cells in a disperse culture flask were digested, and seeded to Millicell CPI at 5×10⁴/cm²; 400 µL of MEM culture solution containing 10% FBS was added to a side A (a luminal side, Apical), and 800 µL of the MEM culture solution was added to a side B (a basolateral side); and the mixture was incubated in an incubator containing 5% CO₂ at 37°C. The solution was changed every day. It should be noted that a pipette should not to touch a cell membrane, thereby avoiding damaging the cell layer; and the transmembrane resistance (i.e., trans-epithelial electrical resistance, TEER) was regularly measured by using Millicell2ERS to test the integrity of the cell monolayer.

The A→B transport from the apical end (A) to the basolateral end (B) of the monolayer membrane of the MDCK cells was observed. For the determination of A→B, 450 µL of a drug or a positive control HEPES solution was added to the apical end of the membrane as a feeding cell, and 1300 µL of a blank HEPES solution was added to the basolateral end as a receiving cell. A Transwell plate was placed on a constant-temperature shaker at 37 °C and shaken; after 90 min, a sample solution was extracted respectively from the feeding cell and the receiving cell; acetonitrile was added to precipitate proteins, and then vortexing, centrifuging and transferring were carried out; and the permeance wasmeasured by LC/MS. Based on this permeance, a membrane permeance coefficient (Papp, i.e., apparent permeability, which was a specific evaluation parameter) was calculated. The Papp greater than 1 × 10⁻⁶ cm/s indicated the absorption of more than 60% of a drug, and the Papp greater than 10 × 10⁻⁶ cm/s indicated the absorption of more than 80% of drug molecules.

| Compound | clogP | MDCK Papp(A-B) [10E-6cm/s] |
|---|---|---|
| 9 | 8.6 | 11.3 |
| 10 | 6.2 | 4.8 |
| 11 | 9.0 | 16.2 |
| 12 | 4.8 | 0.9 |

From the above data, it can be seen that the Papp values of the synthesized compounds are greater than 10⁻⁶, and the Papp values of the compounds 9 and 11 are greater than 10 × 10⁻⁶, showing superior cell permeability.

### Example 27: Ring-closure Verification Test for Building Blocks Linked with Ether Bonds in Cyclic Molecules

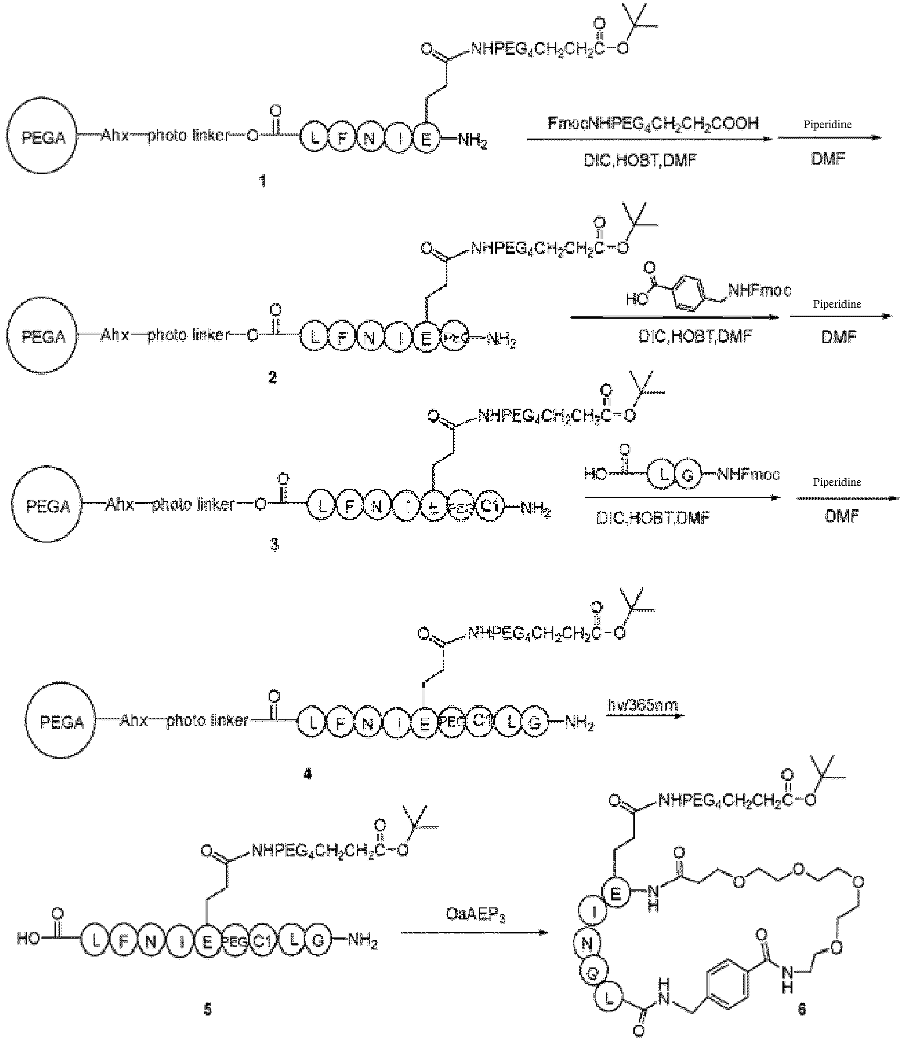

1. DIC (16 mg, 5 eq.), HOBt (17 mg, 5 eq.), and FmocNHPEG₄CH₂CH₂COOH (104 mg, 5 eq.) were stirred in 3 ml of N,N-dimethylformamide for 10 min; then, 100 mg of the swollen resin was added; the mixture was stirred for 1 hour at room temperature; and the resin was washed with N,N-dimethylformamide (3 × 3 mL), dichloromethane (3 × 3 mL), and N,N-dimethylformamide (3 × 5 mL), respectively, and then dried by draining for later use. 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 95mg of resin for later use.
2. DIC (16 mg, 5 eq.), HOBt (17 mg, 5 eq.), and Fmoc-p-benzylamine benzoic acid (40 mg, 5 eq.) were stirred in 3 ml of N,N-dimethylformamide for 10 min; then, 95 mg of swollen resin was added; the mixture was stirred for 1 h at room temperature; the resin was washed with N,N-dimethylformamide (3 × 3 mL), dichloromethane (3 × 3 mL), and N,N-dimethylformamide ( 3 × 5 mL), respectively, and then dried by draining for later use; and 5 mL of 20% piperidine in N,N-dimethylformamide solution was added to the resin and stirred for 1 h at room temperature; and the resin was washed with N,N-dimethylformamide (3 × 5 mL), dichloromethane (3 × 3 mL), and N,N-dimethylformamide (3 × 5 mL), respectively, and then dried by draining to obtain 90 mg of resin for later use.
3. DIC (16 mg, 5 eq.), HOBt (17 mg, 5 eq.), and Fmoc-Gly-Leu-OH (52 mg, 5 eq.) were treated by referring to the condensation operation in step 1. Then, 5 mL of 20% piperidine in N,N-dimethylformamide solution was added to the resin and stirred for 1 hour at room temperature; and the resin was washed with N, N-dimethylformamide (3 × 5 mL), dichloromethane (3 × 3 mL), and N, N-dimethylformamide (3 × 5 mL) respectively, and then dried by draining to obtain 80mg of resin for later use.
4. 80 mg of resin was added to the mixed solution of 0.5 mL of acetonitrile and 0.5 mL of water, and illuminated for 5 h under UV light at 365 nm; and the resin was filtered, dialyzed with a solvent, and then lyophilized to obtain 5 mg of a compound 5.
5. 0.25 mg of the compound 5 was dissolved in 800 µl of water; 100 µl of sodium acetate buffer solution (PH=5.0, 0.5 M), 2 µl of sodium chloride solution (0.25 M), 20 µl of disodium ethylenediaminetetraacetate solution (0.05 M), 10 µl of TCEP solution (0.05 M), and 30 µl of OaAEP3 (1.34 mg/mL) were added respectively; and the mixture underwent reaction overnight at 37°C to obtain a cyclic peptide compound 6.

LCMS:1210.92(M+H)⁺.

### Example 28: Cleavage Verification for Presence and Acid Cleavability of L0

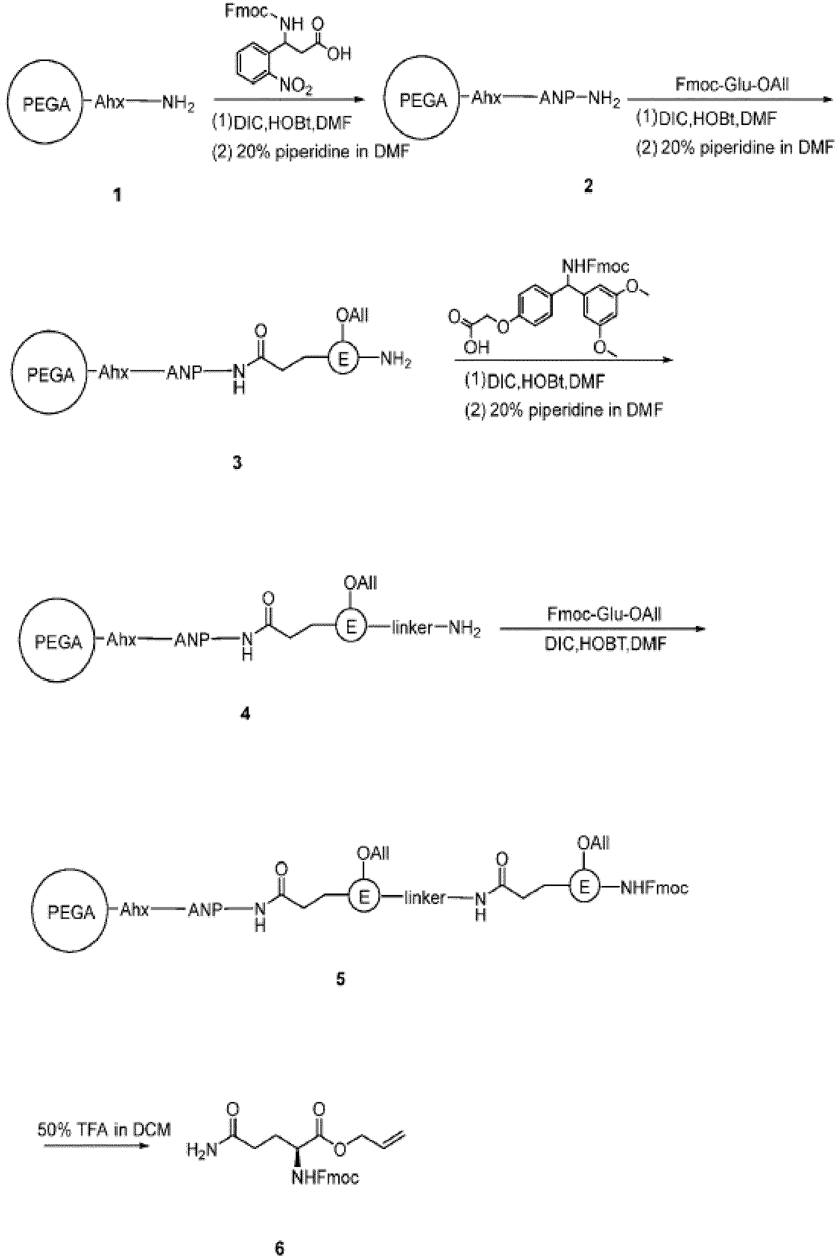

### Synthesis method:

1. DIC (16 mg, 5 eq.), HOBt (17 mg, 5 eq.), and 3-((((9H-fluoren-9-yl)methoxy)carbonyl)amino)-3-(2-nitrophenyl)propionic acid (54 mg, 5 eq.) were stirred in 3 ml of DMF for 10 min; then, 100 mg of the swollen resin was added; the mixture was stirred for 1 hour at room temperature; and the resin was washed with DMF (3 × 3 mL), DCM (3 × 3 mL), and DMF (3 × 5 mL), respectively, and then dried by draining for later use. 5 mL of 20% piperidine in DMF solution was added to the resin and stirred for 1 h at room temperature; and the resin was washed with DMF (3 × 5 mL), DCM (3 × 3 mL), and DMF (3 × 5 mL), respectively, and then dried by draining to obtain 95 mg of resin for later use.
2. DIC (16 mg, 5 eq.), HOBt (17 mg, 5 eq.), and Fmoc-Glu-OAll (52 mg, 5 eq.) were treated by referring to the condensation and Fmoc removal operations in step 1 to obtain 92 mg of resin.
3. DIC (16 mg, 5 eq.), HOBt (17 mg, 5 eq.), and 2-(4-((((((9H-fluoren-9-yl)methoxy)carbonyl)amino)(3,5-dimethoxyphenyl)methyl)pheno xy)acetic acid (68 mg, 5 eq.) were treated by referring to the condensation and Fmoc removal operations in step 1 to obtain 90 mg of resin.
4. DIC (16 mg, 5 eq.), HOBt (17 mg, 5 eq.), and Fmoc-Glu-OAll (52 mg, 5 eq.) were treated by referring to the condensation operation in step 1 to obtain 90mg of resin.
5. 2mL of 50% trifluoroacetic acid in dichloromethane solution was added to the resin, and stirred for 1h at room temperature; the resin was washed with DCM (3 × 3 mL); and the filtrate was spun dry to obtain 4 mg of a compound 6.

LCMS:409.12(M+H)⁺.

Segmented cleavage verification was as follows:

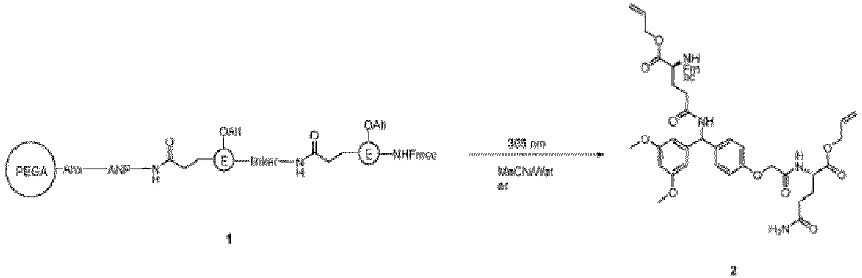

Synthesis method:
1. 100 mg of resin was added to the mixed solution of 0.5 mL of acetonitrile and 0.5 mL of water, and illuminated for 2 h under UV light at 365 nm; and the resin was filtered, dialyzed with a solvent, and then lyophilized to obtain 4 mg of a compound 2.

LCMS:877.96(M+H)⁺.

### Example 29: Verification of DNA Primer Stability in Multi-round Extension Steps

According to the extension step described in the present application, the building blocks are assembled sequentially to form an extended chain, and the DNA tags corresponding to the building blocks are assembled sequentially to form an extended chain. According to the aforementioned synthesis method for resins and construction method for compound libraries, multiple rounds of synthesis were carried out; then, the library compounds synthesized in the fourth, fifth and seventh rounds were subjected to PCR; and PCR products were then detected by agarose gel electrophoresis.

The DNA-containing resin from the fourth round of synthesis was subjected to PCR using primers (TGACTCCCAAATCGATGTG, GCAGGTGAAGCTTGTCTGACAATAGGCTAAA), 2 × taq master mix, and distilled water. The resulting PCR product was detected by agarose gel electrophoresis (PCR product band size: 85 bp).

The DNA-containing resin from the fifth round of synthesis was subjected to PCR using primers (TGACTCCCAAATCGATGTG, GCAGGTGAAGCTTGTCTGACAATAGGCTATC), 2 × taq master mix, and distilled water. The resulting PCR product was detected by agarose gel electrophoresis (PCR product band size: 94 bp).

The DNA-containing resin from the seventh round of synthesis was subjected to PCR using primers (TGACTCCCAAATCGATGTG, GCAGGTGAAGCTTGTCTGACAATAGGCTATC), 2 × taq master mix, and distilled water. The resulting PCR product was detected by agarose gel electrophoresis (PCR product band size: 94 bp).

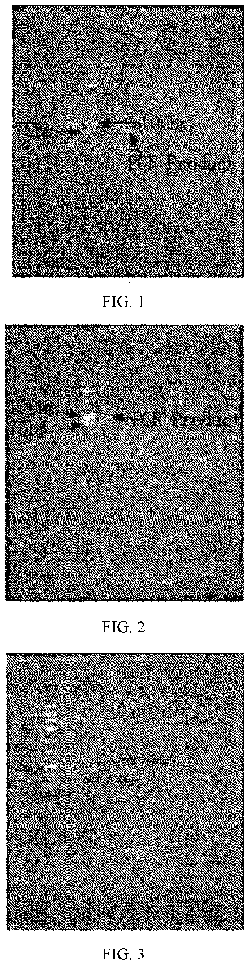

The detection results of agarose gel electrophoresis are shown in FIG. 1 to FIG. 3. FIG. 1 to FIG. 3 show high purity of PCR products from the fourth, fifth, and seventh rounds of synthesis. This indicates that the reaction conditions, in particular the ring-closure reaction conditions, in the synthesis method of the present invention are mild, which is beneficial to maintaining the stability of DNA molecules. Therefore, the compound libraries constructed according to the present application demonstrate better stability and accuracy of the screening results.

### Example 30

Referring to the method described in Example 5, the fourth round of synthesis was carried out to obtain a corresponding monocyclic compound library (with an order of magnitude approaching 100 million).

### Example 31: Screening of Targeting KRAS Protein-Targeting compounds by Using Synthesized DEL

Experimental objective: to screen KRAS-targeting compounds of interest in the compound library of Example 30.

Experimental steps:
1. The compound library was incubated with target proteins.
PBST, 0.25 mg/mL KRAS (dissolved in PBS), Yeast tRNA, 10.2 mg/mL DEL compound library, and 0.1 mg/mL BSA were added to a PCR tube following the volumes in the table below, and incubated for 60 min at room temperature.
PBST, PBS, Yeast tRNA, 10.2 mg/mL DEL compound library, and 0.1 mg/mL BSA were added to a PCR tube following the volumes in Table 1, and incubated for 60 min at room temperature to serve as a BSA control group.

**Table 1**

| Name | Volume | Final concentration |
|---|---|---|
| PBST | 69.45uL | |
| KRAS or PBS | 4.4uL | 500nM |
| Yeast tRNA | 10uL | 1mg/mL |
| BSA | 10uL | 10ug/mL |
| DEL | 6.15uL | 2.5nM |

2. Beads in the experimental group and the BSA group were pretreated separately.
160 uL of beads were added into a PCR tube, which was then placed in a magnetic stand, and a supernatant was discarded after the beads were adsorbed to a tube wall; the PCR tube was removed, 160 uL of PBST was added to resuspend the beads, the PCR tube was then placed in the magnetic stand and a supernatant was discarded; the above steps were repeated 3 times; and the beads are resuspended with 160 uL of PBST and stored at 4°C for later use.
3. Capture/elution
20 uL of pretreated beads were added and then incubated for 30 min at room temperature, during which the temperature of a water bath was adjusted to 72°C. The incubation system was placed in the magnetic stand and a supernatant was discarded; the PCR tube was removed, the beads were resuspended with 100 uL of PBST, the PCR tube was then placed in the magnetic stand and a supernatant was discarded; this step was repeated 5 times; the beads were resuspended with 40 uL of PBST and transferred to a new PCR tube; and the PCR tube was held in a water bath at 72°C for 5 min. The PCR tube was placed in the magnetic stand, and the supernatant was transferred to a new PCR tube; 10 uL of pretreated beads were added to the supernatant, which was incubated for 10 min at room temperature; and the supernatant was used for the next round of screening.
4. The second/third round of screening was carried out.
Round 2: corresponding reagents were added to the supernatant obtained in step 3 according to Table 2, and it should be noted that PBS was added in the BSA group and the corresponding target proteins were added for the experimental group; incubation was carried out for 30 min at room temperature; and step 3 was repeated.

**Table 2**

| Name | Volume |
|---|---|
| KRAS or PBS | 4.4uL |
| Yeast tRNA | 10uL |
| BSA | 10uL |

Round 3: step 3 was repeated, the sample was placed in the magnetic stand and the supernatant (≈ 40 uL) was used for the subsequent PCR analysis.
5. PCR (Bio-Rad T100 PCR Instrument)/NGS was used.
A 50 uL PCR system was prepared according to the conditions in Table 3:

**Table 3**

| Name | Volume | |
|---|---|---|
| Sample from step 4 | 23uL | |
| 2 × Taq mixture | 25uL | Tm 59.1°C |
| Primers for DEl compound library | 1uL | 36 cycles |
| Antisense for DEL | 1uL | |

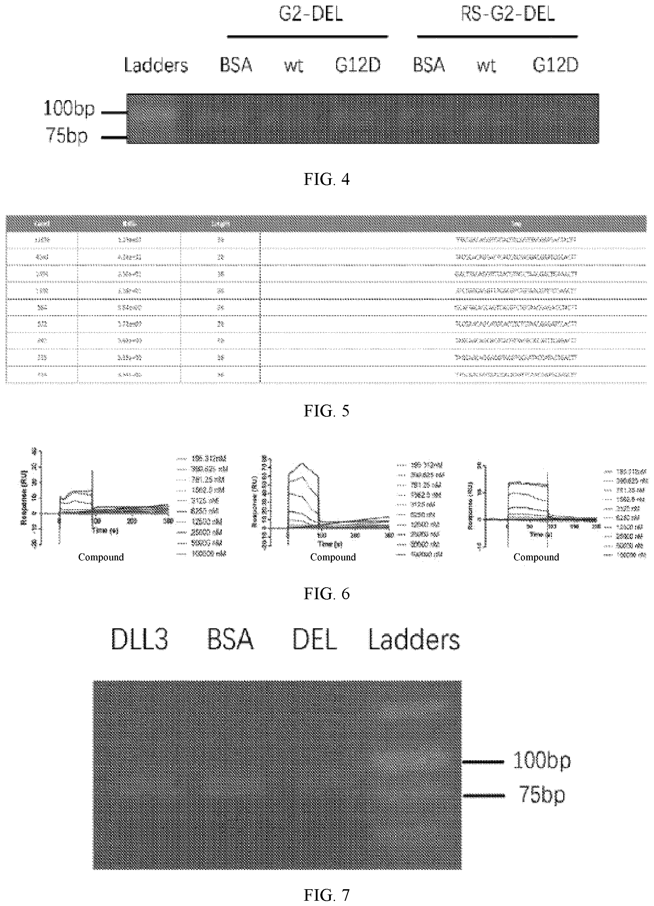

4% agarose gel was made; and 10 uL of a PCR sample was taken for gel analysis, with the results shown in FIG. 4.

### Example 32: NGS Analysis of PCR Sample

NGS analysis was carried out on the PCR sample described in Example 31, with the specific steps as follows.
1. Library construction: a library was constructed by using a kit. The specific process was as follows: the library was constructed directly with >50 ng PCR purified product (a stock solution needed to undergo gel cutting or bead purification). End repair (including 5'-end phosphorylation and 3'-end plus A') was carried out by the End Prep Enzyme Mix, with sequencing linkers at both ends. Fragments were then purified using the DNA Clean Beads and finally amplified with primers P5 and P7. Library quality was tested using the Qseq 100 bioanalyzer (Bioptic, Taiwan, China) and library concentration was detected by Qubit 3.0.
2. Sequencing: after the DNA library was mixed, the sequence information was read by NovaSeq supplied NovaSeq Control Software (NCS)+OLB+GAPipeline-1.6 suppled according to Illumina Novaseq(Illumina, SanDiego, CA, USA).
3. Data analysis- sequencing data quality analysis: preliminary statistical analysis was carried out on the exported raw data. Then, Cutadapt [l] (version 1.9.1) software was used to optimize the raw data by removing primer and linker sequences, bases with mass values below 20 at both ends and sequences with an N base ratio greater than 10%, and then, statistical analysis was carried out on clean data after QC. Pandaseq [2] (version2.7) was used to merge Clean Reads according to an overlap region between two read segments Read1 and Read2 to generate a complete sequence, and the length distribution or other information of the merged sequence was statistically analyzed.

Data splitting: data splitting was carried out according to a barcode sequence, and splitting results were counted.

Tag sequence abundance statistics: a Tag sequence was obtained by anchoring according to upstream and downstream constant region sequences of the Tag sequence. The Tag sequence was divided into sequence units corresponding to different building blocks. Whether each sequence unit belonged to a DNA sequence set corresponding to building blocks was analyzed, and if so, the sequence was extracted and abundance statistics was carried out.

It can be seen from FIG. 5 that there is a significant difference in the abundance value. The sequence with the higher times of repetitions was selected for subsequent verification.

### Example 33: SPR Analysis of Screened Compounds

1. Protein immobilization:
Proteins were immobilized by using a NTA chip. Before immobilization, the chip was regenerated and activated for 120 s by using 0.5M EDTA (pH8.0) and 100 mM NaOH; KRAS proteins were diluted to 5 µg/mL with an analysis buffer; the flow rate was set to 10 µL/min, and the diluted KRAS proteis were injected to a test channel for 360 s to capture the KRAS proteins; and the test channel was rinsed with a PBS buffer until the proteins were captured stably.
2. Sample test conditions:
a PBS buffer (pH7.4) containing 0.05% Tween-20 and 5% DMSO was taken as a running buffer; with the running buffer as a control test sample, series concentrations (0.1953 µM, 0.3906 µM, 0.7812 µM, 1.5625 µM, 3.125 µM, 6.25 µM, 12.5 µM, 25 µM, 50 µM, 100 µM) were set; the flow rate during sample analysis was set to 30 µL/min; the binding time was set to 90 s; and the dissociation time was set to 300 s. At the same time, 8 gradient concentrations of DMSO-containing buffer were set for solvent correction.
3. Parameter fitting:
the experiment ran in multiple cycles, and its response signal takes the analysis time as the abscissa and the response value as the ordinate. The data obtained after solvent correction were subjected to double-reference abatement, and fitted by BIAcore T200 analysis software; and the fitting model used was a 1:1 Langmuir binding model to determine the affinity indicators such as the binding constant and the dissociation constant. The indicators of the dissociation constant are shown in Table 4.

**Table 4: Screened Compounds with Corresponding Dissociation Constants**

| Compound | Dissociation constant Kd value (µM) |
|---|---|
| Compound AQHL-41-04 | 27.43 |
| Compound BQHL-41-05 | 27.46 |
| Compound CQHL-41-03 | 36.31 |

Conclusion: the graph of test results is shown in FIG. 6; from the data in the graph, it can be seen that the compounds screened from the library show certain binding capacity to the target proteins, demonstrating that the target compounds have been caught.

### Example 34: Screening of DLL3 protein-targeting Compounds with synthesized DEL

Experimental objective: to screen K-RAS-targeting compounds of interest in the compound library of Example 30

### Experimental steps:

1. The compound library was incubated with target proteins.
PBST, 40.2 mg/mL DLL3 (dissolved in PBS), Yeast tRNA, 10.2 mg/mL DEL compound library, and 0.1 mg/mL BSA were added to a PCR tube following the volumes in the table below, and incubated for 60 min at room temperature.
PBST, PBS, Yeast tRNA, 10.2 mg/mL DEL compound library, and 0.1 mg/mL BSA were added to a PCR tube following the volumes in Table 1, and incubated for 60 min at room temperature to serve as a BSA control group.

**Table 5**

| Name | Volume | Final concentration |
|---|---|---|
| PBST | 10.85uL | |
| DLL3 or PBS | 63uL | 500nM |
| Yeast tRNA | 10uL | 1mg/mL |
| BSA | 10uL | 10ug/mL |
| DEL | 6.15uL | 2.5nM |

2. Beads in the experimental group and the BSA group were pretreated separately.
160 uL of beads were added into a PCR tube, which was then placed in a magnetic stand, and a supernatant was discarded after the beads were adsorbed to a tube wall; the PCR tube was removed, 160 uL of PBST was added to resuspend the beads, the PCR tube was then placed in the magnetic stand and a supernatant was discarded; the above steps were repeated 3 times; and the beads are resuspended with 160 uL of PBST and stored at 4°C for later use.
3. Capture/elution
20 uL of pretreated beads were added and then incubated for 30 min at room temperature, during which the temperature of a water bath was adjusted to 72°C. The incubation system was placed in the magnetic stand and a supernatant was discarded; the PCR tube was removed, the beads were resuspended with 100 uL of PBST, the PCR tube was then placed in the magnetic stand and a supernatant was discarded; this step was repeated 5 times; the beads were resuspended with 40 uL of PBST and transferred to a new PCR tube; and the PCR tube was held in a water bath at 72°C for 5 min. The PCR tube was placed in the magnetic stand, and the supernatant was transferred to a new PCR tube; 10 uL of pretreated beads were added to the supernatant, which was incubated for 10 min at room temperature; and the supernatant was used for the next round of screening.
4. The second/third round of screening was carried out.
Round 2: corresponding reagents were added to the supernatant obtained in step 3 according to Table 2, and it should be noted that PBS was added in the BSA group and the corresponding target proteins were added for the experimental group; incubation was carried out for 30 min at room temperature; and step 3 was repeated.

**Table 6**

| Name | Volume |
|---|---|
| DLL3 or PBS | 63uL |
| Yeast tRNA | 10uL |
| BSA | 10uL |

Round 3: step 3 was repeated, the sample was placed in the magnetic stand and the supernatant (≈ 40 uL) was used for the subsequent PCR analysis. 5. PCR (Bio-Rad T100 PCR Instrument)/NGS was used.

A 50 uL PCR system was prepared according to the conditions in Table 3:

**Table 7**

| Name | Volume | |
|---|---|---|
| Sample from step 4 | 23uL | |
| 2×Taq mixture | 25uL | Tm 59.1°C |
| Primers for DEl compound library | 1uL | 36 cycles |
| Antisense for DEL | 1uL | |

4% agarose gel was made; and 10 uL of a PCR sample was taken for gel analysis. The results are shown in FIG. 7.

Conclusion: after three rounds of screening and enrichment, the fragments interacting with the DLL3 protein at 500 nM can be screened from the DEL library at 2.5 nM.

### Example 35: NGS Analysis of PCR Sample

NGS analysis was carried out on the PCR sample described in Example 34, with the specific steps as follows.
1. Library construction: a library was constructed by using a kit. The specific process was as follows: the library was constructed directly with >50 ng PCR purified product (a stock solution needed to undergo gel cutting or bead purification). End repair (including 5'-end phosphorylation and 3'-end plus A') was carried out by the End Prep Enzyme Mix, with sequencing linkers at both ends. Fragments were then purified using the DNA Clean Beads and finally amplified with primers P5 and P7. Library quality was tested using the Qseq 100 bioanalyzer (Bioptic, Taiwan, China) and library concentration was detected by Qubit 3.0.
2. Sequencing: after the DNA library was mixed, the sequence information was read by NovaSeq supplied NovaSeq Control Software (NCS)+OLB+GAPipeline-1.6 suppled according to Illumina Novaseq(Illumina, SanDiego, CA, USA).
3. Data analysis- sequencing data quality analysis: preliminary statistical analysis was carried out on the exported raw data. Then, Cutadapt [l] (version 1.9.1) software was used to optimize the raw data by removing primer and linker sequences, bases with mass values below 20 at both ends and sequences with an N base ratio greater than 10%, and then, statistical analysis was carried out on clean data after QC. Pandaseq [2] (version2.7) was used to merge Clean Reads according to an overlap region between two read segments Read1 and Read2 to generate a complete sequence, and the length distribution or other information of the merged sequence was statistically analyzed.

Data splitting: data splitting was carried out according to a barcode sequence, and splitting results were counted.

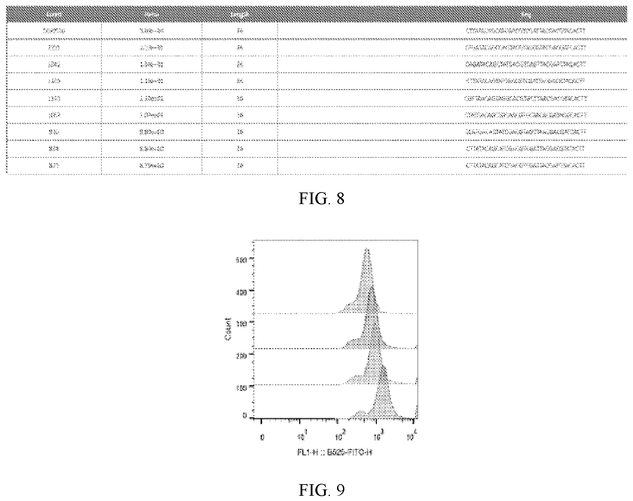

Tag sequence abundance statistics: a Tag sequence was obtained by anchoring according to upstream and downstream constant region sequences of the Tag sequence. The Tag sequence was divided into sequence units corresponding to different building blocks. Whether each sequence unit belonged to a DNA sequence set corresponding to building blocks was analyzed, and if so, the sequence was extracted and abundance statistics was carried out. It can be seen from FIG. 8 that there is a significant difference in the abundance value. The sequence with the higher times of repetitions was selected for subsequent verification.

### Example 36: FACS Analysis of Screened Compounds

The compound obtained in Example 35 was linked to FITC and incubated with DLL3-overexpressed CT-26 cells for flow cytometry analysis, with the specific steps as follows:
1 the CT-26 cells were digested and resuspended in a 25 cm³ flask, then counted, and seeded into a 96-well plate at 1 × 10⁵ cells/well;
2 the compound was added to a culture medium at different concentrations, mixed well and then added to the 96-well plate, which was incubated for 6 h in an incubator at 37°C; and
3 the 96-well plate was centrifuged for 5 min at 1,000 rpm and a supernatant was discarded; 0.25% pancreatin was added at 100 uL/well for digestion for 2 min, and the 96-well plate was then centrifuged and a supernatant was discarded; and the cells were resuspended with PBS + 2% FBS at 200 uL/well.
4 The cells were analyzed by flow cytometer, and green fluorescence channels were selected. The specific results are shown in FIG. 9.

Conclusion: the data (including the data of DMSO, 2.5 uM compound, 5 uM compound, and 10 uM compound from top to bottom) in FIG. 9 show that the compounds screened from the library are concentration-dependent in terms of the binding to the DLL-overexpressed CT-26 cells.

In summary, described above are only preferred embodiments of the present invention, and are not intended to limit the scope of protection of the present invention. Any modification, equivalent replacement, improvement or the like made within the spirit and principle of the present invention shall be regarded as falling within the scope of protection of the present invention.

## Claims

1. A construction method for a cyclic compound library, comprising the steps of:
1) linking a solid-phase carrier G to a molecule M containing a photocleavable group, directly or indirectly, to obtain G—M;
2) performing any of a method 1, a method 2, and a method 3:
the method 1: performing steps a1-g1:
a1. reacting and linking the G—M with an A-end ring-closure molecule A to obtain G-M-A;
b1. reacting and linking the G-M-A with a linker molecule L1 having at least three functional groups to obtain G-M-A-L1;
c1. reacting the G-M-A-L1 sequentially with a starting nucleotide molecule HP and an open primer OP to obtain G-M-A-LI-HP-OP, wherein the starting nucleotide molecule HP is linked to the linker molecule L1;
d1. reacting the resulting product from the previous step with a building block Ci and a DNA tag tag₁ corresponding to the building block C₁, respectively, to link the building block Ci to the L1 and to link the DNA tag tag₁ to the OP, thereby obtaining G-M-A-L1(-HP-OP-tag₁)C₁;
e1. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining G-M-A-L1(-HP-OP-tag₁-······-tagₙ)-C₁-······-Cₙ, wherein 2 ≤ n ≤ 7, and n is a positive integer;
f1. reacting the resulting product from the previous step with a B-end ring-closure molecule B and a closed primer CP, to link the B-end ring-closure molecule B to the Cₙ and to link the closed primer CP to the tagₙ, thereby obtaining G-M-A-L1(-DNA)-C₁-······ -Cₙ-B, i.e., a compound library S1", respectively, wherein HP-OP-tag₁-······-tagₙ-CP forms a complete DNA-encoded sequence; and
g1. decomposing the resulting product from the previous step under a light source to cleave the M from the A, thereby obtaining A-L1(-DNA)-C₁-······-Cₙ-B, i.e., a compound library S1';
the method 2: performing steps a2-g2, wherein the orders of steps e2 and f2 are interchangeable:
a2. reacting and linking the G—M with a linker molecule L1 having at least three functional groups to obtain G-M-L1;
b2. reacting the G-M-L1 sequentially with a starting nucleotide molecule HP and an open primer OP to obtain OP-HP-G-M-L1, wherein the starting nucleotide molecule HP is linked to the solid-phase carrier G;
c2. reacting the OP-HP-G-M-L1 with a building block Ci and a DNA tag tag₁ corresponding to the building block C₁, to link the building block Ci to the L1 and to link the DNA tag tag₁ to the OP, thereby obtaining tag₁-OP-HP-G-M-L1-C₁;
d2. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining tagₙ-······ -tag₁-OP-HP-G-M-L1-C₁······-Cₙ, wherein 0 ≤ n ≤ 7, and n is a positive integer;
e2. reacting the resulting product from the previous step with an A-end ring-closure molecule A to link the A-end ring-closure molecule A to the Cₙ;
f2. reacting the resulting product from the previous step sequentially with building blocks Cₙ₊₁, ······, Cₙ₊ₘ and DNA tag tagₙ₊₁, ······, tagₙ₊ₘ corresponding thereto according to the extension step, to link the building block Cₙ₊₁ to the linker molecule L1 and to link the DNA tag tagₙ₊₁ to the tagₙ, thereby obtaining tagₙ₊ₘ-······-tag₁-OP-HP-G-M-L1(-C₁······ Cₙ-A)-Cₙ₊₁······Cₙ₊ₘ by steps e2 and f2, wherein 0 ≤ n ≤ 7, 0 ≤ m ≤ 7, n and m are both integers, and 2 ≤ n+m ≤ 7; and
g2. reacting the resulting product from the previous step with a B-end ring-closure molecule B and a closed primer CP, to link the B-end ring-closure molecule B to the Cₙ₊ₘ and to link the closed primer CP to the tagₙ₊ₘ, thereby obtaining DNA-G-M-L1(-C₁······ -Cₙ-A)-Cₙ₊₁······Cₙ₊ₘ-B, i.e., a compound library S2', wherein HP-OP-tag₁-······ -tagₙ₊ₘ-CP forms a complete DNA-encoded sequence;
the method 3: performing steps a3-g3, wherein the orders of steps e3 and f3 are interchangeable:
a3. reacting and linking the G—M with a linker molecule L1 having at least four functional groups to obtain G-M-L1;
b3. reacting the G-M-L1 sequentially with a starting nucleotide molecule HP and an open primer OP to obtain G-M-L1-HP-OP, wherein the starting nucleotide molecule HP is linked to the linker molecule L1;
c3. reacting the G-M-LI-HP-OP with a building block C1 and a DNA tag tag₁ corresponding to the building block C₁, to link the building block Ci to the linker molecule L1 and to link the DNA tag tag₁ to the OP, thereby obtaining G-M-L1(-HP-OP-tag₁)-C₁;
d3. defining an extension step comprising linkage reactions of a corresponding set of building blocks and DNA tags, and repeating the extension step to sequentially assemble the building blocks to form an extended chain and to sequentially assemble the DNA tags corresponding to the building blocks to form an extended chain, thereby obtaining G-M-L1(-HP-OP-tag₁······-tagₙ)-C₁······-Cₙ, wherein 2 ≤ n ≤ 7, and n is a positive integer;
e3. reacting the resulting product from the previous step with an A-end ring-closure molecule A to link the A-end ring-closure molecule A to the Cₙ;
f3. reacting the resulting product from the previous step sequentially with building blocks Cₙ₊₁, ······, Cₙ₊ₘ and DNA tag tagₙ₊₁, ······, tagₙ₊ₘ corresponding thereto according to the extension step, to link the building block Cₙ₊₁ to the linker molecule L1 and to link the DNA tag tagn+i to the tagₙ, thereby obtaining G-M-L1(-HP-OP-tag₁······-tagₙ₊ₘ)(-C₁······ -Cₙ-A)-Cₙ₊₁······-Cₙ₊ₘ by steps e3 and f3, wherein 0 ≤ n ≤ 7, 0 ≤ m ≤ 7, n and m are both integers, and 2 ≤ n+m ≤ 7; and
g3. reacting the resulting product from the previous step with a B-end ring-closure molecule B and a closed primer CP, to link the B-end ring-closure molecule B to the Cₙ₊ₘ and to link the closed primer CP to the tagₙ₊ₘ, thereby obtaining G-M-L1(-DNA)(-C1······ -Cₙ-A)-Cₙ₊₁······-Cₙ₊ₘ-B, i.e., a compound library S3', wherein HP-OP-tag1-······ -tagₙ₊ₘ-CP forms a complete DNA-encoded sequence; and
3) subjecting the compound library S1', S2', or S3' to a ring-closure reaction under the action of a cyclase to react the A-end ring-closure molecule A with the B-end ring-closure molecule B for ring formation, thereby obtaining 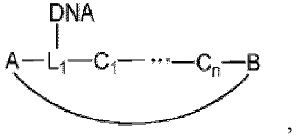 i.e., a cyclic compound library S1, or 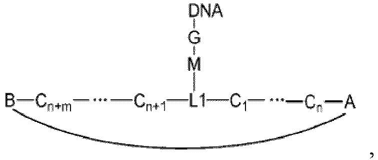 i.e., a cyclic compound library S2, or 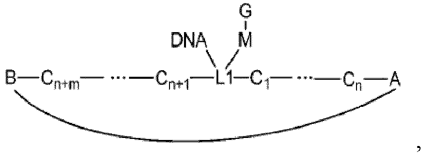 i.e., a cyclic compound library S3. respectively.

2. The method according to claim 1, wherein the solid-phase carrier G is selected from any one or more of the group consisting of a PEG resin, a PEGA resin, a TentaGel resin, and a solid-phase carrier CPG.

3. The method according to claim 2, wherein the solid-phase carrier G has an active functional group R1, R1, which is amino; or the solid-phase carrier G has two active functional groups R1 and R1', with R1 being amino and R1' being carboxyl.

4. The method according to claim 1, wherein the molecule M containing the photocleavable group comprises at least two active functional groups represented by R2 and R3, respectively; R2 is the active functional group responsible for linking the solid-phase carrier G; and R3 is the active functional group responsible for linking the A-end ring-closure molecule A or the linker molecule L1.

5. The method according to claim 4, wherein R2 exists in a form unprotected by a protective group and R3 exists in a form protected by a protective group.

6. The method according to claim 4, wherein in the molecule M containing the photocleavable group, the photocleavable group is: 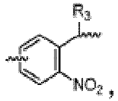 wherein R3 is at a C atom that is located on a side chain ortho to nitro and linked to a benzene ring; R2 is linked to 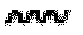 , and R2 is linked to a C atom on the benzene ring by one or more covalent bonds, or R2 is linked to the C atom, which is linked to R3, by one or more covalent bonds; and the benzene ring can comprise 0, 1, or more side chains or substituents that do not interfere with linkage reactions between R2 and R3.

7. The method according to claim 6, wherein the molecule M containing the photocleavable group can be selected from the group consisting of structures below: 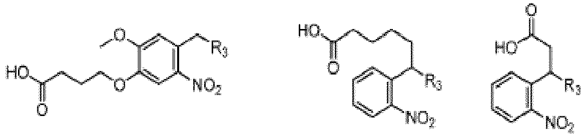 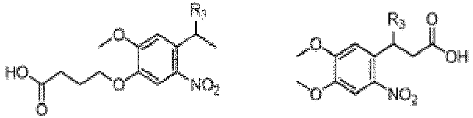 wherein R3 can be selected from the group consisting of -OH, -NH₂, -NHNH₂, -N₃, Cl, and Br; and R2 is represented by carboxyl.

8. The method according to claim 1, wherein when the A-end ring-closure molecule A and the B-end ring-closure molecule B are subjected to the ring-closure reaction under the cyclase, some or all of molecular fragments are removed from the A-end ring-closure molecule A; the A-end ring-closure molecule A has two active functional groups R4 and R5; R4 is the active functional group involved in the ring-closure reaction under the cyclase, and is removed during the ring-closure reaction; R5 is the active functional group responsible for reacting and linking with the linker molecule L1 or the building block, and R5 remains in a cyclic structure of a cyclic compound molecule after the ring-closure reaction; and R4 and R5 exist independently in a form protected or unprotected by a protective group, respectively, and R4 and R5 do not interfere with each other in linkage reaction.

9. The method according to claim 8, wherein a molecular structure of the A-end ring-closure molecule A consists of two moieties: a molecular fragment A1 that is removed during the ring closure reaction under the cyclase and a molecular fragment A0 that remains on the ring during the ring-closure reaction; R4 is located on the molecular fragment A1 that is removed during the ring-closure reaction under the cyclase; and R5 is located on the molecular fragment A0 that remains on the ring during the ring-closure reaction.

10. The method according to claim 9, wherein the molecular fragment A0 structurally comprises a steric hindrance group, which is directly linked to R5; and the steric hindrance block group is an amino acid residue consisting of one or more amino acids.

11. The method according to claim 10, wherein the steric hindrance group is an amino acid residue consisting of 1-10 amino acids.

12. The method according to claim 8, wherein R4 exists in a form unprotected by a protective group, and R5 exists in a form protected by a protective group.

13. The method according to claim 8, wherein R4 is an active functional group complementarily paired with R3 of the molecule M containing the photocleavable group.

14. The method according to claim 9, wherein a structure of the molecular fragment A1 removed from the molecular structure of the A-end ring-closure molecule A is an amino acid residue consisting of one or more amino acids.

15. The method according to claim 9, wherein the moiety A1 removed from the molecular structure of the A-end ring-closure molecule A has an amino acid amino sequence of: FAGDDAE, AYDGE, OCam-Leu, FL,AL, GL,HL, or HV.

16. The method according to claim 8, wherein the A-end ring-closure molecule A is a peptide chain consisting of at least 3 amino acids.

17. The method according to claim 1, wherein in the method 1, the linker molecule L1 has at least three active functional groups R6, R7, and R8; R6, R7 and R8 can exist independently in a form protected or unprotected by a protective group, respectively, and R6, R7 and R8 do not interfere with each other in linkage reaction; and R6 is the active functional group complementarily paired with the active functional group R5 on the A-end ring-closure molecule A, R7 is the active functional group reacted and assembled with the starting nucleotide molecule HP, and R8 is the active functional group reacted and assembled with the building block C1.

18. The method according to claim 1, wherein in the method 2, the linker molecule L1 has at least three active functional groups R6, R7, and R8; R6, R7 and R8 can exist independently in a form protected or unprotected by a protective group, respectively, and R6, R7 and R8 do not interfere with each other in linkage reaction; and R6 is the active functional group complementarily paired with the active functional group R3 of the molecule M containing the photocleavable group, R7 is the active functional group reacted and assembled with the building block C₁, and R8 is the active functional group reacted and assembled with the building block Cₙ₊₁.

19. The method according to claim 1, wherein in the method 3, the linker molecule L1 has at least four active functional groups R6, R6' , R7 and R8; R6, R6', R7 and R8 can exist independently in a form protected or unprotected by a protective group, respectively, and R6, R6', R7 and R8 do not interfere with each other in linkage reaction; R6 is the active functional group complementarily paired with the active functional group R3 of the molecule M containing the photocleavable group, R6' is the active functional group reacted and assembled with the starting nucleotide molecule HP, R7 is the active functional group reacted and assembled with the building block C₁, and R8 is the active functional group reacted and assembled with the building block Cₙ₊₁.

20. The method according to claim 19, wherein the linker molecule L1 comprises a decomposable functional group R_{L}, and when R_{L} is decomposed, the linker molecule L1 is split into two molecular fragments, namely, a molecular fragment comprising R6, R6' and a molecular fragment comprising R7, R8.

21. The method according to claim 20, wherein the decomposable functional group R_{L} is an acid cleavable group, or a photocleavable group having a different cleavage wavelength than the molecule M containing the photocleavable group.

22. The method according to claim 20, wherein the linker molecule L1 can consist of two linker molecules L1', L1" each having three functional groups, and a linker molecule L0 linked between L1' and L1", and the decomposable functional group R_{L} is within a molecular structure of the linker molecule L0; and wherein L1' has three active functional groups R6, R6' and R6", L1" has three active functional groups R7, R8 and R8', L0 has two active functional groups R7 and R7', R7 and R6" are linked by a complementary pairing reaction, and R7' and R8' are linked by a complementary pairing reaction.

23. The method according to claim 22, wherein the linker molecule L0 is selected from the group consisting of structures below:

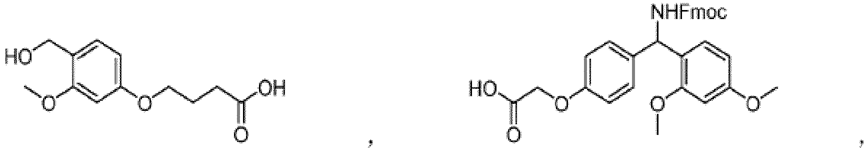

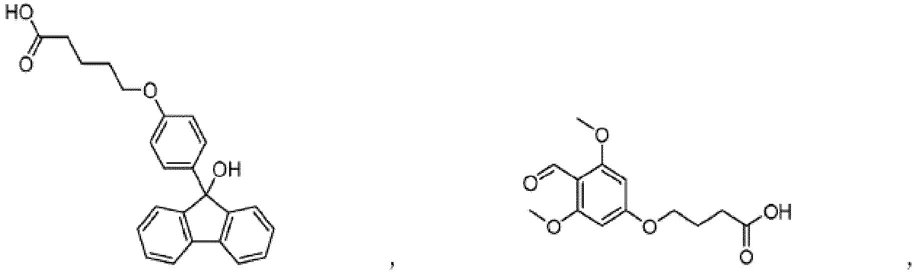

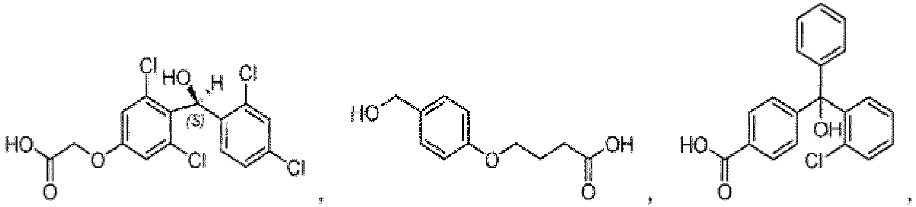

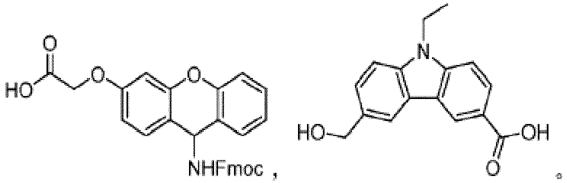

24. The method according to claim 1, wherein the starting nucleotide molecule HP has an active functional group R9, which is complementary paired and reacts with the active functional group of the linker molecule L1, or the active functional group of the solid-phase carrier G.

25. The method according to claim 24, wherein the active functional group R9 of the starting nucleotide molecule HP is amino, and the active functional group, complementarily paired and reacting with R9, of the linker molecule L1 or of the solid-phase carrier G is carboxyl.

26. The method according to claim 1, wherein the building block is a small molecule compound having at least two active functional groups.

27. The method according to claim 26, wherein in the method 1, each of the building blocks is sequentially assembled as follows: a first active functional group of the building block Ci is responsible for assembly with the linker molecule L1, a first active functional group of each of the subsequent building blocks is sequentially assembled with a second active functional group of a previous building block, and a second active functional group of the building block Cₙ is responsible for assembly with the B-end ring-closure molecule B; the first active functional groups and the second active functional groups do not simultaneously exist in a form unprotected by a protective group, and the first active functional group and the second active functional group do not interfere with each other; and a third functional group exists in a form protected by a protective group, and can be used for subsequent compound modification and adjustment.

28. The method according to claim 26, wherein in the method 2 or method 3, the first active functional group of the building block C₁ is responsible for assembly with an active functional group of the linker molecule L1, a first active functional group of each of the building blocks between C₁ and Cₙ and a first active functional group of the building block Cₙ are each sequentially assembled with a second active functional group of a previous building block, and a second active functional group of the building block Cₙ is responsible for assembly with the A-end ring-closure molecule A; a first active functional group of the building block Cₙ₊₁ is responsible for assembly with another active functional group of the linker molecule L1, a first active functional group of each of the building blocks between Cₙ₊₁ and Cₙ₊ₘ and a first active functional group of the building block Cₙ₊ₘ are each sequentially assembled with a second active functional group of a previous building block, and a second active functional group of the building block Cₙ₊ₘ is responsible for assembly with the B-end ring-closure molecule B; and the first active functional groups and the second active functional groups do not simultaneously exist in a form unprotected by a protective group, and the first active functional groups and the second active functional groups do not interfere with each other.

29. The method according to claim 27 or 28, wherein the adjacent building blocks are linked by the following chemical bonds: an amide bond, or an ester bond, an amide bond, an ester bond, an acid bond, an amine bond, or an imide bond.

30. The method according to claim 26, wherein the building block is a compound having double active functional groups comprising both amino and carboxyl.

31. The method according to claim 26, wherein the building block is selected from the group consisting of substituted or unsubstituted dicarboxylic acid, substituted or unsubstituted diamine, substituted or unsubstituted glycol, α,β-unsaturated aldehyde, α,β-unsaturated ketone, α,β-unsaturated acid, a carbon-carbon double bond or carbon-carbon triple bond containing active groups (hydroxyl, amido, aldehyde, carboxyl, sulfonate or halogen), an aromatic ring compound containing two or more active groups, a natural amino acid or an unnatural amino acid, and an N- substituted amino acid.

32. The method according to claim 26, wherein the building block further comprises a skeleton structure, which is linked into a ring or is linked to a ring in a form of a side chain of the ring.

33. The method according to claim 32, wherein at least one of the individual building blocks comprises the skeleton structure, which has an E3 ligase substrate structure and can bind to an E3 ligase.

34. The method according to claim 32, wherein the skeleton structure is selected from the group consisting of:

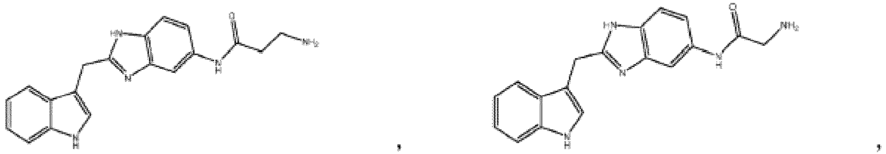

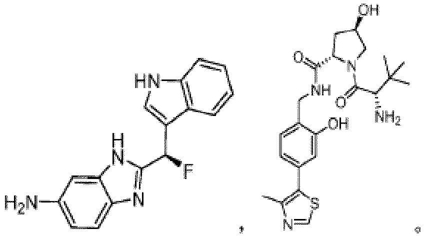

35. The method according to claim 32, wherein each of the building blocks comprises at least one extra-ring side chain in total.

36. The method according to claim 32, wherein each of the building blocks comprises at least two extra-ring side chains in total, and wherein the at least two extra-ring side chains are linked by means of a chemical reaction to form a bicyclic structure.

37. The method according to claim 32, wherein the DNA tags are sequentially linked by means of DNA ligases; and in one extension step, an assembly reaction is completed for one DNA tag and the building block corresponding to the DNA tag, such that the chain of the building blocks and the chain of the DNA tags are extended, respectively.

38. The method according to claim 1, wherein the B-end ring-closure molecule B is a compound having double active functional groups, which are represented by R10 and R11, respectively; R10 is the active functional group to react and assemble with a second active functional group of the last building block, and R11 is the active functional group responsible for the ring-closure reaction; and R10 and R11 can exist independently in a form protected or unprotected by a protective group, respectively, and R10 and R11 do not interfere with each other in linkage reaction.

39. The method according to claim 38, wherein R10 exists in a form protected by a protective group, and R11 exists in a form unprotected by a protective group.

40. The method according to claim 38, wherein one of the two active functional groups R10 and R11 of the B-end ring-closure molecule B is amino, and the other is carboxyl.

41. The method according to claim 40, wherein the B-end ring-closure molecule B is a peptide chain consisting of 2 to 10 amino acids.

42. The method according to claim 40, wherein the B-end ring-closure molecule B is a dipeptide protected by Fmoc.

43. The method according to claim 40, wherein a molecular structure of the B-end ring-closure molecule B has amino acid residues GL, LL, QL, KL, GF, and Gl.

44. The method according to claim 1, wherein in the step 3), the compound library S1', S2' or S3' is subjected to the ring-closure reaction under the action of the cyclase to react the A-end ring-closure molecule A with the B-end ring-closure molecule B to form an amide bond, thereby forming a ring; in a ring formation reaction, a free-end active functional group of the B-end ring-closure molecule B reacts with the A-end ring-closure molecule A to remove part or all of the molecular fragments from the A-end ring-closure molecule A, and a free-end active functional group of the B-end ring-closure molecule B is linked to a remaining part after removal to form a cyclic structure; and the cyclase is selected from ligases VyPAL2, Butelase1, PatG, PagG, ominiligase-1, PCY1, or OaAEP1B&3-5.

45. The method according to claim 44, wherein the formed cyclic structure can be a single ring or double rings or a cyclic structure with side chains.

46. The method according to claim 44, wherein the ring-closure reaction under the cyclase occurs at a temperature of 25-45°C, preferably 30-45°C, and more preferably 35-40°C.

47. The method according to claim 44, wherein the ring-closure reaction under the cyclase occurs in a pH range of 4.5-6.0, preferably 4.8-5.5, and more preferably 4.9-5.3.

48. The method according to claim 44, wherein the ring-closure reaction under the cyclase has a reaction time of 12-48 hours, preferably 18-36 hours, and more preferably 20-24 hours.

49. The method according to claim 44, wherein in the ring-closure reaction under the cyclase, a pH value is adjusted by using a sodium acetate buffer solution.

50. The method according to claim 44, wherein disodium ethylenediamine tetraacetate, sodium chloride, and TCEP are added to a ring-closure reaction system under the cyclase.

51. The method according to claim 1, wherein the compound library S3' is first decomposed under a light source to cleave the M from the L1, thereby obtaining DNA-L1(-C1······ -Cₙ-A)-Cₙ₊₁······-Cₙ₊ₘ-B, i.e., a compound library S4'; the compound library S4' is then subjected to the ring-closure reaction under the action of the cyclase to react the A-end ring-closure molecule A with the B-end ring-closure molecule B to form a ring, thereby obtaining 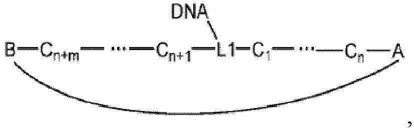 i.e., a cyclic compound library S4; or the compound library S3 is decomposed under a light source to cleave the M from the L1, thereby obtaining 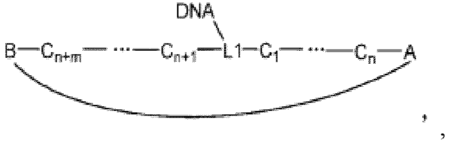 i.e., a compound library S4.

52. A cyclic compound library having a general structural formula of: 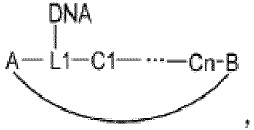
wherein 2 ≤ n ≤ 7 and n is a positive integer,
L1 is a linker molecule having at least three functional groups, and a DNA-encoded sequence is linked to the L1 by means of an amide bond; Ci to Cₙ are building blocks that are linked end to end and each have double active functional groups; A represents an A-end ring-closure molecule, which is an amino acid residue; B represents a B-end ring-closure molecule, which is an amino acid residue; the L1 and the A are linked by means of an amide or ester bond; the building block Cₙ and the B are linked by means of an amide or ester bond; and the A and the B react under the action of a cyclase to form a peptide bond and are then linked to form a ring.

53. A cyclic compound library having a general structural formula of: 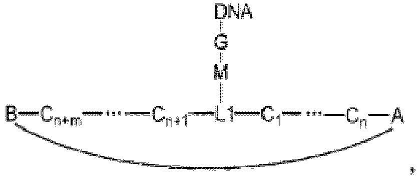
wherein 0 ≤ n ≤ 7, 0 ≤ m ≤ 7, and 2 ≤ n+m ≤ 7;
L1 is a linker molecule having at least three functional groups, and a DNA-encoded sequence is linked to a solid-phase carrier G by means of an amide bond; C₁ to Cₙ are building blocks that are linked end to end and each have double active functional groups, and Cₙ₊₁ to Cₙ₊ₘ are building blocks that are linked end to end and each have double active functional groups; G represents a solid-phase carrier, and M represents a molecule containing a photocleavable group; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

54. A cyclic compound library having a general structural formula of: 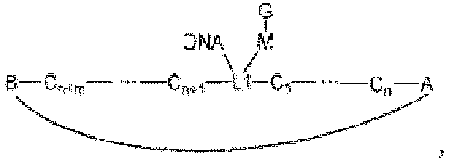
wherein 0 ≤ n ≤ 7, 0 ≤ m ≤ 7, n and m are integers, and 2 ≤ n+m ≤ 7;
L1 is a linker molecule having at least four functional groups, and a DNA-encoded sequence is linked to the L1 by means of an amide bond; Ci to Cₙ are building blocks that are linked end to end and each have double active functional groups, and Cₙ₊₁ to Cₙ₊ₘ are building blocks that are linked end to end and each have double active functional groups; G represents a solid-phase carrier, and M represents a molecule containing a photocleavable group; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

55. A cyclic compound library having a general structural formula of: 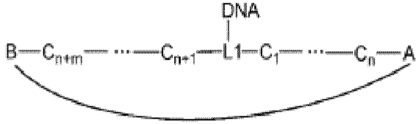
wherein 0 ≤ n ≤ 7, 0 ≤ m ≤ 7, n and m are integers, and 2 ≤ n+m ≤ 7;
L1 is a linker molecule having at least four functional groups, and a DNA-encoded sequence is linked to the L1 by means of an amide bond; C₁ to Cₙ are building blocks that are linked end to end and each have double active functional groups, and Cₙ₊₁ to Cₙ₊ₘ are building blocks that are linked end to end and each have double active functional groups; A represents an A-end ring-closure molecule, which is an amino acid residue; B represents a B-end ring-closure molecule, which is an amino acid residue; the building block Cₙ and the A are linked by means of an amide or ester bond; the building block Cₙ₊ₘ and the B are linked by means of an amide or ester bond; and the A and the B react under the action of a cyclase to form a peptide bond and are then linked to form a ring.

56. The cyclic compound library according to any one of claims 52-55, wherein each of the building blocks is independently selected from the group consisting of substituted or unsubstituted amino acid, substituted or unsubstituted dicarboxylic acid, substituted or unsubstituted diamine, substituted or unsubstituted glycol, α,β-unsaturated aldehyde, α,β-unsaturated ketone, α,β-unsaturated acid, a natural amino acid or an unnatural amino acid, and an N-substituted amino acid, respectively.

57. The cyclic compound library according to claim 56, wherein at least one of the individual building blocks comprises a skeleton structure, which has an E3 ligase substrate structure and can bind to an E3 ligase.

58. The cyclic compound library according to claim 56, wherein the skeleton structure is selected from the group consisting of:

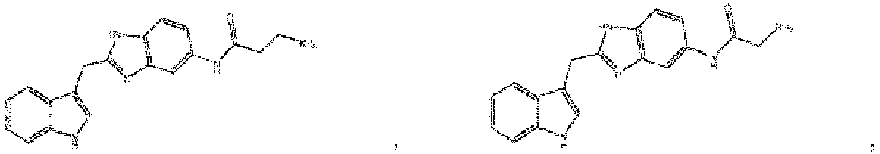

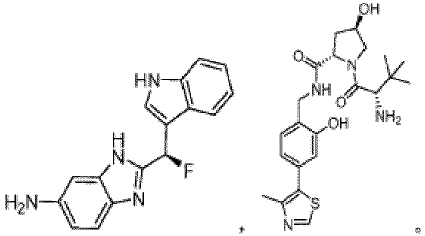

59. The cyclic compound library according to claim 57, wherein each of the individual building blocks comprises at least one extra-ring side chain in total.

60. The cyclic compound library according to claim 57, wherein each of the individual building blocks comprises at least two extra-ring side chains in total, and the at least two extra-ring side chains are linked by a chemical reaction to form a bicyclic structure.
